# FASCICULE DE BREVET EUROPEEN

(11) **EP 2 350 117 B1**
(45) Date de publication et mention de la délivrance du brevet: **10.09.2014**
(21) Numéro de dépôt: 09760175.1
(22) Date de dépôt: 20.10.2009
(51) Int. Cl.: C07K 7/08, C07K 14/775, A61K 38/04

(54) **DERIVES PEPTIDIQUES ET LEUR UTILISATION COMME VECTEURS DE MOLECULES SOUS FORME DE CONJUGUES**
PEPTIDDERIVATE UND DEREN VERWENDUNG ALNG ALS TRÄGER FÜR MOLEKÜLE IN FORM VON KONJUGATEN
PEPTIDE DERIVATIVES AND USE THEREOF AS CARRIERS FOR MOLECULES IN THE FORM OF CONJUGATES

(30) Priorité: 22.10.2008 FR 0857159
(43) Date de publication de la demande: 03.08.2011
(73) Titulaire: Vect-Horus, 13344 Marseille Cedex 15 (FR); Centre National de la Recherche Scientifique, 75794 Paris Cedex 16 (FR); Université d'Aix-Marseille, 13007 Marseille (FR)
(72) Inventeur: KHRESTCHATISKY, Michel, F-13007 Marseille (FR); DAVID, Marion, F-13010 Marseille (FR); MOLINO, Yves, F-13620 Carry Le Rouet (FR); VLIEGHE, Patrick, F-83150 Bandol (FR)
(74) Mandataire: Becker, Philippe
(86) Numéro de dépôt international: PCT/FR2009/051991
(87) Numéro de publication internationale: WO 2010/046588

(56) Documents cités:
- WO-A-99/47566

## Description

L'invention concerne des dérivés peptidiques (peptides et pseudo-peptides) et leur utilisation comme vecteurs de molécules d'intérêt. L'invention concerne également des conjugués contenant un dérivé peptidique de l'invention lié à une molécule d'intérêt. Les peptides et conjugués prodrogues de l'invention sont utilisables pour vectoriser des molécules d'intérêt pharmaceutique ou diagnostique, comme par exemple des molécules thérapeutiques, des agents d'imagerie ou de diagnostic, ou des sondes moléculaires, à travers les membranes cellulaires, et notamment pour favoriser leur transport à travers la barrière hémato-encéphalique (BHE).

### CONTEXTE DE L'INVENTION

Selon *IMS Health,* le marché mondial des médicaments destinés à traiter les pathologies du système nerveux central (SNC, cerveau et moelle épinière) était d'environ 70 milliards de dollars en 2007, dont la part des produits issus des technologies de *drug delivery* est estimé à près de 9 milliards de dollars (Jain, 2008, Jain PharmaBiotech Report, Drug Delivery in CNS disorders). Ainsi, à ce jour le SNC fait partie des 3 aires thérapeutiques les plus importantes avec le cardiovasculaire et l'oncologie. Même si le nombre de personnes souffrant de troubles et de pathologies du SNC à travers le monde est plus important que celui des personnes atteintes par des maladies cardiovasculaires ou des cancers, la neurologie reste un marché sous-exploité. Ceci s'explique par le fait que 98% des médicaments potentiels destinés à traiter les pathologies du SNC ne traversent pas la barrière hémato-encéphalique ou BHE (Pardridge, 2003, Mol. Interv., 3, 90-105).

En effet, le cerveau est protégé contre les substances potentiellement toxiques par la présence de deux systèmes principaux de barrières physiologiques : la BHE, et la barrière sang-liquide céphalo-rachidien (BS-LCR). La BHE est considérée comme la principale voie pour la captation de ligands plasmatiques. Sa superficie est environ 5000 fois supérieure à celle de la BS-LCR. La longueur totale des vaisseaux sanguins constitutifs de la BHE est d'environ 600 km. Chaque cm³ de cortex cérébral contient l'équivalent d'1 km de vaisseaux sanguins. La surface totale de la BHE est estimée à 20 m² (De Boer et al., 2007, Clin. Pharmacokinet., 46 (7), 553-576). Ainsi, l'endothélium cérébral, qui constitue la BHE, représente un obstacle majeur à l'utilisation de médicaments potentiels contre de nombreux troubles du SNC, mais également une surface importante d'échange potentiel entre le sang et le tissu nerveux.

En règle générale, seules quelques petites molécules lipophiles d'environ 450 à 600 Daltons peuvent passer à travers la BHE (soit seulement 2% des candidats-médicaments), c'est-à-dire, passer depuis le sang jusqu'au cerveau. Le poids moléculaire et la taille de nombreux candidats-médicaments, qui montrent des résultats prometteurs dans les études animales pour le traitement des troubles du SNC, sont considérablement plus importants. Ainsi, la plupart des molécules telles que les peptides ou protéines thérapeutiques sont généralement exclues du passage/transport depuis le sang jusqu'au cerveau, en raison de la faible perméabilité des cellules endothéliales des capillaires cérébraux (*brain capillary endothelial cells* ou BCECs) pour ces candidats-médicaments. Les BCECs organisées en vaisseaux sont entourées d'une lame basale, de pieds d'astrocytes, de péricytes et de cellules microgliales et neuronales. L'association étroite des cellules endothéliales avec les pieds astrocytaires est responsable du développement et du maintien des propriétés d'imperméabilité de la BHE à la plupart des molécules, assurant ainsi un contrôle strict et efficace des échanges moléculaires entre le sang et le cerveau afin de maintenir l'homéostasie cérébrale. Les BCECs sont étroitement liées par des jonctions serrées, comparativement aux autres cellules endothéliales d'autres organes, qui sont fenestrées. Ces jonctions serrées empêchent ainsi tout transport para-cellulaire à travers la BHE.

La BHE est considérée comme l'obstacle majeur à franchir dans le développement de nouvelles thérapies destinées à traiter les pathologies cérébrales, et notamment pour l'utilisation de molécules susceptibles de traiter les désordres du SNC (Neuwelt et al., 2008, Lancet Neurol., 7, 84-96).

L'une des raisons pouvant expliquer pourquoi aucun traitement efficace n'est actuellement disponible pour les principales pathologies cérébrales (cancer du cerveau, maladies de Parkinson et d'Alzheimer, accidents vasculaires cérébraux (AVC), etc.) est que les développeurs de candidats-médicaments destinés à traiter des pathologies du cerveau, réalisent en interne des programmes de recherche (*brain drug-discovery programs*) en investissant peu d'efforts dans la problématique du passage de la BHE et dans le ciblage préférentiel du SNC, et notamment du cerveau (*brain drug-targeting programs*), (Pardridge, 2003, Mol. Interw., 3, 90-105). En effet, un candidat-médicament doit répondre à certaines règles structurales, physico-chimiques, pharmacochimiques et pharmacologiques afin d'avoir toutes les chances de devenir un médicament pour traiter une pathologie ou un trouble du SNC (Pajouhesh et al., 2005, NeuroRx, 2 (4), 541-553*).* Dans le développement d'un candidat-médicament, la sélectivité et la spécificité (profilage pharmacologique) d'une molécule pour sa cible sont essentielles à son activité thérapeutique (efficacité). La biodisponibilité et la toxicité potentielle (profilage pharmaceutique) d'une molécule sont quant à elles cruciales pour son devenir en tant que médicament. Autrement dit, toute molécule susceptible de devenir un médicament destiné à traiter une pathologie ou un trouble du SNC doit d'une part passer au travers de la BHE, d'autre part, conserver son activité biologique, et présenter de bonnes propriétés de pharmacocinétique (PK), d'adsorption, de métabolisme, de distribution et d'excrétion (ADME) et de pharmacodynamie (PD), avec une faible toxicité (Tox). Ainsi, la balance hydrophile/lipophile de la molécule en développement est particulièrement difficile à trouver pour les chimistes médicinaux dans cette aire thérapeutique du SNC.

Le problème majeur dans le traitement des troubles et pathologies du SNC réside donc dans le fait que les molécules administrées ne passent pas la BHE et ne peuvent donc atteindre leur(s) cible(s) dans le SNC. Les cellules endothéliales des vaisseaux et capillaires du SNC constitutives de la BHE font obstacle aux molécules qui ne peuvent passer du sang au tissu nerveux. En effet, ces cellules endothéliales et les pieds d'astrocytes qui les entourent constituent une barrière physique liée notamment à l'existence de jonctions serrées entre les cellules endothéliales qui limitent/empêchent tout passage/transport par la voie para-cellulaire, et également une barrière physiologique, puisque ces cellules disposent de systèmes d'efflux efficaces qui restreignent tout passage/transport par la voie trans-cellulaire. Ces propriétés limitent donc fortement le passage des substances du plasma sanguin vers l'espace extracellulaire cérébral.

En effet, certaines molécules, qui sont capables de franchir la BHE, sont expulsées activement du cerveau vers le système sanguin par des protéines de transport *multidrug resistant* (MDR). Ces systèmes d'efflux par transport actif (*active efflux transport,* AET) contrôlent généralement l'efflux actif des petites molécules depuis le cerveau vers le système sanguin. Le système d'AET modèle au niveau de la BHE est le transporteur *ATP Binding Casette* (ABC) à savoir la glycoprotéine P (P-glycoprotein, P-gp) ; toutefois d'autres systèmes d'AET sont présents au niveau de la BHE tel que la *MDR-associated protein 1* (MRP1). La P-gp, qui est principalement localisée à la surface luminale des cellules endothéliales des capillaires cérébraux, est un élément essentiel dans la fonction de barrière physiologique de la BHE prévenant l'entrée dans le cerveau de la plupart des xénobiotiques mais également des candidats-médicaments et autres molécules d'intérêt thérapeutique pouvant être actives dans le SNC.

Une des priorités de la recherche, dans la découverte de molécules destinées à traiter, diagnostiquer ou imager les troubles ou les pathologies cérébrales, est donc de trouver des moyens permettant d'augmenter l'efficacité de passage des substances actives à travers la BHE.

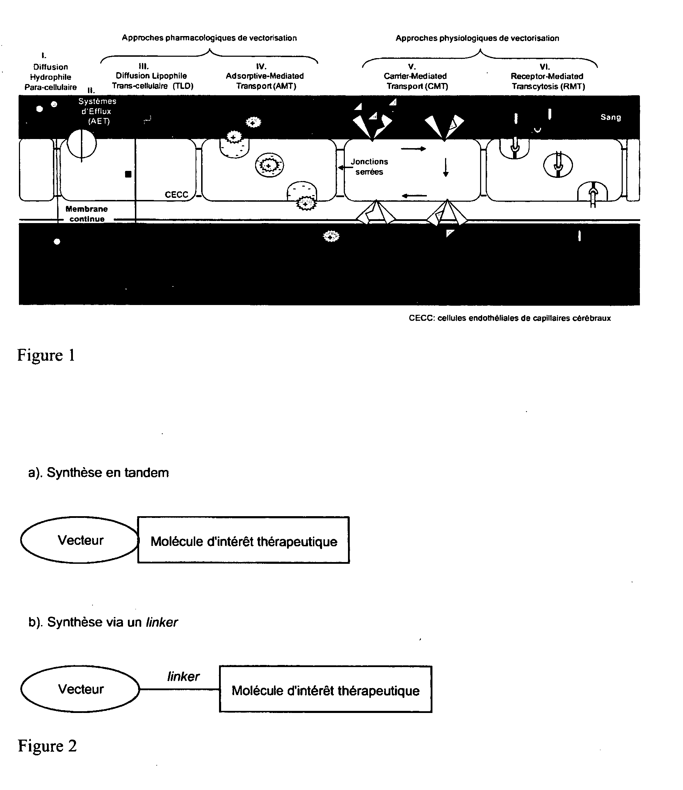

A cet égard, les stratégies de vectorisation de molécules au travers de la BHE, actuellement étudiées et utilisées par les développeurs de candidats-médicaments afin de permettre à une molécule d'intérêt thérapeutique d'atteindre le SNC, peuvent être divisées selon trois principales stratégies (Figure 1), (Pardridge, 2007, Pharm. Res., 24 (9), 1733-1744 ; De Boer et al., 2007, Clin. Pharmacokinet., 46 (7), 553-576 *;* De Boer et al., 2007, Annu. Rev. Pharmacol. Toxicol., 47, 327-355 ; Jones et al., 2007, Pharm. Res., 24 (9), 1759-1771).

### Approches neurochirurgicales

Les approches neurochirurgicales peuvent être mise en oeuvre par injection intra-ventriculaire cérébrale directe de la substance active, injection intracérébrale ou infusion intrathécale, ou par perturbation de la BHE (rupture temporaire de l'intégrité de la BHE).

Le problème principal des approches neurochirurgicales par injection intra-ventriculaire, en dehors des coûts relatifs à l'acte neurochirurgical, est que le médicament n'est pas délivré directement au niveau du parenchyme cérébral mais dans le liquide céphalo-rachidien. En effet, l'infusion intra-ventriculaire implique le placement d'un cathéter dans les ventricules (Aird, 1984, Exp. Neurol., 86, 342-358*).* Cette technique très invasive n'est pas efficace pour le transport de substances actives dans le parenchyme cérébral. En effet, le volume d'écoulement depuis le liquide céphalo-rachidien jusqu'au parenchyme cérébral lors de la délivrance d'un médicament par infusion intra-ventriculaire est régi par une diffusion anormalement lente de sa convection (de son transport), car le cerveau ne possède pas de débit volumique intra-parenchymateux.

De même pour l'injection intracérébrale, la diffusion d'une substance active dans le cerveau décroît très rapidement depuis le site d'injection jusqu'au site de lésion. En effet, la concentration cérébrale d'une substance active diminue de 90% à une distance de 500 µm de son site d'injection.

L'infusion intrathécale implique le placement d'un cathéter dans le cerveau connecté à une pompe qui délivre la substance active à un débit prédéfini. Du fait que le cerveau soit le seul organe qui n'ait pas de système lymphatique, servant normalement à ramener les fluides extracellulaires jusqu'à la circulation générale, la distribution d'une substance active par infusion intrathécale au niveau cérébral est très lente. Ceci diminue la concentration de la substance active au site de lésion.

De plus, les risques infectieux sont importants au cours de tels actes neurochirurgicaux, notamment de par la présence d'un cathéter. Dans ces conditions, le confort des patients n'est pas optimal.

L'interruption temporaire de l'imperméabilité de la BHE est associée à une ouverture transitoire des jonctions serrées des cellules endothéliales des capillaires cérébraux. C'est le cas des substances vasoactives comme les leukotriènes ou bradykinines (Baba et al., 1991, J. Cereb. Blood Flow Metab., 11, 638-643). Cette stratégie est également invasive et nécessite un accès artériel au niveau de la carotide chez les sujets/patients mis sous sédatifs. Le problème majeur rencontré par la rupture temporaire de l'intégrité de la BHE, outre les dépenses relatives à l'acte du radiologue pour l'accès à la carotide, est que la BHE ne reste ouverte que pendant une courte période de temps, limitant de fait la possibilité de délivrer en chronique un médicament. En outre, la rupture temporaire de la BHE permet aux protéines plasmatiques d'entrer dans le cerveau (alors que ces protéines peuvent être toxiques pour le cerveau) et peut également faciliter l'entrée d'agents infectieux. Ce type de rupture de la BHE peut donc conduire à des perturbations neuropathologiques chroniques et est associé à des risques importants d'infection (Salahuddin et al., 1988, Acta Neuropathol., 76, 1-10).

### Approches pharmacologiques de vectorisation

Les stratégies pharmacologiques pour le transport de molécules comprennent la diffusion trans-cellulaire de molécules rendues plus lipophiles par l'addition de groupements lipidiques sur la substance active (*Transcellular Lipophilic Diffusion* ou TLD) voire l'utilisation des liposomes (Zhou et al., 1992, J. Control. Release, 19, 459-486), et le transport par adsorption ionique via des molécules vecteurs chargées positivement ou par cationisation de la molécule active (*Adsorptive*-*Mediated Transport* ou AMT).

L'addition d'un groupement lipidique permet la conversion chimique des molécules hydrophiles en molécules plus lipophiles notamment au travers d'approches prodrogues. Cependant, la synthèse de tels composés conduit à des molécules qui dépassent le seuil de transport optimal pour traverser la BHE, notamment en ce qui concerne le poids moléculaire qui devient supérieur à la limite optimale de 450 Daltons (Pajouhesh et al., 2005, NeuroRx, 2 (4), 541-553). Pour cette même raison, les liposomes ou même les petites vésicules ou nanoparticules (micelles, nanosphères, nanocapsules) sont généralement trop grosses, pas assez spécifiques pour la BHE, et par conséquent relativement inefficaces pour le transport de molécules d'intérêt thérapeutique (ou d'agents d'imagerie ou de diagnostic, ou de toute autre molécule comme une sonde moléculaire) à travers la BHE (Levin, 1980, J. Med. Chem., 23, 682-684 ; Schackert et al., 1989, Selective Cancer Ther., 5, 73-79). Ainsi, les principaux problèmes rencontrés par les technologies de lipidisation (TLD) sont leur faible spécificité pour cibler et traverser spécifiquement la BHE par rapport à d'autres membranes cellulaires, la diminution des valeurs plasmatiques de l'aire sous la courbe de la drogue (ASC), et leur utilisation généralement limitée à la vectorisation de petites molécules.

Dans les approches d'AMT, le principal problème rencontré est la faible spécificité pour cibler et traverser spécifiquement la BHE par rapport à d'autres membranes cellulaires. En effet, l'AMT est basée sur des molécules cationiques s'adsorbant sur des cellules dont la membrane est chargée négativement, ce qui est le cas de la plupart des cellules. La diminution des valeurs plasmatiques de l'ASC de la drogue, leur utilisation généralement limitée à la vectorisation de petites molécules, et leur cytotoxicité sont autant de facteurs qui pénalisent l'approche de vectorisation via AMT.

### Approches physiologiques de vectorisation

Les stratégies fondées sur des approches physiologiques de vectorisation consistent à exploiter les différents mécanismes de transport naturel au niveau de la BHE. Ces mécanismes de transport actif de molécules au travers de la BHE se font soit via couplage à un substrat spécifique d'un récepteur ou par mimétisme moléculaire avec le substrat spécifique d'un récepteur (*Carrier-Mediated Transport* ou CMT), soit via couplage ou fusion à un ligand ciblant spécifiquement un récepteur (*Receptor-Mediated Transport* ou RMT).

A titre d'exemple des molécules comme la L-DOPA (maladie de Parkinson), le melphalan (cancer du cerveau), l'α-méthyl-DOPA (hypertension artérielle) et la gabapentine (épilepsie) passent dans le cerveau par CMT via le *large neutral amino-acid transporter* (LAT1), (Pardridge, 2003, Mol. Interv., 3, 90-105). Ces molécules ont des structures chimiques proches de la phénylalanine, l'un des substrats naturels de LAT1. Toutefois, les principaux problèmes rencontrés par les approches CMT sont leur grande sélectivité/spécificité pour des conjugués qui imitent/miment étroitement le substrat du récepteur/transporteur endogène, et par conséquent leur utilisation qui reste limitée à la vectorisation de petites molécules.

Le RMT fait appel à un système de transport récepteur-dépendant. La vectorisation est réalisée via des mécanismes d'endocytose par le ciblage des récepteurs/transporteurs endogènes présents au niveau des capillaires cérébraux. Parmi les différents récepteurs humains de la BHE impliqués dans le RMT, on recense notamment: le récepteur à la transferrine (TfR), le récepteur à l'insuline (IR), les récepteurs aux *low-density lipoproteins* (LDL) permettant le transport du cholestérol dont le récepteur au LDL (LDLR) et les membres de la famille des *low*-*density lipoprotein receptor-related protein* (LRP), ou encore le récepteur à l'*insulin-like growth factor* (IGFR), le récepteur à la toxine diphtérique (DTR) ou *heparin binding epidermal growth factor-like growth factor* (HB-EGF), ainsi que les *scavenger receptors* (SCAV-Rs) dont le *scavenger receptor class B type I* (SR-BI). Dans le RMT, les récepteurs sur la membrane d'une cellule endothéliale de la BHE lient leur ligand, ce qui entraîne l'endocytose du complexe constitué du récepteur/transporteur et de son ligand dans une vésicule qui se forme à la surface de la cellule et pénètre ensuite dans la cellule endothéliale de la BHE. Le complexe ligand/récepteur peut passer au travers de la cellule endothéliale (transcytose), et par conséquent traverser ainsi la BHE pour agir dans le tissu nerveux. Ce processus de RMT ne dépend pas de la taille de ce qui est endocyté. Ainsi, le RMT est un mécanisme qui permet le transport depuis le sang jusque dans le cerveau de molécules telles que l'insuline, des protéines transporteuses de fer, le cholestérol, divers dérivés peptidiques et protéines, etc. Par exemple, la transferrine est utilisée comme vecteur ligand du TfR présent sur la BHE (Jefferies et al., 1984, Nature, 312, 162-163 ; Friden et al., 1983, Science, 259, 373-377 ; Friden, 1994, Neurosurgery, 35, 294-298*),* et la molécule à transporter (substance active) est couplée à la transferrine (vecteur ligand). Bien que cette stratégie de vectorisation à l'aide d'une macromolécule permette une augmentation du passage des molécules d'intérêt conjuguées à travers la BHE, elle présente quelques inconvénients. D'abord, le couplage de la molécule au vecteur se fait généralement par des méthodes d'expression génétique (fusion) limitant ainsi le nombre de molécules à transporter à seulement des polypeptides ou des protéines. Ensuite, le système de couplage de la molécule au vecteur est assez complexe, un couplage chimique ou biochimique traditionnel ne permet pas d'obtenir des systèmes macromoléculaires bien définis d'un point de vue structurel et moléculaire.

### RESUME DE L'INVENTION

La présente invention permet de palier ces inconvénients. L'invention montre qu'il est possible de concevoir des peptides ou pseudo-peptides de taille réduite capables de véhiculer à travers les membranes cellulaires, et plus spécifiquement la BHE, des substances de masse et/ou volume importants. L'invention propose ainsi de nouveaux peptides, conjugués et compositions, permettant d'améliorer la biodisponibilité de molécules d'intérêt, et notamment d'améliorer leur accès (ciblage) au SNC.

Plus particulièrement, les inventeurs ont mis au point des dérivés peptidiques capables de lier le LDLR humain. Les inventeurs ont montré que ces dérivés étaient capables de traverser la BHE. Les inventeurs ont également montré que ces dérivés permettaient de véhiculer, dans les cellules de la BHE, des molécules d'intérêt thérapeutique ou diagnostique. En outre, les inventeurs ont mis au point des peptides capables de lier le LDLR sans compétition avec le ligand naturel, et donc sans interférence avec le transport du LDL. Ces dérivés peptidiques représentent donc de nouveaux produits (vecteurs) particulièrement avantageux pour la conception et la vectorisation de médicaments ou d'agents de diagnostic, notamment pour atteindre le SNC.

Un objet de l'invention réside ainsi dans un peptide ou pseudo-peptide tel que défini dans les revendications. L'invention décrit ainsi des peptides ou pseudopeptides comprenant une séquence d'acides aminés naturels et/ou non naturels, caractérisé en ce qu'il contient au plus 30 résidus d'acides aminés et en ce qu'il lie le LDLR humain à la surface de membranes cellulaires. Les peptides comprennent au moins 5 résidus d'acides aminés, de préférence au moins 6, 7, ou 8. Dans un mode de mise en oeuvre préféré, les peptides de l'invention lient également le LDLR murin. De manière tout particulièrement avantageuse et préférée, les peptides de l'invention présentent la capacité de passer la BHE et potentiellement les membranes de cellules cancéreuses ou infectieuses.

Un autre objet de l'invention concerne un conjugué comprenant un peptide ou pseudo-peptide tel que défini ci-dessus couplé à une substance d'intérêt. Comme il sera indiqué dans la suite, le couplage est avantageusement covalent et peut être réalisé de manière à se dissocier après le passage de membranes cellulaires, afin de libérer la substance d'intérêt en un site d'intérêt. Selon la nature du couplage, la libération de la substance peut s'opérer par exemple de manière passive ou sous l'action d'enzymes ou de conditions physiologiques déterminées.

Un autre objet de l'invention concerne un procédé de préparation d'un conjugué tel que défini ci-dessus.

Un autre objet de l'invention concerne une composition pharmaceutique ou diagnostique comprenant un conjugué de l'invention.

Un autre objet de l'invention concerne l'utilisation d'un peptide ou pseudo-peptide, ou d'un conjugué, tels que définis ci-dessus, pour la préparation d'un médicament, agent de diagnostic ou d'imagerie.

Un autre objet de l'invention concerne une méthode pour améliorer ou permettre le passage d'une molécule à travers la BHE, comprenant le couplage de cette molécule à un peptide ou pseudo-peptide tel que défini ci-dessus.

Un autre objet de l'invention réside dans une méthode améliorée de traitement d'une pathologie chez un sujet par un médicament, l'amélioration consistant à coupler ce médicament à un peptide ou pseudo-peptide tel que défini ci-dessus.

L'invention est utilisable chez tout mammifère, notamment tout être humain.

### LEGENDE DES FIGURES

Figure 1
   Schéma illustrant les différents modes de passage de molécules naturelles ou pharmacologiques à travers la BHE, adapté d'après Abbott et Romero, 1996, Mol. Med. Today, 2 (3), 106-113*.*
Figure 2
   Schéma comparatif de la synthèse en tandem et de la synthèse via un *linker* d'un conjugué vecteur/molécule d'intérêt thérapeutique.
Figure 3
   A - Schéma du plasmide utilisé pour cloner les hLDLR et mLDLR.
   B - Schéma représentant la protéine de fusion exprimée par les cellules transfectées.
Figure 4
   Western blot réalisé sur des lignées cellulaires CHO exprimant de manière constitutive les protéines de fusion hLDLR-GFP ou GFP (utilisée comme contrôle). Une bande de 190 kDa correspondant à la taille de la protéine de fusion hLDLR-GFP est détectée avec l'anticorps anti-hLDLR.
Figure 5
   Immunocytochimie sur cellules CHO non perméabilisées, exprimant de manière stable soit A - la GFP seule (contrôle), soit B - la construction hLDLR-GFP. Les noyaux cellulaires sont marqués au Hoechst (bleu, A1 et B1). La fluorescence de la GFP est visible en vert (A2 et B2), celle du marquage du domaine extracellulaire du hLDLR par l'anticorps anti-hLDLR est visible dans le rouge (A3 et B3) et la superposition des marquages rouges et verts est visible en jaune/orange (A4 et B4). Noter que seules les cellules transfectées de manière stable avec la construction hLDLR-GFP expriment le récepteur à la membrane (B3).
Figure 6
   A - Cellules de la lignée CHO-hLDLR-GFP exprimant le hLDLR-GFP (vert) incubées avec du DiI-LDL (rouge), la superposition des marquages rouges et vert est visible en jaune/orange, noter le fort marquage des cellules.
   B - Cellules d'une lignée CHO-TfR-GFP exprimant le hTfR-GFP (vert) incubées avec du DiI-LDL (rouge) : absence de liaison et d'endocytose du DiI-LDL.
   C - Cellules de la lignée CHO-hLDLR-GFP exprimant le hLDLR-GFP (vert) incubées avec de la transferrine couplée à du *Texas Red* (rouge) : absence de liaison et d'endocytose du ligand Tf.
      Noter la différence d'intensité de marquage entre A d'une part, B et C d'autre part, où seul le marquage des récepteurs hTfr et hLDLR fusionnés à la GFP est détecté.
Figure 7
   Lignées CHO exprimant de manière stable A - la GFP seule (vert), et B - le hLDLR-GFP (vert), et immunomarquage avec un anticorps anti-protéine de l'enveloppe virale d'un clone de virus bactériens présentant un peptide (SEQ ID NO : 1) affin pour le hLDLR (points rouges). Les noyaux cellulaires sont marqués au Hoechst (bleu, A1 et B1). La fluorescence de la GFP est visible en vert (A2 et B2), celle du marquage de l'enveloppe virale du virus bactérien par l'anticorps anti-protéine virale est visible dans le rouge (A3 et B3) et la superposition des marquages rouges et verts est visible en jaune/orange (A4 et B4). Noter que les cellules qui n'expriment pas le hLDLR en A ne fixent pas de virus bactériens.
Figure 8
   Comparaison par immunocytochimie de la liaison de virus bactériens contrôles (A-C) et de virus bactériens présentant des peptides affins pour le hLDLR (SEQ ID NO : 1), (B, D) sur des fibroblastes humains (A-B) et sur des cellules endothéliales microvasculaires de cerveau porcin (C-D). Les virus bactériens sont révélés avec un anticorps anti-protéine de l'enveloppe virale.
Figure 9
   Evaluation par FACS de l'interaction entre cellules CHO-hLDLR-GFP et un clone de virus bactériens présentant le peptide SEQ ID NO : 11 affin pour le hLDLR comparé à des virus bactériens contrôles ne présentant pas de peptides affins pour ce récepteur. Le signal en Q2 montre une combinaison de 2 signaux sur les cellules qui sont positifs d'une part si le hLDLR-GFP est exprimé (fluorescence GFP, axe horizontal) et d'autre part si des virus bactériens affins pour ce récepteur sont liés aux cellules via les peptides qu'ils présentent (marquage par immunocytochimie, axe vertical).
   A - Cellules CHO-hLDLR-GFP incubées avec l'anticorps anti-protéine de l'enveloppe virale et son anticorps secondaire APC.
   B - Cellules CHO-hLDLR-GFP incubées avec des virus bactériens contrôles (négatifs), l'anticorps anti-protéine de l'enveloppe virale et son anticorps secondaire APC.
   C - Cellules CHO-hLDLR-GFP incubées avec un clone de virus bactériens présentant un peptide affin pour le hLDLR (SEQ ID NO: 11), l'anticorps anti-protéine de l'enveloppe virale et son anticorps secondaire APC. On observe un déplacement important du signal en Q2.
Figure 10
   Evaluation par FACS de l'interaction entre fibroblastes humains et un clone de virus bactériens présentant le peptide SEQ ID NO : 12 affin pour le hLDLR, comparé à des virus bactériens contrôles ne présentant pas de peptides affins pour ce récepteur. Le signal en Q2 montre une combinaison de 2 signaux sur les cellules qui sont positifs d'une part si le hLDLR est exprimé (marquage par immunocytochimie, axe horizontal) et d'autre part si des virus bactériens affins pour ce récepteur sont liés aux cellules via les peptides qu'ils présentent (marquage par immunocytochimie, axe vertical).
   A - Fibroblastes humains incubés avec l'anticorps anti-protéine de l'enveloppe virale et son anticorps secondaire APC.
   B - Fibroblastes humains incubés avec l'anticorps anti-LDLR et son anticorps secondaire Alexa 488.
   C - Fibroblastes humains incubés avec des virus bactériens contrôles (négatifs), l'anticorps anti-protéine de l'enveloppe virale, l'anticorps anti-LDLR et les anticorps secondaires APC et Alexa 488.
   D - Fibroblastes humains incubés avec un clone de virus bactériens présentant le peptide SEQ ID NO : 12 affin pour le hLDLR, l'anticorps anti-protéine de l'enveloppe virale, l'anticorps anti-LDLR et les anticorps secondaires APC et Alexa 488. On observe un déplacement important du signal en Q2.
Figure 11
   Evaluation par FACS de l'interaction entre cellules endothéliales HUVEC et 2 clones de virus bactériens présentant les peptides, l'un cyclique (SEQ ID NO : 11), l'autre linéaire (SEQ ID NO : 21), affins pour le hLDLR, comparé à des virus bactériens contrôles ne présentant pas de peptides affins pour ce récepteur.
   A - Cellules endothéliales HUVEC incubées avec l'anticorps anti-protéine de l'enveloppe virale et son anticorps secondaire APC.
   B - Cellules endothéliales HUVEC incubées avec l'anticorps anti-LDLR et son anticorps secondaire Alexa 488.
   C - Cellules endothéliales HUVEC incubées avec des virus bactériens contrôles (négatifs), l'anticorps anti-protéine de l'enveloppe virale, l'anticorps anti-LDLR et les anticorps secondaires APC et Alexa 488.
   D - Cellules endothéliales HUVEC incubées avec un clone de virus bactériens présentant le peptide cyclique (SEQ ID NO : 11) affin pour le hLDLR, l'anticorps anti-protéine de l'enveloppe virale, l'anticorps anti-LDLR et les anticorps secondaires APC et Alexa 488. On observe un déplacement important du signal en Q2.
   E - Cellules endothéliales HUVEC incubées avec un clone de virus bactériens présentant le peptide linéaire (SEQ ID NO : 21) affin pour le hLDLR, l'anticorps anti-protéine de l'enveloppe virale, l'anticorps anti-LDLR et les anticorps secondaires APC et Alexa 488. On observe un déplacement important du signal en Q2.
Figure 12
   Evaluation par FACS sur cellules HUVEC d'une compétition entre les peptides présentés par les virus bactériens affins pour le LDLR et son ligand naturel le LDL.
   A - Cellules endothéliales HUVEC incubées avec l'anticorps anti-protéine de l'enveloppe virale et son anticorps secondaire APC.
   B - Cellules endothéliales HUVEC incubées avec l'anticorps anti-LDLR et son anticorps secondaire Alexa 488.
   C - Cellules endothéliales HUVEC incubées avec des virus bactériens contrôles (négatifs), l'anticorps anti-protéine de l'enveloppe virale, l'anticorps anti-LDLR et les anticorps secondaires APC et Alexa 488.
   D - Cellules endothéliales HUVEC incubées avec des virus bactériens contrôles (négatifs), l'anticorps anti-protéine de l'enveloppe virale, l'anticorps anti-LDLR, le LDL, et les anticorps secondaires APC et Alexa 488. Aucun effet n'est observé dû à la présence du LDL.
   E - Cellules endothéliales HUVEC incubées avec un clone de virus bactériens présentant le peptide linéaire (SEQ ID NO : 21) affin pour le hLDLR, l'anticorps anti-protéine de l'enveloppe virale, l'anticorps anti-LDLR et les anticorps secondaires APC et Alexa 488.
   F - Cellules endothéliales HUVEC incubées avec un clone de virus bactériens présentant le peptide linéaire (SEQ ID NO : 21) affin pour le hLDLR, l'anticorps anti-protéine de l'enveloppe virale, l'anticorps anti-LDLR, le LDL et les anticorps secondaires APC et Alexa 488. Le LDL diminue fortement la liaison des peptides SEQ ID NO : 21 présentés par les virus bactériens.
   G - Cellules endothéliales HUVEC incubées avec un clone de virus bactériens présentant le peptide cyclique (SEQ ID NO : 11) affin pour le hLDLR, l'anticorps anti-protéine de l'enveloppe virale, l'anticorps anti-LDLR et les anticorps secondaires APC et Alexa 488.
   H - Cellules endothéliales HUVEC incubées avec un clone de virus bactériens présentant le peptide cyclique (SEQ ID NO : 11) affin pour le hLDLR, l'anticorps anti-protéine de l'enveloppe virale, l'anticorps anti-LDLR, le LDL et les anticorps secondaires APC et Alexa 488. Aucun effet n'est observé dû à la présence du LDL.
   I - Graphe représentant le pourcentage de déplacement du signal de fluorescence dans la zone Q2 des graphiques A-H obtenus par FACS. On ne mesure aucun déplacement en Q2 pour les virus bactériens contrôles qui ne présentent pas de peptides affins pour le hLDLR. Pour les virus bactériens présentant le peptide SEQ ID NO : 21, plus de 55,8% du signal se déplace en Q2 indiquant une affinité des peptides présentés par ces virus bactériens pour le hLDLR des cellules HUVEC. L'addition de LDL entraîne une perte du signal en Q2 de 85% au niveau des virus bactériens contrôles, indiquant une compétition entre le virus bactérien correspondant à la séquence peptidique SEQ ID NO : 21 et le LDL au niveau du site de liaison du LDL au hLDLR. Le signal en Q2 des virus bactériens qui présentent le peptide SEQ ID NO: 11 également important (déplacement supérieur à 70% par rapport aux virus bactériens contrôles) n'est pratiquement pas déplacé (17%) par l'ajout de LDL.
Figure 13
   Double marquage en immunofluorescence de coupes à congélation de cerveau de souris C57BL6, 2h après injection dans la veine de queue de souris de virus bactériens contrôles (A-B) et de virus bactériens présentant un peptide affin (peptide SEQ ID NO : 1) pour le hLDLR et pour le mLDLR (C-D). Les vaisseaux cérébraux sont marqués avec un IgG anti-souris, en vert (A-C), les virus bactériens sont marqués avec un anticorps anti-protéine de l'enveloppe virale, en rouge (B-D).
Figure 14
   Schéma général des peptides synthétisés conjugués à la rhodamine ou au S-Tag, avec bras espaceur en C-terminal (C-term).
Figure 15
   Evaluation par fluorescence et immunocytochimie de la liaison et de l'endocytose des peptides affins pour le hLDLR (SEQ ID NO : 1/rhodamine en A et, SEQ ID NO : 2/S-Tag en C) sur les cellules de la lignée CHO-hLDLR-GFP comparé à un peptide contrôle (en B). La fluorescence de la GFP est visible en vert (A1, B1 et C1), celle liée aux peptides, révélée grâce à la rhodamine ou par liaison d'un anticorps anti-S-Tag couplé à un Alexa594 est visible dans le rouge (A2, B2 et C2), et la superposition des marquages rouges et vert est visible en jaune/orange (A3, B3 et C3). Noter les forts taux de marquage intracellulaire dans le rouge avec les peptides SEQ ID NO : 1/rhodamine en A2 et, SEQ ID NO : 2/S-Tag en C2, et la superposition du marquage hLDLR-GFP (vert, A1, C1) avec les peptides liés ou internalisés par endocytose (A2, C2).
Figure 16
   Quantification des taux de liaison (A-C) et d'endocytose (B-D) des peptides SEQ ID NO : 11 et SEQ ID NO : 2 et d'un peptide contrôle sur lignée CHO-hLDLR-RFP (A-B) et sur fibroblastes humains (C-D) respectivement.
Figure 17
   Evaluation par immunocytochimie de l'interaction entre cellules CHO-hLDLR-GFP et le peptide SEQ ID NO : 11 affin pour le hLDLR, conjugué au S-Tag, comparé à un peptide contrôle également conjugué au S-Tag. Le signal en Q2 montre une combinaison de 2 signaux sur les cellules qui sont positifs d'une part si le hLDLR-GFP est exprimé (fluorescence GFP, axe horizontal) et d'autre part si le peptide contrôle, et le peptide SEQ ID NO : 11 affin pour ce récepteur, est lié aux cellules (marquage par immunocytochimie, axe vertical).
   A - Cellules incubées avec l'anticorps secondaire APC.
   B - Cellules incubées avec le peptide contrôle conjugué au S-Tag, l'anticorps anti S-Tag et l'anticorps secondaire APC.
   C - Cellules incubées avec le peptide SEQ ID NO : 11 affin pour le LDLR, conjugué au S-Tag, l'anticorps anti S-Tag et l'anticorps secondaire APC. On observe un déplacement important du signal en Q2.
Figure 18
   Evaluation de la toxicité des peptides sur une barrière de cellules endothéliales dans le modèle de BHE *in vitro* par co-incubation avec le *Lucifer Yellow* (LY, analyse fluorimétrique) et analyse du taux de passage du LY en fonction du temps et en absence ou en présence d'un peptide contrôle et du peptide SEQ ID NO : 1, tous deux conjugués à la rhodamine.
Figure 19
   Evaluation après lyse des cellules et analyse par fluorimétrie des taux de liaison aux cellules endothéliales du modèle BHE *in vitro* et/ou internalisation dans ces cellules, des peptides contrôle et SEQ ID NO : 1, tous deux conjugués à la rhodamine.
Figure 20
   Evaluation par fluorimétrie des taux de passage (Perméabilité, Pe) à travers les cellules endothéliales d'un modèle BHE *in vitro* des peptides contrôle et SEQ ID NO : 1, tous deux conjugués à la rhodamine. La mesure de Pe du LY permet de vérifier l'intégrité des modèles de BHE *in vitro.*

### DESCRIPTION DETAILLEE DE L'INVENTION

L'invention concerne des dérivés peptidiques capables de lier le LDLR humain et leur utilisation dans le domaine pharmaceutique, notamment pour véhiculer, dans les cellules de la BHE, des molécules d'intérêt thérapeutique ou diagnostique.

Le LDLR humain est une protéine transmembranaire de 839 acides aminés comprenant trois régions : la région extracellulaire (1-768), la région transmembranaire (768-790) et la région cytoplasmique (790-839). La région extracellulaire est divisée en deux sous-régions : celle de liaison des LDL (1-322) et celle en dehors de la zone de liaison des LDL (322-768) (voir WO2007/014992).

Le cerveau a un besoin important de LDL pour son bon fonctionnement. Les ligands naturels au LDLR sont le LDL et plus particulièrement l'apolipoprotéine B (ApoB) et l'apolipoprotéine E (ApoE) constitutives des particules de LDL permettant ainsi le transport de cholestérol, contenu dans ces particules, au travers des membranes cellulaires et plus particulièrement de la BHE.

Il a ainsi été démontré que le LDLR permettait la transcytose de particules de LDL au travers de la BHE (Dehouck et al., 1997, J. Cell Biol., 138 (4), 877-889), via un processus de RMT, dans des vésicules endosomiques particulières qui empêchent la fusion au lysosome. Ces lipoprotéines, ayant passé la BHE par transcytose, sont alors captées par les neurones et/ou les astrocytes (Spencer et al., 2007, Proc. Natl. Acad. Sci. USA, 104 (18), 7594-7599). Cette propriété a été utilisée pour vectoriser des molécules d'intérêt thérapeutique par des nanoparticules sur lesquelles sont conjuguées des apolipoprotéines entières (constitutives du LDL) (Kreuter et al., 2007, J. Control. Release, 118, 54-58). Dans cette étude, toutefois, une apolipoprotéine entière a été utilisée, et sous forme couplée à une nanoparticule afin de mimer la structure de la particule LDL. WO99/47566 mentionne des peptides susceptibles de lier le LDL.

La présente invention montre, pour la première fois, qu'il est possible de concevoir de petits peptides capables de lier le LDLR et de passer à travers la BHE. L'invention offre de nombreux avantages par rapport aux stratégies de l'art antérieur, qui découlent notamment du choix du récepteur, de la stratégie de conception des peptides, et de leur nature. L'invention montre que de tels peptides ou pseudo-peptides sont sélectifs pour certaines membranes cellulaires et peuvent être utilisés pour délivrer aussi bien des petites molécules chimiques (qu'elles soient ou non lipophiles), que des macromolécules telles que des protéines d'intérêt thérapeutique. Les peptides ou pseudo-peptides vecteurs peuvent être aisément synthétisés par voie chimique, et la plupart des molécules d'intérêt thérapeutique, ou des agents d'imagerie ou de diagnostic peuvent être couplées au peptide ou pseudo-peptide vecteur de façon simple et efficace au travers d'une stratégie prodrogue via un bras espaceur (synthèse via *linker*) ou par couplage direct (synthèse en tandem) entre les deux entités (Figure 2). Les peptides et pseudo-peptides de l'invention peuvent être conçus pour adopter une configuration cyclique, et donc plus résistante à la protéolyse. Par ailleurs, les peptides et pseudo-peptides de l'invention peuvent être conçus pour lier le LDLR sans compétition avec le ligand naturel. L'invention a en effet permis la découverte d'un nouveau site de liaison sur le LDLR, différent du site de liaison des LDL. De ce fait, l'utilisation de peptides et pseudo-peptides de l'invention ciblant ce site permet un transport efficace sans perturbation substantielle de la liaison du ligand naturel.

Les travaux de recherche réalisés dans le cadre de la présente invention ont permis à la Demanderesse de montrer que les peptides ou pseudo-peptides linéaires ou cycliques qu'elle a mis au point peuvent être utilisés comme vecteurs de molécules d'intérêt thérapeutique, ou d'agents d'imagerie ou de diagnostic, ou de toute autre molécule comme une sonde moléculaire, dans le traitement, l'imagerie et/ou le diagnostic de pathologies neurologiques, ainsi que de pathologies infectieuses ou cancéreuses cérébrales ou non.

Les peptides ou pseudo-peptides linéaires ou cycliques décrits dans la présente invention possèdent la capacité de cibler des récepteurs/transporteurs cellulaires, des types cellulaires particuliers et/ou de passer les membranes cellulaires notamment celles des barrières physiologiques du cerveau et plus particulièrement la BHE ou la barrière sang-rétine (BSR).

Les peptides ou pseudo-peptides linéaires ou cycliques décrits dans la présente invention possèdent la capacité de cibler des récepteurs/transporteurs cellulaires, des types cellulaires particuliers, notamment des cellules cancéreuses, du tissu nerveux ou non et/ou de passer les membranes cellulaires notamment celles des barrières physiologiques du SNC et plus particulièrement la barrière hémato-tumorale (BHT) au niveau des tumeurs du tissu nerveux.

Les peptides ou pseudo-peptides linéaires ou cycliques décrits dans la présente invention possèdent la capacité de cibler des récepteurs/transporteurs cellulaires, des types cellulaires particuliers et/ou de passer les membranes cellulaires, notamment celles des barrières physiologiques du SNC, pour traiter plus particulièrement des pathologies infectieuses cérébrales ou autres, de type bactériennes, virales, parasitaires ou fongiques.

Les peptides ou pseudo-peptides linéaires ou cycliques décrits dans la présente invention possèdent la capacité de se fixer sur un LDLR murin ou humain de la membrane cellulaire et de traverser ladite membrane grâce à ce récepteur par transcytose.

Les peptides ou pseudo-peptides linéaires ou cycliques décrits dans la présente invention possèdent la capacité de se fixer sur le LDLR à la surface des membranes cellulaires des barrières physiologiques du cerveau de type murin et humain et de traverser ladite barrière physiologique grâce au LDLR par RMT.

L'invention concerne donc plus particulièrement des peptides et pseudo-peptides qui présentent une affinité pour le LDLR, et leur utilisation comme vecteurs de molécules d'intérêt thérapeutique ou d'agents d'imagerie ou de diagnostic, ou de toute autre molécule comme une sonde moléculaire. De tels peptides sont utilisables dans de nombreuses indications, notamment dans le traitement, l'imagerie et/ou le diagnostic de pathologies neurologiques, ainsi que de pathologies infectieuses ou cancéreuses, cérébrales ou non.

Un objet de l'invention réside donc dans un peptide ou pseudo-peptide tels que définis dans les revendications. Au sens de l'invention, le terme peptide ou pseudo-peptide désigne une molécule comprenant un enchaînement de résidus d'acides aminés, qui peuvent être naturels ou non, éventuellement modifiés ou fonctionnalisés, et liés entre eux par des liaisons peptidiques, non peptidiques ou peptidiques modifiées. Les peptides peuvent être cycliques ou non, et présenter, le cas échéant, une ou des extrémités protégées.

Un objet préféré de l'invention réside dans un peptide ou pseudo-peptide tel que défini ci-dessus, caractérisé en ce qu'il présente la capacité de passer la BHE.

Dans un premier mode de mise en oeuvre, les peptides ou pseudo-peptides de l'invention répondent à la formule générale (I) suivante :

X-M-P-R-Y (I)

dans laquelle :
X est un groupe de formule (Xaa)ᵢZ(Xaa)ⱼ et Y est un groupe de formula (Xaa)ₖW(Xaa)ₗ, dans lesquelles Xaa représente un acide aminé naturel ou non, y compris un acide aminé de configuration D, un acide aminé non code, ou un acide aminé contenant une liaison peptidomimétique, Z et W représentent deux acides aminés identiques ou différents permettant la cyclisation du peptide, et i, j, k et l sont des nombres entiers, identiques ou différents, compris entre 0 et 5 ;
   - M désigne la méthionine ou un de ses isostères ou un de ses analogues,
   - P désigne la proline ou un de ses isostères ou un de ses analogues, et
   - R désigne l'arginine ou un de ses isostères ou un de ses analogues.

L'invention résulte notamment de l'identification, par des stratégies de comparaison de la séquence de peptides ligands, de motifs assurant la liaison et le transport par le LDLR mais sans compétition avec le ligand naturel. L'absence de compétition avec le ligand naturel montre que les motifs de liaison découverts par les inventeurs impliquent un nouveau site de liaison sur le LDLR, ce qui constitue une découverte inattendue et particulièrement avantageuse dans le cadre de la vectorisation de composés *in vivo.*

Les acides aminés, naturels ou non, constitutifs des groupes X et Y des peptides ou pseudo-peptides de l'invention peuvent être identiques ou différents et choisis parmi :
- la glycine (Gly, G) ou acide 2-aminoéthanoïque, la sarcosine (Sar) ou N-méthylglycine (MeGly), la N-éthylglycine (EtGly), l'allylglycine (allylGly) ou acide 2-aminopent-4-énoïque, la 2-cyclopentylglycine (Cpg), la 2-cyclohexylglycine (Chg), la 2,2-dipropylglycine (Dpg), la 2-(3-indolyl)glycine (IndGly), la 2-indanylglycine (Igl), la 2-néopentylglycine (NptGly), la 2-octylglycine (OctGly), la 2-propargylglycine (Pra) ou acide 2-amino pent-4-ynoïque, la 2-phénylglycine (Phg), la 2-(4-chlorophényl)glycine, l'azaglycine (AzGly), le glycinol ou 2-aminoéthanol,
- l'alanine (Ala, A) ou acide 2-aminopropanoïque, la bêta-alanine (β-Ala) ou acide 3-aminopropanoïque, la déshydroalanine, la N-méthylalanine, la 3-cyclopropylalanine (Cpa), la 3-cyclohexylalanine (Cha), la 3-cyclopentylalanine,
- la 3-(1-naphtyl)alanine (1Nal), la 3-(2-naphtyl)alanine (2Nal), la 3-(3-pyridyl)alanine, la 3-(2-thiényl)alanine (Thi), l'alaninol ou 2-aminopropanol,
- la valine (Val, V) ou acide 2-amino-3-méthylbutanoïque, la N-méthylvaline (MeVal), la norvaline (Nva) et ses dérivés méthylés et/ou hydroxylés, la 5-hydroxynorvaline (Hnv) et ses dérivés, la 3-mercaptovaline (pénicillamine, Pen), le valinol ou 2-amino-3-méthylbutanol,
- la leucine (Leu, L) ou acide 2-amino-4-méthylpentanoïque, la norleucine (Nle) ou acide 2-aminohexanoïque, la 3-hydroxyleucine, la 6-hydroxynorleucine, la tert-leucine (Tle) ou acide 2-amino-3,3-diméthylbutanoïque, l'homoleucine ou acide 3-amino-5-méthylhexanoïque, la 2,3-déshydroleucine, le leucinol ou 2-amino-4-méthylpentanol,
- l'isoleucine (Ile, I) ou acide 2-amino-3-méthylpentanoïque, l'allo-isoleucine (alle), la N-méthylisoleucine (Melle), l'isoleucinol ou 2-amino-3-méthylpentanol,
- l'acide aspartique (Asp, D) ou acide 2-aminobutanedioïque et ses dérivés estérifiés ou amidés latéralement, l'acide 3-méthylaspartique, l'aspartinol,
- l'asparagine (Asn, N) ou acide 2-amino-3-carbamoylpropanoïque ou acide 2-aminosuccinamique et ses dérivés N-substitués, la N-éthylasparagine (EtAsn), l'asparaginol,
- l'acide glutamique (Glu, E) ou acide 2-aminopentanedioïque et ses dérivés estérifiés ou amidés latéralement, l'acide pyroglutamique (Pyr) ou acide pidolique ou 5-oxoproline, l'acide gamma-carboxyglutamique ou acide 4-carboxyglutamique (Gla), le glutarinol,
- la glutamine (Gln, Q) ou acide 2-amino-4-carbamoylbutanoïque et ses dérivés N-substitués, le glutaminol,
- l'acide diaminoéthanoïque, l'acide 2,3-diaminopropanoïque (Dpr ou Dap), l'acide 3-mercaptopropanoïque (Mpa), l'acide 2-amino-3-guanidinopropanoïque (Agp), l'acide 2-aminobutanoïque (Abu), l'acide 4-aminobutanoïque (4Abu) ou GABA, l'acide 2-aminoisobutanoïque (Aib), l'acide 3-aminoisobutanoïque (bAib), l'acide 2,4-diaminobutanoïque (Dab), l'acide 3,4-diaminobutanoïque (Dbu), l'acide 2-amino-4-cyanobutanoïque (Cba), l'acide 2-amino-4-guanidinobutanoïque (Agb), l'acide 5-aminopentanoïque (Ava), l'acide 4-amino-3-hydroxy-5-phénylpentanoïque (AHPPA), l'acide 4-amino-5-cyclohexyl-3-hydroxypentanoïque (ACHPA), l'acide 6-aminohexanoïque (Acp ou Ahx), l'acide para-aminobenzoïque (PABA), l'acide méta-aminométhylbenzoïque ou acide 3-aminométhylbenzoïque, l'acide para-aminométhylbenzoïque (PAMBA) ou acide 4-aminométhylbenzoïque, l'acide 2-aminoadipique (Aad) ou acide 2-aminohexanedioïque, l'acide 3-aminoadipique (bAad) ou acide 3-aminohexanedioïque, l'acide 2-aminopimélique (Apm) ou acide 2-amino-heptanedioïque (Ahe), l'acide 4-amino-3-hydroxy-6-méthylheptanoïque ou statine (Sta), l'acide 2,2-diaminopimélique (Dpm) ou acide 2,2-diamino-heptanedioïque, la desmosine (Des) ou acide 2-amino-6-[4-(4-amino-4-carboxybutyl)-3,5-bis-(3-amino-3-carboxypropyl)-pyridin-1-yl]hexanoïque, l'isodesmosine (Ide) ou acide 2-amino-6-[2-(4-amino-4-carboxybutyl)-3,5-bis-(3-amino-3-carboxypropyl)-pyridin-1-yl]hexanoïque,
- l'ornithine (Orn) ou acide 2,5-diaminopentanoïque et ses dérivés epsilon-aminés ou amidés, la canaline ou acide 2-amino-4-(aminooxy)butanoïque, l'ornithinol,
- la lysine (Lys, K) ou acide 2,6-diaminohexanoïque et ses dérivés epsilon-aminés ou amidés, l'homolysine ou acide 2,7-diaminoheptanoïque (hLys), la 5-hydroxylysine (Hyl) ou acide 2,6-diamino-5-hydroxyhexanoïque, l'allo-hydroxylysine (aHyl), la 6-N-méthyllysine (MeLys), la S-aminoéthylcystéine ou acide 2-amino-3-(2-aminoéthylthio)propanoïque, la 3-méthyl-S-aminoéthylcystéine ou acide 2-amino-3-(2-aminoéthylthio)butanoïque, le lysinol,
- l'arginine (Arg, R) ou acide 2-amino-5-guanidinopentanoïque ou acide 2-amino-5-(diaminométhylidène amino)pentanoïque, l'homoarginine ou acide 2-amino-6-guanidinohexanoïque, la N-hydroxyarginine, la citrulline (Cit) ou acide 2-amino-5-(carbamoylamino)pentanoïque, l'acide 2-amino-5-(4-carbamimidoylphényl)pentanoïque, l'acide 2-amino-5-(1*H*-imidazol-2-ylamino)pentanoïque, la canavanine ou acide 2-amino-4-(guanidinooxy)butanoïque, l'argininol,
- l'histidine (His, H) ou acide 2-amino-3-(1H-imidazol-4-yl)propanoïque et ses dérivés N-substitués, l'histidinol,
- la sérine (Ser, S) ou acide 2-amino-3-hydroxypropanoïque et ses dérivés O-substitués (éthers, etc.), l'acide 2-amino-4-hydroxybutanoïque ou homosérine (Hse) et ses dérivés O-substitués (éthers, etc.), le sérinol ou 2-amino-propan-1,3-diol,
- la thréonine (Thr, T) ou acide 2-amino-3-hydroxybutanoïque et ses dérivés O-substitués (éthers, etc.), l'allo-thréonine (allo-Thr) et ses dérivés O-substitués (éthers, etc.), le thréoninol (Thol),
- la phénylalanine (Phe, F) ou acide 2-amino-3-phénylpropanoïque, la 2-fluorophénylalanine, la 3-fluorophénylalanine, la 4-fluorophénylalanine, la 3,4-difluorophénylalanine, la pentafluorophénylalanine, la 2-chlorophénylalanine, la 3-chlorophénylalanine, la 4-chlorophénylalanine, la 3,4-dichlorophénylalanine, la 4-bromophénylalanine, la 3-iodophénylalanine, la 4-iodophénylalanine, la 4-nitrophénylalanine, la 2-méthoxyphénylalanine, la 3-méthoxyphénylalanine, la 4-méthylphénylalanine, la 4-aminophénylalanine, la 4-guanidinophénylalanine, la 3,4-dihydroxyphénylalanine ou DOPA, l'acide 2-amino-4-phénylbutanoïque ou homophénylalanine, la 4-biphénylylalanine (Bip), la 4-benzoylphénylalanine (Bpa), le phénylalaninol (Phol),
- la tyrosine (Tyr, Y) ou acide 2-amino-3-(p-hydroxyphényl)propanoïque et ses dérivés O-substitués (éthers, etc.), la 3-iodotyrosine, la 3,5-diiodotyrosine, la 2-bromotyrosine, la 3,5-dibromotyrosine, la 2,6-diméthyltyrosine, la 3-nitrotyrosine, la 3-sulfotyrosine, le tyrosinol ou 2-amino-4-hydroxybenzènepropanol,
- le tryptophane (Trp, W) ou acide 2-amino-3-(1H-indol-3-yl)propanoïque et ses dérivés N-substitués, le 2-méthyltryptophane, le 5-hydroxytryptophane (5-HTP), le tryptophanol,
- la cystéine (Cys, C) ou acide 2-amino-3-mercaptopropanoïque et ses dérivés S-substitués, la S-acétylcystéine ou acide 2-amino-3-(acétylthio)propanoïque, la sélénocystéine (Sec, U) ou acide 2-amino-3-(séléno)propanoïque, le cystéinol,
- la méthionine (Met ou M) ou acide 2-amino-4-(méthylthio)butanoïque, l'homométhionine ou acide 3-amino-5-(méthylthio)pentanoïque, le méthioninol,
- la proline (Pro, P) ou acide pyrolidine-2-carboxylique, l'homoproline ou acide 2-(2-pyrrolidinyl)éthanoïque, la 3-hydroxyproline (3Hyp), la 4-hydroxyproline (4Hyp), la 3-méthylproline, la 3,4-déshydroproline, la 3,4-méthanoproline, la 4-aminoproline, la 4-oxoproline, la thioproline ou acide thiazolidine-4-carboxylique (Thz), l'acide 2-oxothiazolidine-4-carboxylique, l'acide indoline-2-carboxylique (Idc), l'acide pipécolique (Pip) ou acide piperidine-2-carboxylique, l'acide nipécotique (Nip) ou acide piperidine-3-carboxylique, l'acide 4-oxopipécolique, l'acide 4-hydroxypipécolique, l'acide amino-1-cyclohexane carboxylique, le prolinol.

Comme indiqué, on préfère des peptides comprenant au moins 1, de préférence 2 résidus d'acides aminés pouvant permettre la cyclisation du peptide. De tels acides aminés sont typiquement choisis parmi Cys, Mpa, Pen, la déshydroalanine, ou allylGly.

La cyclisation est typiquement obtenue par formation d'un pont disulfure entre deux résidus cystéine (ou Pen), l'une présente dans le groupe X, l'autre dans le groupe Y. Une cystéine en positon N-terminale (N-term) peut par ailleurs être remplacée par un Mpa pour la cyclisation via un pont disulfure.

Un résidu cystéine peut également être remplacé par la déshydroalanine, pour la cyclisation via un pont lanthionine, ou par l'allylGly pour une cyclisation par métathèse via un pont dicarba.

Un pont lactame peut-être créé entre la fonction acide latérale d'un résidu Glu (ou Asp) et une fonction amine latérale sur une Lys ou une amine N-term. De même une cyclisation entre la fonction amine N-term et la fonction acide C-terminale ou C-term (tête/queue) peut être réalisée via une liaison amide, tout comme une cyclisation entre la fonction amine latérale d'une Lys et la fonction acide C-term du peptide.

De préférence, dans les peptides ou pseudo-peptides de formule (I), l'un au moins des groupes X et Y contient un résidu cystéine.

Dans un mode de mise en oeuvre particulièrement préféré, les résidus Z et W représentent chacun une cystéine.

Comme indiqué, M représente un résidu méthionine, ou l'un de ses isostères ou l'un de ses analogues. Les isostères ou analogues de la méthionine sont choisis de préférence parmi Nle, l'homométhionine, Pen, Mpa.

Comme indiqué, P représente un résidu proline, ou l'un de ses isostères ou l'un de ses analogues. Les isostères ou analogues de la proline sont choisis de préférence parmi les isostères cyclopentèniques, la 3,4-déshydroproline, la 3,4-méthanoproline, l'homoproline, 3Hyp, 4Hyp, la 3-méthylproline, la 4-aminoproline, la 4-oxoproline, Thz, l'acide 2-oxothiazolidine-4-carboxylique, Idc, Pip, Nip, l'acide 4-oxopipécolique, l'acide 4-hydroxypipécolique, l'acide amino-1-cyclohexane carboxylique.

Comme indiqué, R représente un résidu arginine, ou l'un de ses isostères ou l'un de ses analogues. Les isostères ou analogues de l'arginine sont choisis de préférence parmi l'homoarginine, la N-hydroxyarginine, Agp, Agb, Cit, l'acide 2-amino-5-(4-carbamimidoylphényl)pentanoïque, l'acide 2-amino-5-(1*H*-imidazol-2-ylamino)pentanoïque, Orn, Lys et ses isostères/analogues, la S-aminoéthylcystéine, la 3-méthyl-S-aminoéthylcystéine, hLys, Hyl, aHyl, MeLys, His et ses isostères/analogues, Nle, l'acide diaminoéthanoïque, Dpr, Dbu.

Parmi les peptides ou pseudo-peptides de formule (I), on préfère tout particulièrement ceux dans lesquels :
- i est un entier choisi parmi 0, 1, 3 ou 4 ; et/ou
- j est un entier choisi parmi 0, 3 ou 4 ; et/ou
- k est un entier choisi parmi 0, 1 ou 3 ; et/ou
- l est un entier choisi parmi 0, 1 ou 3.

Des peptides ou pseudo-peptides préférés au sens de l'invention répondent à la formule générale (Xaa)i-Cys-(Xaa)j-Met-Pro-Arg-(Xaa)k-Cys-(Xaa)l, dans laquelle Xaa désigne tout résidu d'acide aminé naturel ou non naturel et i, j, k et 1 sont des nombres entiers, identiques ou différents, compris entre 0 et 5. De préférence, i = 0, 1, 3 ou 4 ; j = 0, 3 ou 4 ; k = 0, 1 ou 3 ; et/ou 1 = 0, 1 ou 3.

Des exemples particuliers de peptides selon l'invention répondant à la formule (I) sont décrits dans les séquences SEQ ID NO : 1 à SEQ ID NO : 11 et SEQ ID NO : 30 à SEQ ID NO : 65 suivantes :
SEQ ID NO : 1, HLDCMPRGCFRN ;
SEQ ID NO : 2, ACQVKSMPRC ;
SEQ ID NO : 3, ACTTPMPRLC ;
SEQ ID NO : 4, ACKAPQMPRC ;
SEQ ID NO : 5, ACLNPSMPRC ;
SEQ ID NO : 6, ACLVSSMPRC ;
SEQ ID NO : 7, ACLQPMPRLC ;
SEQ ID NO : 8, ACPVSSMPRC ;
SEQ ID NO : 9, ACQSPMPRLC ;
SEQ ID NO : 10, ACLTPMPRLC ;
SEQ ID NO : 11, DSGLCMPRLRGCDPR ;
SEQ ID NO : 30, ACMPRLRGCA ;
SEQ ID NO : 31, ASGLCMPRLRGCDPR ;
SEQ ID NO : 32, DAGLCMPRLRGCDPR ;
SEQ ID NO : 33, DSALCMPRLRGCDPR ;
SEQ ID NO : 34, DSGACMPRLRGCDPR ;
SEQ ID NO : 35, DSGLCMPRARGCDPR ;
SEQ ID NO : 36, DSGLCMPRLAGCDPR ;
SEQ ID NO : 37, DSGLCMPRLRACDPR ;
SEQ ID NO : 38, DSGLCMPRLRGCAPR ;
SEQ ID NO : 39, DSGLCMPRLRGCDAR ;
SEQ ID NO : 40, DSGLCMPRLRGCDPA ;
SEQ ID NO : 41, SGLCMPRLRGCDPR ;
SEQ ID NO : 42, GLCMPRLRGCDPR ;
SEQ ID NO : 43, CMPRLRGC ;
SEQ ID NO : 44, CMPRARGC ;
SEQ ID NO : 45, CMPRLAGC ;
SEQ ID NO : 46, CMPRLRAC ;
SEQ ID NO : 47, CMPRLKGC ;
SEQ ID NO : 48, (D)-CMPRLRGC ;
SEQ ID NO : 49, CMPR-(D)-LRGC ;
SEQ ID NO : 50, CMPRL-(D)-RGC ;
SEQ ID NO : 51, CMPRLRG-(D)-C ;
SEQ ID NO : 52, (D)-CMPRLRG-(D)-C ;
SEQ ID NO : 53, (D)-CMPRLRSarC ;
SEQ ID NO: 54, (D)-CMPRKRGC ;
SEQ ID NO : 55, (D)-CMPRLRCG ;
SEQ ID NO : 56, (D)-CMPRLCRG ;
SEQ ID NO : 57, (D)-CMPRCLRG ;
SEQ ID NO : 58, CMPRGC ;
SEQ ID NO : 59, PenMPRLRGC ;
SEQ ID NO : 60, (D)-PenMPRLRGC ;
SEQ ID NO : 61, (D)-CMPRLRGPen ;
SEQ ID NO : 62, PenMPRLRGPen ;
SEQ ID NO : 63, (D)-PenMPRLRGPen ;
SEQ ID NO : 64, (D)-PenMPRLRG-(D)-Pen ;
SEQ ID NO : 65, MpaMPRLRGC.

Comme illustré dans la section expérimentale, ces peptides lient le LDLR sans compétition avec le LDL, présentent une affinité élevée, sont capables de traverser la BHE et de véhiculer des molécules d'intérêt.

Dans un mode particulièrement préféré de mise en oeuvre, on préfère les peptides de formule (I) ayant une configuration cyclique. On préfère tout particulièrement le peptide comprenant la séquence SEQ ID NO : 11, ou une séquence dérivée de celle-ci, par exemple SEQ ID NO : 30 et 48.

Selon une autre variante, les peptides ou pseudo-peptides de l'invention sont choisis parmi les peptides de séquence SEQ ID NO : 12 à SEQ ID NO : 29 suivantes :
SEQ ID NO : 12, MTVMPTGLWNPLIPS ;
SEQ ID NO : 13, SASWFAVPIPPLRLE ;
SEQ ID NO : 14, MTPMSTPRMLPVYVA ;
SEQ ID NO : 15, MTATHLSTLFQPLTY ;
SEQ ID NO : 16, MSPIPPAASTWANTL ;
SEQ ID NO : 17, MTANPLQNAPGPLSL ;
SEQ ID NO : 18, MQTAPPPPLTRVQWS ;
SEQ ID NO : 19, GTPRMHIPLNVDHLP ;
SEQ ID NO : 20, LTLPPISGLSSYPLP ;
SEQ ID NO : 21, TPSAHAMALQSLSVG ;
SEQ ID NO : 22, LTLPPISGLSSYPLP ;
SEQ ID NO : 23, MGTLNAPTAYPQDSL ;
SEQ ID NO : 24, LTNPPAYLPQNTDPH ;
SEQ ID NO : 25, MGLPLPYIQTILHTP ;
SEQ ID NO : 26, SAALIAMSSFKSITA ;
SEQ ID NO : 27, SGFAFARSVPTESRR ;
SEQ ID NO : 28, MTSPYMSLPPSTDDM ;
SEQ ID NO : 29, LTNPPAYLPQNTDPH.

Comme indiqué précédemment, les peptides ou pseudo-peptides linéaires ou cycliques de l'invention peuvent comprendre des liaisons peptidiques, non-peptidiques et/ou peptidiques modifiées. Dans un mode préféré, les peptides ou pseudo-peptides comprennent au moins une liaison peptidomimétique, choisie de préférence parmi l'intercalation d'un groupe méthylène (-CH₂-) ou phosphate (-PO₂-), amine secondaire (-NH-) ou oxygène (-O-), des alpha-azapeptides, alpha-alkylpeptides, N-alkylpeptides, phosphonamidates, depsipeptides, hydroxyméthylènes, hydroxyéthylènes, dihydroxyéthylènes, hydroxyéthylamines, retro-inverso, méthylèneoxy, cétométhylène, esters, phosphinates, phosphiniques, phosphonamides, analogues carba.

Par ailleurs, dans un mode particulier de mise en oeuvre, les peptides ou pseudo-peptides de l'invention comprennent une fonction N-term et/ou C-term respectivement protégée, par exemple, par une acylation, ou par une amidation ou une estérification.

Les peptides ou pseudo-peptides de l'invention peuvent être synthétisés par toute technique connue en soi de l'homme du métier (synthèse chimique, biologique, génétique, etc.). Ils peuvent être conservés tels quels, ou formulés en présence d'une substance d'intérêt ou de tout excipient acceptable.

Pour les synthèses chimiques, sont utilisés des appareils commerciaux permettant d'incorporer aussi bien des acides aminés naturels que non naturels, tels que les énantiomères D et des résidus ayant des chaînes latérales avec des hydrophobicités et des encombrements stériques différents de ceux de leurs homologues naturels (acides aminés dits exotiques c'est-à-dire non codés), ou une séquence peptidique contenant une ou plusieurs liaisons peptidomimétiques pouvant inclure notamment l'intercalation d'un groupe méthylène (-CH₂-) ou phosphate (-PO₂-), d'une amine secondaire (-NH-) ou d'un oxygène (-O-).

Au cours de la synthèse, il est possible d'introduire diverses modifications chimiques, comme par exemple, mettre en N-term, C-term ou sur une chaîne latérale un dérivé lipidique (ou phospho lipidique) ou un constituant d'une nanoparticule, de façon à pouvoir incorporer le peptide ou pseudo-peptide de l'invention au sein d'une membrane lipidique telle que celle d'un liposome constitué d'une ou plusieurs couches ou bicouches lipidiques, ou d'une nanoparticule.

On peut également obtenir les peptides de l'invention, ou partie de ceux-ci de nature protéique à partir d'une séquence d'acide nucléique codant pour celui-ci. La présente invention a aussi pour objet une molécule d'acide nucléique comprenant, ou constituée par, une séquence nucléique codant pour un peptide tel que défini ci-avant. Plus particulièrement l'invention concerne une molécule d'acide nucléique comprenant au moins une séquence codant pour un composé de formule générale (I) ou correspondant à l'une des séquences SEQ ID NO : 12 à SEQ ID NO : 29 ou une partie de celui-ci de nature protéique. Ces séquences d'acides nucléiques peuvent être des ADN ou ARN et être associées à des séquences de contrôle et/ou être insérées dans des vecteurs d'expression biologiques.

Le vecteur d'expression biologique utilisé est choisi en fonction de l'hôte dans lequel il sera transféré. Il peut s'agir par exemple d'un plasmide, cosmide, virus, etc. Ces acides nucléiques et vecteurs d'expression biologiques constituent des objets particuliers de l'invention, et sont utiles pour produire les peptides de l'invention, ou partie de ceux-ci de nature protéique, dans un hôte cellulaire. La préparation de ces vecteurs d'expression biologiques ainsi que la production ou l'expression dans un hôte des peptides peuvent être réalisées par les techniques de biologie moléculaire et de génie génétique bien connues de l'homme de l'art.

Comme indiqué, les peptides de l'invention sont particulièrement utiles pour formuler des substances d'intérêt thérapeutique ou diagnostique, et notamment pour favoriser leur biodistribution et/ou leur passage à travers la BHE.

A cet égard, un objet de l'invention réside dans l'utilisation d'un peptide ou pseudo-peptide linéaire ou cyclique tel que défini précédemment, comme vecteur pour le transfert/transport de molécules d'intérêt thérapeutique, ou d'agents d'imagerie ou de diagnostic, ou de toute autre molécule.

Un autre objet de l'invention réside dans l'utilisation d'un peptide ou pseudo-peptide linéaire ou cyclique tel que défini précédemment pour la préparation d'un médicament capable de traverser la BHE.

Un autre objet de l'invention concerne une méthode pour permettre ou améliorer le passage d'une molécule à travers la BHE, comprenant le couplage de la molécule à un peptide ou pseudo-peptide de l'invention.

Les peptides ou pseudo-peptides linéaires ou cycliques de l'invention permettent de faire traverser la BHE à une substance active qui ne passe pas ou peu cette barrière. Ils peuvent donc être utilisés dans le traitement, la prévention ou le diagnostic de toute maladie affectant le SNC, mais aussi comme transporteurs de matériel biologique (biotransporteurs) dans le cadre d'études menées sur diverses familles de molécules avec des modèles de membranes cellulaires et plus particulièrement de BHE.

A cet égard, la présente demande décrit différents composés conjugués prodrogues comprenant un peptide ou pseudo-peptide tel que défini précédemment. Le terme « conjugué » désigne une molécule résultant de la combinaison entre un ou plusieurs peptides ou pseudo-peptides de l'invention et une ou plusieurs molécules d'intérêt. Comme il sera décrit plus en détails dans la suite du texte, la conjugaison peut être de nature chimique, comme par le biais d'un bras espaceur (*linker* ou *spacer*), ou de nature génétique, comme par exemple la technologie de recombinaison génétique, telle que dans une protéine de fusion avec par exemple une molécule marqueur ou traceur (par exemple, la GFP, la β-galactosidase, etc.) ou une molécule thérapeutique (par exemple un facteur de croissance, facteur neurotrophique, etc.).

Ainsi, un objet particulier de l'invention concerne un composé conjugué de formule (II) suivante :

VxDy (II)

dans laquelle V représente un peptide ou pseudo-peptide linéaire ou cyclique de l'invention, D représente une substance active ou d'intérêt, et x et y sont des nombres entiers compris entre 1 et 5. Dans un mode particulier, x et y sont égal à 1, ou x est supérieur à y.

A titre d'exemple, le conjugué pour lequel V = cMPRLRGC, x =1, D = Y-(D)-AGFLR et y = 1 a été synthétisé. Dans le cas présent, la substance active est un peptide thérapeutique analgésique, la dalargine. Le couplage direct (synthèse en tandem) de ce peptide thérapeutique en N-term du peptide vecteur SEQ ID NO : 48 donne le conjugué SEQ ID NO : 66, Y-(D)-AGFLR-(D)-CMPRLRGC.

Un autre objet particulier de l'invention concerne un composé conjugué de formule (III) suivante :

VxLzDy (III)

dans laquelle V représente un peptide ou pseudo-peptide linéaire ou cyclique de l'invention, L représente un bras espaceur, D représente une substance active ou d'intérêt, x et y sont des nombres entiers compris entre 1 et 5 et z est un entier compris entre 1 et 10. Dans un mode particulier, x=z=y=1 ou x=z>y ou z>x>y.
A titre d'exemple, les synthèses via *linkers* des conjugués pour lequel V = cMPRLRGC (avec x =1) et D = Y-(D)-AGFLR (avec y = 1) ont été réalisées avec L = GGG ou GFLG ou ALAL ou β-Ala ou Ahx ou GFAL (avec z =1).
SEQ ID NO : 67, Y-(D)-AGFLRGGG-(D)-CMPRLRGC
SEQ ID NO : 68, Y-(D)-AGFLRGFLG-(D)-CMPRLRGC
SEQ ID NO : 69, Y-(D)-AGFLRALAL-(D)-CMPRLRGC
SEQ ID NO : 70, Y-(D)-AGFLR-β-Ala-(D)-CMPRLRGC
SEQ ID NO : 71, Y-(D)-AGFLR-Ahx-(D)-CMPRLRGC
SEQ ID NO : 72, Y-(D)-AGFLRGFAL-(D)-CMPRLRGC

Les exemples contenus dans la présente demande montrent, dans des expériences de perfusion cérébrale chez la souris, que ces conjugués sont capables de passer la BHE, illustrant le mécanisme d'action des peptides vecteurs de l'invention.

La substance active ou d'intérêt peut être toute molécule d'intérêt pharmaceutique, notamment thérapeutique, un agent de diagnostic ou d'imagerie médicale, ou une sonde moléculaire. Il peut s'agir en particulier de toute entité chimique ayant un intérêt biologique telle qu'une petite molécule chimique (antibiotique, antiviral, immuno-modulateur, anticancéreux, anti-inflammatoire, etc.), un peptide ou polypeptide, une protéine (enzyme, hormone, cytokine, apolipoprotéine, facteur de croissance, antigène, anticorps ou une partie d'anticorps), un acide nucléique (acide ribonucléique ou acide désoxyribonucléique d'origine humaine, virale, animale, eucaryote ou procaryote, végétale, synthétique, etc., pouvant avoir une taille variable, allant du simple oligonucléotide au génome ou fragment de génome), un génome viral ou un plasmide, un ribozyme, un marqueur ou traceur. De manière générale, la « substance d'intérêt » peut être tout principe actif de médicament, qu'il s'agisse d'un produit chimique, biochimique, naturel ou synthétique. L'expression « petite molécule chimique » désigne une molécule d'intérêt pharmaceutique ayant un poids moléculaire de 1000 Daltons au maximum, typiquement compris entre 300 et 700 Daltons.

Un autre objet particulier de l'invention concerne un composé de formule (IV) suivante :

VxLz (IV)

dans laquelle V représente un peptide ou pseudo-peptide linéaire ou cyclique de l'invention, L représente un bras espaceur, x est un nombre entier compris entre 1 et 5 et z est un entier compris entre 1 et 10. Dans un mode particulier, x=z=1 ou z>x.

Dans les composés conjugués de l'invention, le couplage entre V et D, ou entre V et L d'une part et entre L et D d'autre part, peut être réalisé par tout moyen de liaison acceptable compte tenu de la nature chimique, de l'encombrement et du nombre de substance(s) active(s) et de peptide(s) ou pseudo-peptide(s) associés. Le couplage peut ainsi être réalisé par une ou plusieurs liaisons covalentes, hydrophobes ou ioniques, clivables ou non en milieu physiologique ou à l'intérieur de cellules. Par ailleurs, D peut être couplé à V, le cas échéant via L, au niveau de différents groupes réactifs, et notamment d'une ou des extrémités N-term et/ou C-term de V et/ou au niveau d'un ou plusieurs groupements réactifs portés par les chaînes latérales des acides aminés naturels ou non, constitutifs de V.

Le couplage peut être effectué en n'importe quel site du peptide ou pseudo-peptide, dans lequel des groupements fonctionnels tels que -OH, -SH, -CO₂H, -NH₂, -SO₃H,-PO₂H sont naturellement présents ou ont été introduits. Ainsi, une molécule d'intérêt thérapeutique, ou un agent de diagnostic (ou d'imagerie médicale) ou toute autre molécule comme une sonde moléculaire peut être liée (couplée) au vecteur peptidique ou pseudo-peptidique linéaire ou cyclique de l'invention par une liaison covalente soit au niveau des extrémités N-term ou C-term, soit au niveau des groupements réactifs portés par les chaînes latérales des acides aminés naturels ou non, de cette séquence peptidique.

De même, le couplage peut être effectué en n'importe quel site de la substance active ou d'intérêt (molécule d'intérêt thérapeutique, agent de diagnostic ou d'imagerie médicale, toute autre molécule comme une sonde moléculaire), où par exemple des groupements fonctionnels tels que -OH, -SH, -CO₂H, -NH₂, -SO₃H, -PO₂H sont naturellement présents ou ont été introduits.

Il est préférable que l'interaction soit suffisamment solide pour que le peptide ne se dissocie pas de la substance active avant d'avoir atteint son site d'action. Pour cette raison, le couplage préféré selon l'invention est un couplage covalent, mais il pourrait cependant s'agir d'un couplage non covalent. La substance d'intérêt peut être couplée directement au peptide (synthèse en tandem) soit à l'une de ces extrémités terminales (N-term ou C-term), soit au niveau d'une chaîne latérale d'un des acides aminés constitutifs de la séquence (Figure 2). La substance d'intérêt peut aussi être couplée indirectement par le biais d'un bras de liaison ou bras espaceur (synthèse via *linker*), soit à l'une des extrémités terminales des peptides, soit au niveau d'une chaîne latérale d'un des acides aminés constitutifs de la séquence (Figure 2). On peut citer comme moyen de couplage chimique covalent, faisant appel ou pas à un bras espaceur, ceux choisis parmi des agents bi- ou multifonctionnels contenant des groupements alkyle, aryle ou peptidique par des esters, aldéhydes ou acides d'alkyle ou d'aryle, des groupements anhydrides, sulfhydriles ou carboxyles, des groupes dérivés du bromure ou chlorure de cyanogène, du carbonyldiimidazole, des esters de succinimide ou des halogénures sulfoniques.

A cet égard, un objet de l'invention réside également dans un procédé de préparation d'un composé conjugué tel que défini ci-avant, caractérisé en ce qu'il comprend une étape de couplage entre un peptide ou pseudo-peptide V et une substance D, le cas échéant via L, de préférence par voie chimique, biochimique, enzymatique, ou par génie génétique.

Un autre objet de l'invention concerne une composition pharmaceutique caractérisée en ce qu'elle comprend au moins un composé conjugué tel que défini ci-avant et un ou des excipients pharmaceutiquement acceptables.

Un autre objet de l'invention concerne une composition diagnostique caractérisée en ce qu'elle comprend un agent de diagnostic ou d'imagerie médicale constitué d'un composé conjugué tel que défini ci-avant.

Le conjugué peut être utilisé sous forme de tout sel pharmaceutiquement acceptable. Par sels pharmaceutiquement acceptables, on entend par exemple et de manière non limitative des sels d'addition basique ou acide pharmaceutiquement acceptables, hydrates, esters, solvates, précurseurs, métabolites ou stéréoisomères, desdits vecteurs ou conjugués chargés avec au moins une substance d'intérêt.

L'expression sels pharmaceutiquement acceptables se réfère aux sels non toxiques, qui peuvent être généralement préparés en faisant réagir une base libre avec un acide organique ou inorganique convenable. Ces sels conservent l'efficacité biologique et les propriétés des bases libres. Comme exemples représentatifs de tels sels, on peut citer les sels hydrosolubles et hydroinsolubles, tels que les acétates, N-méthylglucamine ammonium, ansonates (4,4-diaminostilbènes-2,2'-disulfonates), benzènesulfonates, benzonates, bicarbonates, bisulfates, bitartrates, borates, bromhydrates, bromures, buryrates, camsylates, carbonates, chlorhydrates, chlorures, citrates, clavulariates, dichlorhydrates, diphosphates, édétates, édétates de calcium, édisylates, estolates, ésylates, fumarates, gluceptates, gluconates, glutamates, glycolylarsanylates, hexafluorophosphates, hexylrésorcinates, hydrabamines, hydroxynaphtoates, iodures, isothionates, lactates, lactobionates, laurates, malates, maléates, mandélates, mésylates, méthylbromures, méthylnitrates, méthylsulfates, mucates, napsylates, nitrates, 3-hydroxy-2-naphtoates, oléates, oxalates, palmitates, pamoates (1,1-méthylène-bis-2-hydroxy-3-naphtoates, ou emboates), pantothénates, phosphates, picrates, polygalacturonates, propionates, p-toluènesulfonates, salicylates, stéarates, subacétates, succinates, sulfates, sulfosalicylates, suramates, tannates, tartrates, téoclates, tosylates, triéthiodides, trifluoroacétates, valérates.

Les compositions de l'invention comprennent avantageusement un véhicule ou excipient pharmaceutiquement acceptable. Le véhicule pharmaceutiquement acceptable peut être choisi parmi les véhicules utilisés de manière classique selon chacun des modes d'administration. En fonction du mode d'administration prévu, les composés peuvent être sous forme solide, semi-solide ou liquide. Pour les compositions solides, telles que comprimés, pilules, poudres ou granulés à l'état libre ou inclus dans des gélules, la substance active peut être combinée avec : a) des diluants, par exemple le lactose, le dextrose, le sucrose, le mannitol, le sorbitol, la cellulose et/ou la glycine ; b) des lubrifiants, par exemple la silice, le talc, l'acide stéarique, son sel de magnésium ou de calcium et/ou le polyéthylène glycol ; c) des liants, par exemple le silicate de magnésium et d'aluminium, la pâte d'amidon, la gélatine, la gomme adragante, la méthylcellulose, la carboxyméthylcellulose sodique et/ou la polyvinylpyrrolidone ; d) des désintégrants, par exemple l'amidon, l'agar, l'acide alginique ou son sel de sodium, ou des mélanges effervescents ; et/ou e) des absorbants, colorants, aromatisants et édulcorants. Les excipients peuvent être par exemple du mannitol, lactose, amidon, stéarate de magnésium, saccharine sodique, talc, cellulose, glucose, sucrose, carbonate de magnésium et analogues de qualité pharmaceutique. Pour les compositions semi-solides, telles que suppositoires, l'excipient peut, par exemple, être une émulsion ou suspension grasse, ou à base de polyalkylène glycol, tel que le polypropylène glycol. Les compositions liquides, en particulier injectables ou à inclure dans une capsule molle, peuvent être préparées par exemple par dissolution, dispersion, etc. de la substance active dans un solvant pharmaceutiquement pur tel que, par exemple, l'eau, le sérum physiologique, le dextrose aqueux, le glycérol, l'éthanol, une huile et ses analogues.

Les compositions ou conjugués de l'invention peuvent être administrés par toute voie adaptée et, de manière non limitative par la voie parentérale, comme par exemple, sous forme de préparations injectables par voie sous-cutanée, intraveineuse ou intramusculaire ; par voie orale (ou *per os*), comme par exemple, sous forme de comprimés enrobés ou non, de gélules, de poudres, de granulés, de suspensions ou de solutions orales (une telle forme pour l'administration par voie orale peut être soit à libération immédiate, soit à libération prolongée ou retardée) ; - voie rectale, comme par exemple, sous forme de suppositoires ; - voie topique, notamment transdermique, comme par exemple, sous la forme de patchs, de pommades ou de gels ; - voie intra-nasale, comme par exemple sous forme d'aérosols et de sprays ; - voie perlinguale ;-voie intraoculaire.

Les compositions pharmaceutiques comprennent typiquement une dose efficace d'un peptide ou pseudo-peptide ou conjugué de l'invention. Une « dose thérapeutiquement efficace » telle qu'elle est décrite ici s'entend comme la dose qui donne un effet thérapeutique pour une condition et un régime d'administration donnés. C'est typiquement la dose moyenne d'une substance active à administrer pour améliorer sensiblement certains des symptômes associés à une maladie ou à un état pathologique. Par exemple, dans le traitement d'un cancer cérébral ou non, d'une pathologie, d'une lésion ou d'un trouble du SNC, la dose d'une substance active qui diminue, empêche, retarde, supprime ou arrête l'une des causes ou l'un des symptômes de la maladie ou du trouble serait thérapeutiquement efficace.

Une « dose thérapeutiquement efficace » d'une substance active n'est pas tenue de guérir une maladie ou un trouble mais fournira un traitement pour cette maladie ou ce trouble de façon à ce que son apparition soit retardée, entravée ou empêchée, ou que ses symptômes soient atténués, ou que son terme soit modifié ou, par exemple, soit moins sévère ou que la récupération du patient soit accélérée.

Il est entendu que la « dose thérapeutiquement efficace » pour une personne en particulier dépendra de divers facteurs, incluant l'activité/efficacité de la substance active, son heure d'administration, sa voie d'administration, son taux d'excrétion et de métabolisation, les associations/interactions médicamenteuses et la sévérité de la maladie (ou du trouble) traitée à titre préventif ou curatif, ainsi que l'âge, le poids corporel, l'état de santé global, le sexe et/ou le régime alimentaire du patient.

Selon la substance couplée, les conjugués et compositions de l'invention peuvent être utilisés pour le traitement, la prévention, le diagnostic ou l'imagerie de nombreuses pathologies, notamment de pathologies affectant le SNC, de pathologies infectieuses ou de cancers.

A cet égard, l'invention concerne l'utilisation de conjugués ou compositions pharmaceutiques tels que décrits ci-avant pour le traitement ou la prévention de pathologies ou troubles du SNC, de tumeurs cancéreuses cérébrales ou d'autres cellules cancéreuses, et de pathologies infectieuses cérébrales ou autres, de type bactériennes, virales, parasitaires ou fongiques.

L'invention concerne également l'utilisation de conjugués ou compositions pharmaceutiques tels que décrits ci-avant pour le diagnostic ou l'imagerie de pathologies ou troubles du SNC, de tumeurs cancéreuses cérébrales ou d'autres cellules cancéreuses, et de pathologies infectieuses cérébrales ou autres, de type bactériennes, virales, parasitaires ou fongiques.

L'invention concerne également l'utilisation d'un conjugué ou d'une composition tels que définis ci-avant pour le traitement, l'imagerie et/ou le diagnostic d'une tumeur cancéreuse cérébrale ou d'un autre type de cellules cancéreuses. Des études ont en effet montré que des patients atteints de certains cancers présentent une hypocholestérolémie. Cette hypocholestérolémie est la conséquence d'une sur-utilisation du cholestérol par les cellules cancéreuses. Ces dernières induisent pour survivre une augmentation du niveau d'expression du LDLR au sein des organes tumoraux (Henricksson et al., 1989, Lancet, 2 (8673), 1178-1180). Il existe ainsi une corrélation entre l'augmentation du niveau d'expression du LDLR par les cellules et certains cancers. Il a également été récemment démontré que le nombre de LDLR était très important à la surface de certaines cellules pathologiques comme les cellules cancéreuses. Il est généralement admis que 1 000 à 3 000 LDLR sont présents à la surface d'une cellule non pathologique. De même, des neurones non pathologiques ne présentent qu'un faible nombre de LDLR (Pitas et al., 1987, J. Biol. Chem., 262, 14352-14360). Dans le cas du glioblastome, il a été démontré une surexpression du LDLR. Ainsi, il a été dénombré à la surface des cellules cérébrales tumorales de 125 000 (pour les cellules U-251) à 900 000 (pour les cellules SF-767) LDLR (Malentiska et al., 2000, Cancer Res., 60, 2300-2303 ; Nikanjam et al., 2007, Int. J. Pharm., 328, 86-94). Notons également que beaucoup de cellules tumorales surexpriment le LDLR, telles que celles du cancer de la prostate (Chen et al., 2001, Int. J. Cancer, 91, 41-45), du cancer du colon (Niendorf et al., 1995, Int. J. Cancer, 61, 461-464), des leucémies (Tatidis et al., 2002, Biochem. Pharmacol., 63, 2169-2180), du cancer colorectal (Caruso et al., 2001, Anticancer Res., 21, 429-433), du cancer du sein (Graziani et al., 2002, Gynecol. Oncol., 85, 493-497*),* ainsi que les cancers du foie, du pancréas, des ovaires, du poumon, de l'estomac, etc.

L'invention concerne également l'utilisation d'un conjugué ou d'une composition tels que définis ci-avant pour le traitement, l'imagerie et/ou le diagnostic de pathologies infectieuses cérébrales ou autres, de type bactériennes, virales, parasitaires ou fongiques, telles que, et de manière non limitative, le SIDA, voire les méningites, etc. Le LDLR est également présent sur les cellules hépatiques. Il est à présent connu que l'endocytose du virus de l'hépatite C (VHC) peut se faire par l'intermédiaire du LDLR. Le LDLR pourrait servir de récepteur viral à un stade précoce de l'infection des hépatocytes humains par le VHC *(*Molina et al., 2007, J. Hepatol., 46 (3), 411-419*).* Les conjugués de l'invention peuvent donc être utilisés pour cibler spécifiquement des cellules pathologiques, infectées par des virus tels que ceux des hépatites B et C, qui expriment le LDLR et/ou pour moduler le processus d'infection des cellules saines par les virus via le LDLR.

L'invention concerne également l'utilisation d'un conjugué ou d'une composition tels que définis ci-avant pour le traitement, l'imagerie et/ou le diagnostic de pathologies neurodégénératives telles que de manière non limitative la maladie d'Alzheimer, la maladie de Parkinson, la maladie de Creutzfeldt-Jacob, les accidents vasculaires cérébraux (AVC), l'encéphalite bovine spongiforme, la sclérose en plaques, la sclérose latérale amyotrophique, etc.

L'invention concerne également l'utilisation d'un conjugué ou d'une composition tels que définis ci-avant pour le traitement, l'imagerie et/ou le diagnostic de pathologies neurologiques telles que de manière non limitative l'épilepsie, la migraine, les encéphalites, les douleurs du SNC, etc.

L'invention concerne également l'utilisation d'un conjugué ou d'une composition tels que définis ci-avant pour le traitement, l'imagerie et/ou le diagnostic de pathologies neuropsychiatriques telles que de manière non limitative la dépression, l'autisme, l'anxiété, la schizophrénie, etc.

Les termes « traitement », « traiter » et autres expressions semblables signifient l'obtention d'un effet pharmacologique et/ou physiologique, par exemple, l'inhibition de la croissance des cellules cancéreuses, la mort des cellules cancéreuses ou l'amélioration d'une maladie ou d'un trouble neurologique. L'effet peut être prophylactique ou préventif afin d'empêcher totalement ou partiellement l'aggravation d'une maladie ou d'un symptôme de celle-ci, chez une personne malade, ou sa propagation, chez des sujets sains, et/ou peut être thérapeutique afin de traiter totalement ou partiellement une maladie et/ou ses effets néfastes liés. Le terme « traitement » tel qu'il est utilisé dans le présent document couvre tout traitement d'une maladie chez un mammifère, et plus particulièrement un homme, et comprend : (a) la prévention d'une maladie (par exemple, la prévention du cancer) ou d'une condition pouvant survenir chez une personne prédisposée à cette pathologie ou trouble, mais qui n'a pas encore été diagnostiquée comme atteinte, (b) le ralentissement d'une maladie (par exemple, en arrêtant son développement), ou (c) le soulagement d'une maladie (par exemple, en réduisant les symptômes associés à une maladie). Ce terme « traitement » couvre également toute administration d'une substance active afin de soigner, de guérir, de soulager, d'améliorer, de diminuer ou d'inhiber une condition chez un individu ou patient, y compris, sans s'y limiter, l'administration à une personne en ayant besoin d'un médicament composé d'un vecteur ou d'un conjugué tels que décrits dans le présent document.

La présente invention concerne encore l'utilisation d'un peptide ou pseudo-peptide linéaire ou cyclique, de l'invention pour augmenter l'activité biologique d'une substance active ou d'intérêt (molécule d'intérêt thérapeutique, agent de diagnostic ou d'imagerie médicale, toute autre molécule comme une sonde moléculaire) à laquelle il est couplé.

La présente invention concerne aussi l'utilisation d'un peptide ou pseudo-peptide linéaire ou cyclique, de l'invention, pour diminuer la toxicité de la substance active ou d'intérêt (molécule d'intérêt thérapeutique, agent de diagnostic ou d'imagerie médicale, toute autre molécule comme une sonde moléculaire) à laquelle il est couplé.

D'autres aspects et avantages de la présente invention apparaîtront à la lecture des exemples ci-dessous, qui sont de nature illustrative seulement mais ne limitent pas la portée de la présente demande.

### EXEMPLES

### EXEMPLE I

Clonage du LDLR humain et murin dans un vecteur d'expression.

Les peptides ont été identifiés sur la base de leur interaction et affinité pour les récepteurs humain et murin aux *low-density lipoproteins* (hLDLR et mLDLR), impliqués notamment dans l'endocytose et la transcytose (transport trans-cellulaire, notamment à travers la BHE) du cholestérol. Le préalable à la caractérisation de ces peptides était l'établissement dans des cellules eucaryotes (*chinese Hamster Ovary Cells,* CHO) de lignées stables exprimant de manière constitutive et à taux élevés le hLDLR et le mLDLR. Ces lignées sont utilisées : i) pour la caractérisation de peptides se fixant sur le hLDLR exprimé à la surface des cellules, dans sa configuration native ; ii) pour vérifier que le hLDLR et le mLDLR peuvent internaliser les peptides sélectionnés par endocytose.

La construction de ces lignées est brièvement décrite.

Les séquences des ARN messagers codant pour le hLDLR et le mLDLR sont disponibles dans les banques de données (numéros d'accès: NM_000527 et NM_010700 respectivement). Les amorces nécessaires à l'amplification des ADNc par PCR ont été sélectionnées, comportant à leur extrémité (en caractères gras) les sites de restriction nécessaires (HindIII et SalI pour le LDLR humain et HindIII et KpnI pour le LDLR murin) au clonage dans le vecteur d'expression pEGFP-N1 (Clontech) :
hLDLR
Amorce *Forward :* ATATAT**AAGCTT**CGAGGACACAGCAGGTCGTGAT
Amorce *Reverse :* TTAATT**GTCGACC**ACGCCACGTCATCCTCCAGACT
mLDLR
Amorce *Forward* : ATATAT**AAGCTT**GACGCAACGCAGAAGCTAAG
Amorce *Reverse :* TTAATT**GGTACC**GTTGCCACATCGTCCTCCAG

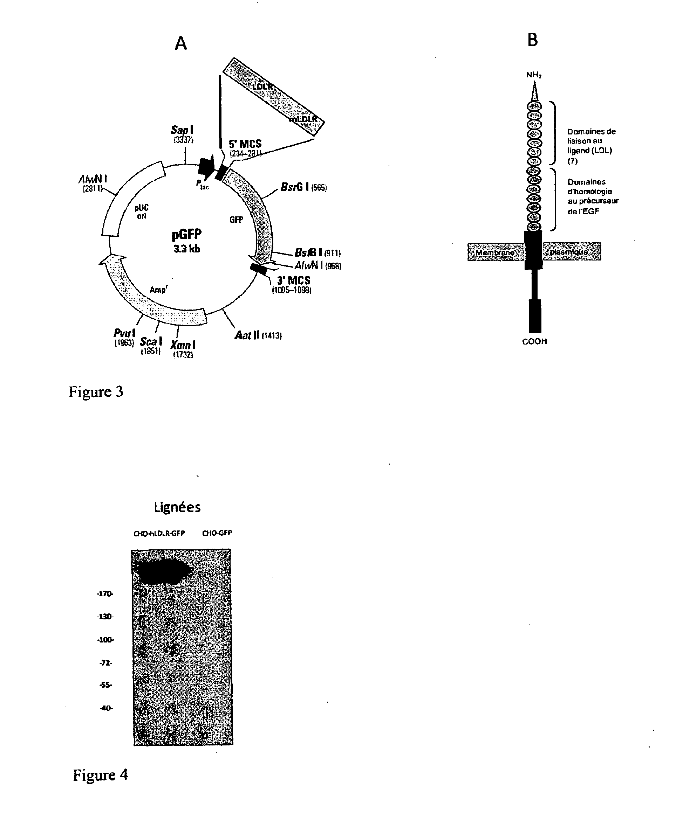

Les ARN totaux préparés à partir de cerveaux humains et murins ont été transformés en ADNc par reverse transcription pour l'amplification par PCR de fragments d'ADN codant pour le hLDLR et le mLDLR. Après amplification, les produits PCR ont été digérés par les enzymes de restriction HindIII-SalI et HindIII-KpnI respectivement et ligués dans le vecteur d'expression pEGFP-N1 (Clontech), digéré par les mêmes enzymes de restriction. Après transfection dans des cellules eucaryotes, ce vecteur permet l'expression, sous contrôle du promoteur CMV, de LDLR fusionnés à la GFP à leur extrémité C-term, c'est à dire à l'extrémité de leurs domaines intracellulaires (Figure 3). Après transformation de bactéries compétentes *E. Coli* DH5α, obtention de colonies isolées, préparation de l'ADN des plasmides, les constructions ont été intégralement séquencées sur les 2 brins pour vérification.

### EXEMPLE II

Etablissement de lignées cellulaires CHO exprimant de manière stable les LDLR humain et murin.

Des transfections transitoires dans différentes lignées cellulaires (CHO, COS, N2A et HEK293) ont été réalisées pour déterminer sur cellules vivantes ou fixées les niveaux d'expression et la localisation membranaire du hLDLR et du mLDLR. Le récepteur est visible directement sur cellules vivantes, sous microscopie à fluorescence, sans nécessité d'immunomarquage, grâce à la fluorescence verte émise par la GFP fusionnée en C-term de ces récepteurs. Des transfectants stables ont été sélectionnés par dilution limite et grâce au gène de résistance à la généticine (G418) porté par le vecteur d'expression. Ces lignées ont été amplifiées tout en gardant une pression de sélection. Dans l'exemple évoqué ici, l'expression du hLDLR-GFP de la taille attendue a été vérifiée par Western Blot sur lysats cellulaires avec des anticorps dirigés contre le LDLR humain et contre la GFP. Une protéine correspondant aux tailles combinées de la GFP et du hLDLR (190 kDa) est reconnue par les anticorps anti-hLDLR (Figure 4) et anti-GFP dans des extraits cellulaires préparés à partir des lignées stables. Une lignée CHO exprimant la GFP de manière constitutive a été utilisée comme contrôle négatif. L'anticorps anti-hLDLR ne détecte aucune protéine dans la lignée GFP.

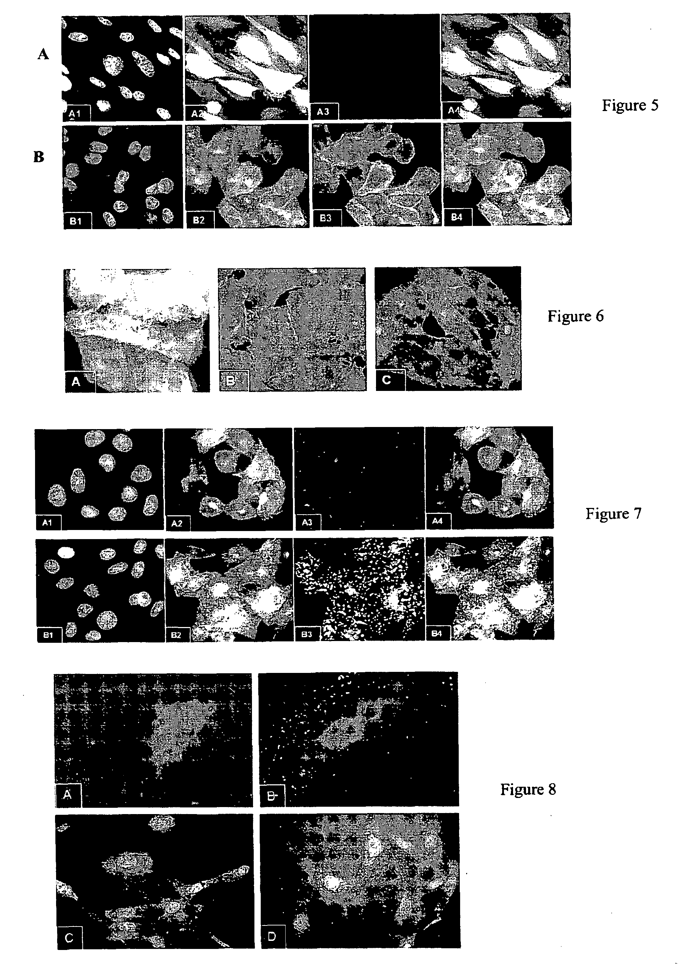

L'immunocytochimie avec l'anticorps anti-hLDLR sur cellules fixées (PFA) des lignées CHO-GFP (utilisée comme contrôle) et CHO-hLDLR-GFP montre que la fusion hLDLR-GFP est bien exprimée dans les cellules transfectées. Des expériences d'immunocytochimie sur cellules non perméabilisées avec du Triton X100 montrent que le domaine extracellulaire du LDLR est bien détecté au niveau extracellulaire (Figure 5).

Une co-localisation entre le hLDLR et son ligand naturel le LDL, rendu fluorescent par adsorption du DiI, un marqueur fluorescent, est montrée. Ce ligand naturel fluorescent (DiI-LDL) est rapidement internalisé (endocytose) comme visualisé sous microscopie à fluorescence sur cellules fixées (Figure 6-A). Par contre, le DiI-LDL n'est pas endocyté par la lignée contrôle CHO-GFP, ou par une autre lignée contrôle de cellules CHO, qui surexprime par exemple le récepteur humain de la transferrine (hTfR, Figure 6-B), un autre récepteur impliqué dans la transcytose. De plus, l'activité d'endocytose de la lignée CHO-LDLR-GFP est spécifique au ligand du LDLR puisque dans cette lignée, on n'observe pas d'endocytose de ligand qui ne lui soit pas spécifique, à titre d'exemple la transferrine (Tf) marquée avec un fluorochrome rouge (le *Texas Red,* Figure 6-C).

La fonctionnalité des récepteurs (capacité d'endocytose) est confirmée par des expériences en temps réel, sous vidéo-microscopie à fluorescence montrant que le LDL, ligand naturel du hLDLR, marqué au DiI, est effectivement transporté rapidement et très efficacement dans les cellules exprimant le hLDLR-GFP comparé aux cellules exprimant la GFP seule ou aux cellules exprimant un autre récepteur impliqué dans l'endocytose tel le hTfR (contrôles négatifs). A l'inverse, des expériences de vidéo-microscopie réalisées avec la Tf marquée au *Texas Red,* qui est très efficacement endocytée par des cellules exprimant le hTfR-GFP, confirment que la transferrine n'est pas endocytée par les cellules d'une lignée contrôle GFP ni par celles d'une lignée hLDLR.

Malgré un haut taux d'expression du hLDLR dans les lignées CHO-hLDLR-GFP, le système d'endocytose est non seulement efficace, mais il a conservé sa sélectivité. La présence de la fusion GFP n'a altéré ni les propriétés d'insertion dans la membrane du hLDLR, ni l'exposition à l'extérieur de la cellule du domaine extracellulaire du hLDLR, ni la fonctionnalité du récepteur dans les processus d'endocytose.

### EXEMPLE III

Crible de banques de plusieurs milliards de séquences peptidiques aléatoires présentées par des virus bactériens sur les lignées CHO-hLDLR-GFP et identification de virus bactériens et donc de séquences peptidiques présentant une affinité pour le hLDLR. Les approches de crible avec des banques de séquences peptidiques aléatoires présentées par des virus bactériens ont été utilisées sur la lignée CHO-hLDLR-GFP, après épuisement des banques de virus bactériens sur une lignée contrôle CHO-GFP exprimant la GFP seule. Après lavage abondant puis élution à l'acide, les virus bactériens fixés sur les lignées sont amplifiés par infection de bactéries *E. Coli* ER 2738 en milieu liquide ou sur boîtes de Pétri. Les cribles réitératifs sur lignées cellulaires des virus bactériens élués puis réamplifiés ont permis la caractérisation de virus bactériens présentant une forte affinité pour le hLDLR exprimé à la surface des cellules. Parmi ces virus bactériens, n'ont été retenus que ceux qui se lient également à la lignée murine (CHO-mLDLR-GFP).

Cinq cribles indépendants ont été réalisés et au total, environ 200 clones de virus bactériens (plages retard) ont été séquencés après amplification par PCR des régions du génome du virus bactérien codant pour les peptides présentés par ces virus bactériens. De nombreux virus bactériens présentaient des séquences identiques. Deux familles de séquences de peptides ont été obtenues : des peptides cycliques avec motif conservé, SEQ ID NO : 1 à SEQ ID NO : 11 et SEQ ID NO : 30 à SEQ ID NO : 65, et des peptides linéaires sans motif conservé SEQ ID NO : 12 à SEQ ID NO : 29. (cf. liste des séquences du présent document).

Les clones de virus bactériens identifiés suite à la liaison des peptides qu'ils présentent et qui se lient au hLDLR-GFP exprimé par la lignée stable CHO-hLDLR-GFP ont été mis en présence des cellules des lignées LDLR et après lavage intensif, ont été détectés avec un anticorps dirigé contre la protéine de l'enveloppe virale du virus bactérien suivi d'un second anticorps couplé à l'Alexa 594. (Figure 7). Ces virus bactériens ne se fixent pas sur les cellules de la lignée contrôle CHO-GFP exprimant la GFP seule.

Des lots de virus bactériens purifiés avec des titres identiques ont été mis en compétition sur les lignées et après lavage intensif et élution à l'acide, clonage et séquençage d'une cinquantaine de virus bactériens, les plus affins parmi les peptides cycliques et linéaires ont été identifiés.

Ceux qui n'ont pas été sélectionnés ou retenus pour d'autres expérimentations ne sont en aucun cas dénués d'intérêt, ils ne peuvent pas être considérés comme non fonctionnels. Ces peptides conservent un potentiel important en tant que candidats-vecteurs, ils sont par conséquent également revendiqués par la présente invention.

### EXEMPLE IV

Interaction des peptides présentés par les virus bactériens avec le hLDLR des lignées CHO-LDLR-GFP et avec le hLDLR de cellules non modifiées génétiquement.

Les virus bactériens présentant des peptides affins pour le hLDLR ont été incubés avec des cellules adhérentes, non modifiées génétiquement, et connues pour exprimer de manière constitutive ou inductible le hLDLR, notamment des fibroblastes humains, des cellules endothéliales humaines préparées à partir de cordon ombilical (HUVEC) ou des cellules endothéliales microvasculaires de cerveau de porc. Les approches d'immunocytochimie avec les anticorps dirigés contre la protéine de l'enveloppe virale du virus bactérien montrent que les peptides affins pour le hLDLR, présentés par les virus bactériens, se lient également au LDLR présents sur des cellules primaires, non génétiquement modifiées, par exemple des fibroblastes humains et des cellules endothéliales microvasculaires de cerveau de porc (Figure 8).

Les approches d'immunocytochimie de nature qualitative ont été complétées avec des approches quantitatives de cytométrie en flux (FACS) avec des cellules en suspension (lignée GFP, lignée CHO-hLDLR-GFP, fibroblastes humains, cellules HUVEC). Ces cellules ont été cultivées dans du milieu de culture, elles ont été décollées et dissociées mécaniquement (sans trypsine), centrifugées, resuspendues et incubées en présence de différents clones de virus bactériens et de l'anticorps anti-LDLR, pendant 20 min à 4°C afin d'éviter tout processus d'endocytose. Après lavages, les virus bactériens liés aux cellules, ont été révélés avec un anticorps anti-protéine de l'enveloppe virale. Les analyses par FACS ont été réalisées avec un FACSCanto (Beckton Dickinson) utilisant le logiciel BD FACSDiva. Le nombre de cellules positives a été standardisé à 5000 évènements pour chaque essai. Les résultats ont été exprimés en unités arbitraires de fluorescence.

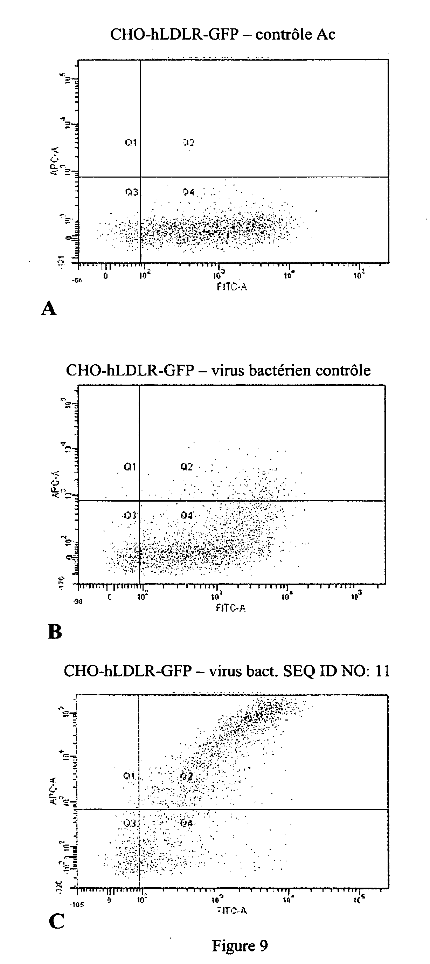

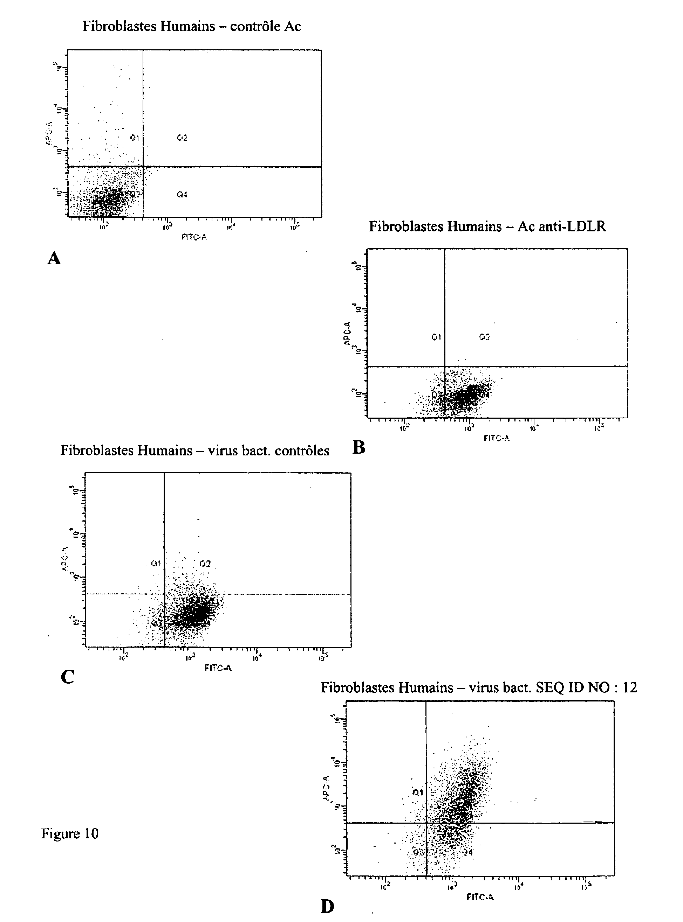

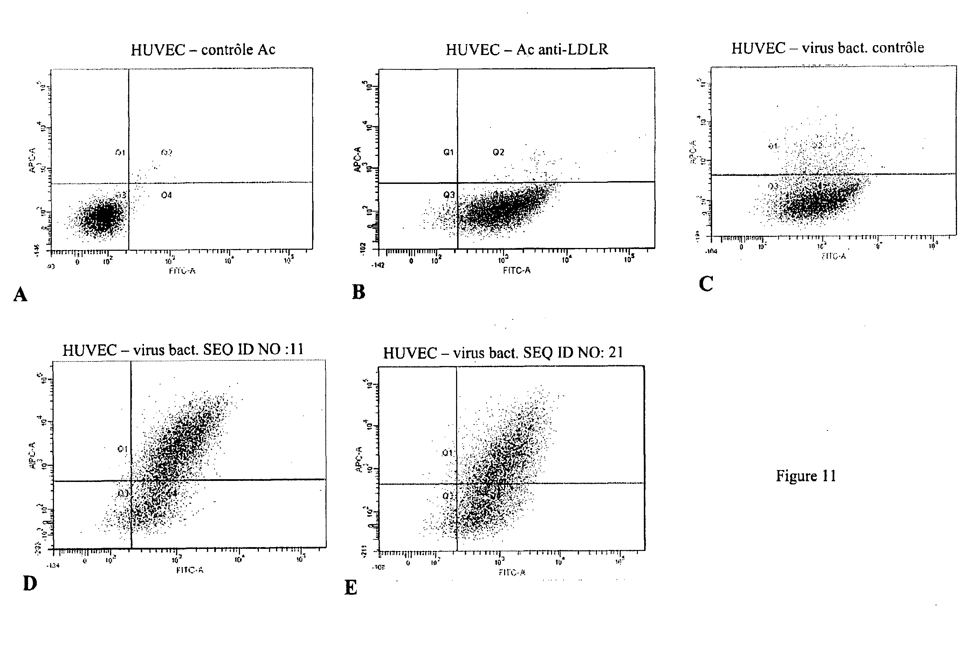

Ces approches ont démontré une corrélation entre le taux d'expression du hLDLR déterminé par l'anticorps anti-hLDLR révélé par anticorps secondaire Alexa 488 (axe horizontal) sur différents types cellulaires (Figures 9-11), et un anticorps dirigé contre la protéine d'enveloppe du virus bactérien, révélé par un anticorps Alexa 647 ou un anticorps Allophycocyanine ou APC (axe vertical). Les Figures 9, 10, 11 correspondent aux résultats obtenus pour les peptides SEQ ID NO : 11, SEQ ID NO : 12 et SEQ ID NO : 21 sur des cellules de la lignée CHO-hLDLR-GFP (Figure 9), des fibroblastes humains (Figure 10) et des cellules HUVEC (Figure 11).

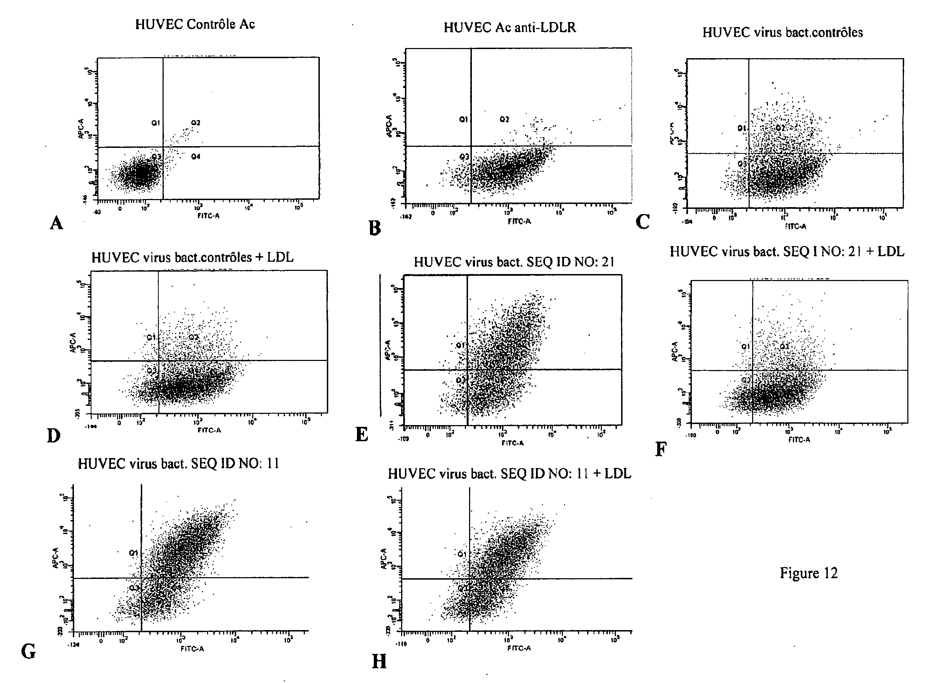

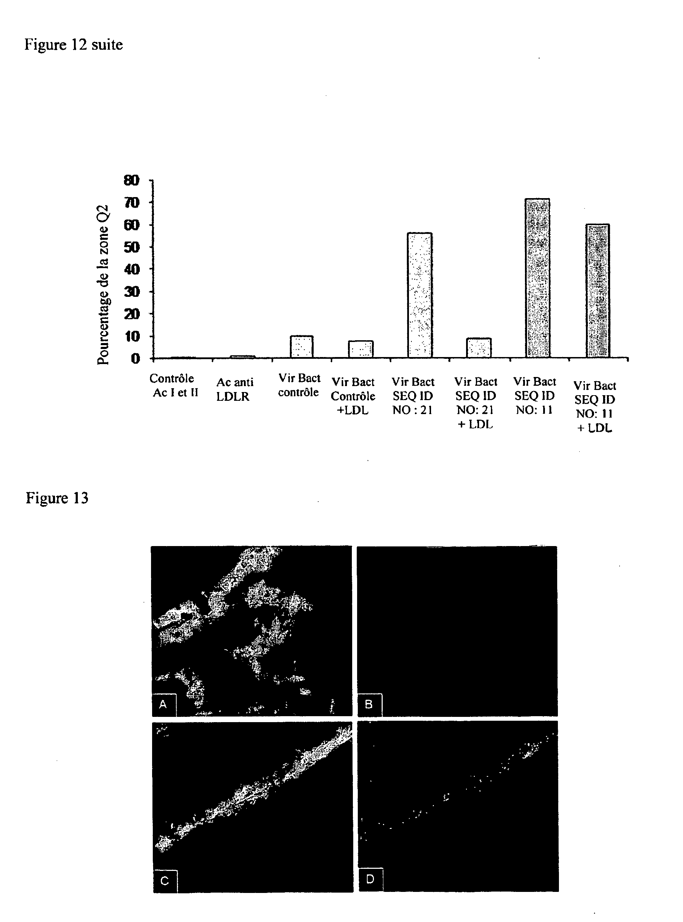

L'hypothèse, selon laquelle les peptides présentés par les virus bactériens affins pour le hLDLR pourraient interagir avec les domaines de liaison du LDL, le ligand naturel du hLDLR, a été évaluée sur différents types cellulaires, notamment sur cellules endothéliales HUVEC par des expériences de FACS, similaires à celles présentées ci-dessus, comme montré ici pour exemple (Figure 12). Dans un premier temps il est montré que le taux de liaison des virus bactériens présentant des peptides affins pour le hLDLR (SEQ ID NO : 21 et SEQ ID NO : 11 sont utilisés ici comme exemples) est proportionnel au taux d'expression du LDLR par les cellules HUVEC. Des expériences de FACS réalisées en présence de LDL, ont permis de démontrer et de quantifier un déplacement important (85%) du LDL par les peptides linéaires (par exemple SEQ ID NO : 21), mais minime (17%) par les peptides cycliques (par exemple SEQ ID NO : 11).

### EXEMPLE V

Interaction des peptides présentés par les virus bactériens affins pour le hLDLR avec les cellules endothéliales de vaisseaux cérébraux de souris *in vivo.*

Les virus bactériens présentant des peptides affins pour le hLDLR (SEQ ID NO : 1 est décrit ici pour exemple) et des virus bactériens contrôles (ne présentant pas de peptide affin pour le hLDLR) ont été injectés dans la veine de queue de souris C57BL6 anesthésiée à l'halothane. Après 15 min et 2h post injection, les souris ont été sacrifiées et perfusées avec du NaCl 0,9%. Le cerveau, le foie et les reins ont été congelés dans l'isopentane et des coupes à congélation ont été réalisées pour un double marquage par immunohistochimie permettant de visualiser d'une part les vaisseaux sanguins avec une IgG anti-souris et un anticorps anti-protéine de l'enveloppe virale permettant de visualiser les virus bactériens liés aux cellules endothéliales *in vivo.* Les expériences montrent que les virus bactériens présentant le peptide SEQ ID NO : 1 affin pour le hLDLR et pour le mLDLR se lient aux cellules endothéliales de la paroi des vaisseaux, ce qui n'est pas le cas des virus bactériens contrôles (Figure 13).

### EXEMPLE VI

Synthèse des peptides correspondant aux virus bactériens affins pour le hLDLR, couplage à une molécule traceur (biotine, fluorescéine, ou S-Tag avec une activité enzymatique).

Les peptides ont été synthétisés par la méthode de synthèse en phase solide (*solid phase peptide synthesis* ou SPPS) sur un synthétiseur automatique, modèle Advanced ChemTech Apex396 (AAPPTec), ou un synthétiseur automatique assisté par les micro-ondes, modèle Liberty™ (CEM), en utilisant une stratégie Fmoc/tBu sur résines Rink Amide AM sur polystyrène-1%DVB, Wang sur polystyrène-1%DVB, Barlos (2-*chlorotrityl chloride*) sur polystyrène-1%DVB, ou Sieber Amide sur polystyrène-1%DVB. La charge (ou substitution) est comprise entre 0,25 et 1,6 mmol/g suivant la résine utilisée.

Les acides aminés N-protégés par un Fmoc (ou un Boc pour certaines extrémités N-term) et/ou protégés par des fonctions orthogonales (acido-labiles notamment) au niveau de leurs chaînes latérales, les réactifs chimiques de couplage et de déprotection, et les solvants sont achetés auprès de sociétés spécialisées et utilisés tels quels.

Les résines Rink Amide et Wang permettent de synthétiser les séquences peptidiques totalement déprotégées sur leurs chaînes latérales et leurs extrémités C-term. On parle alors de synthèse peptidique SPPS orthogonale à 2 dimensions (Fmoc/tBu).

Les résines « *hypersensitive acid labile ou HAL »* de type Barlos et Sieber permettent la libération de la fonction acide ou amide terminale (en C-term) tout en conservant les protections latérales orthogonales des différents acides aminés du peptide synthétisé ainsi que la protection amine terminale (N-term) de la fonction amine de son dernier acide aminé (par exemple, N-acétylation pour des questions de stabilité de la séquence peptidique néo-synthétisée). Ce type de résines via une stratégie de synthèse Fmoc (en Prot₁) permet d'utiliser des protections latérales orthogonales acido-labiles (Prot₂ de type Boc, tBu, OtBu, Trt, Mmt, Acm, etc.) clivables uniquement en milieu fortement acide, alors que le décrochage du peptide protégé se fait en conditions acides très douces. Ce type de clivage permet de récupérer la séquence peptidique totalement protégée sur ses fonctions latérales (Prot₂) en vue du couplage de la molécule d'intérêt thérapeutique sur le peptide. On parle alors de synthèse peptidique SPPS orthogonale à 3 dimensions (Barlos ou Sieber/Fmoc/tBu).

Les protections latérales orthogonales (Prot₂) standard utilisées pour chaque acide aminé au cours de la synthèse peptidique sont : Arg(N-Pbf), Arg(N-Pmc), Asn(N-Trt), Asp(O-tBu), Cys(S-Acm), Cys(S-Mmt), Cys(S-4MeBn), Cys(S-tBu), Cys(S-Tmob), Cys(S-Trt), Glu(O-tBu), Gln(N-Trt), His(N-Trt), Lys(N-Boc), Ser(O-tBu), Thr(O-tBu), Trp(N-Boc), Tyr(O-tBu) *(Applied Biosystems, 1998, Cleavage, Deprotection, and Isolation of Peptides after Fmoc Synthesis. Technical Bulletin).* Gly, Ala, Val, Leu, Ile, Phe, Met et Pro ne possèdent pas de protections latérales, puisque leurs structures chimiques respectives ne le nécessitent pas.

Les couplages d'acides aminés se font via activation de la fonction acide de l'acide aminé n+1 à l'aide de DIEA/HBTU/HOBt ou DIPC/HOBt dans du DMF.

La déprotection du groupement Fmoc (Prot₁) d'un nouvel acide aminé ainsi couplé est réalisée à l'aide de 20% de pipéridine dans le DMF.

Le dernier acide aminé couplé au cours de la séquence peptidique sera soit protégé par une fonction Boc (en vue de libérer sa fonction amine terminale libre en fin de synthèse), soit acétylé (afin de stabiliser le néo-peptide synthétisé mais également de réduire les risques de réactions secondaires lors du couplage covalent de la molécule d'intérêt thérapeutique en C-term par exemple).

Suivant le peptide synthétisé, les ponts disulfures sont obtenus par cyclisation intramoléculaire à partir de 2 fonctions thiols de 2 Cys convenablement protégées (Acm, Trt, tBu, etc.), soit en solution, soit sur la résine, à l'aide de réactifs classiquement utilisés par l'homme de l'art : I₂/DMF, I₂/HFIP/DCM, TFA/DMSO/anisole, I₂/DCM/MeOH/H₂O, etc. Une Cys en positon N-term peut être avantageusement remplacée par un acide Mpa pour la cyclisation via un pont disulfure. Des ponts lanthionines (par cyclisation via déshydroalanine) ou dicarba (par cyclisation via allylGly) peuvent également être obtenus par des voies de synthèse connues de l'homme de l'art. Un pont lactame peut-être créé entre la fonction acide latérale d'un résidu Glu (ou Asp) et une fonction amine latérale sur une Lys ou une amine N-term. De même une cyclistion entre la fonction amine N-term et la fonction acide C-term (tête/queue) peut être réalisée via une liaison amide, tout comme une cyclisation entre la fonction amine latérale d'une Lys et la fonction acide C-term du peptide.

Le clivage des peptides des résines Barlos ou Sieber se fait par des méthodes classiquement utilisées par l'homme de l'art, soit avec 0,5% de TFA (v/v) dans du DCM, soit avec de l'AcOH/TFE/DCM (1/1/3), soit avec du HFIP (30%) dans du DCM, soit avec du TFE (30%) dans du DCM, etc.

Les déprotections des chaînes latérales, et le clivage des peptides des résines Rink Amide ou Wang, sont réalisés par des méthodes classiquement utilisées par l'homme de l'art : soit avec un mélange TFA/H₂O/TIS ou TIPS (95/2,5/2,5), soit avec TFA/H₂O/EDT/TIS ou TIPS (94/2,5/2,5/1), soit avec TFA/thioanisole/H₂O (94/5/1), soit avec TFA/TIS/H₂O/thioanisole (90/5/3/2), soit avec TFA/H₂O/phénol/thioanisole/EDT (82,5/5/5/5/2,5), etc.

Les biotines, fluorescéines, ou S-Tag (cf. EXEMPLE VII, ci-dessous) sont introduits en C-term selon des procédés de synthèse et de couplage classiques connus de l'homme de l'art.

Les peptides sont isolés et purifiés par HPLC sur un Appareil Beckman System Gold 126 avec une colonne Chromolith C18 (4,6 x 50 mm) ou Nucleosil C18 (10 x 250 mm) avec par exemple un gradient de 0 à 100% d'acétonitrile dans une phase aqueuse (H₂O + 0,1% TFA) en 3,5 min puis 100 à 0% en 1,5 min (débit : 1 à 5 ml/min), ou sur une chaîne Waters 1525 avec une colonne (phase stationnaire) Chromolith Speed ROD RP-18 (4,6 x 50 mm) avec détection par un équipement Waters 996 PDA (190 - 400 nm), ou sur une chaîne Waters Alliance 2690 avec une colonne (phase stationnaire) Chromolith Performance RP-18 (3 x 100 mm) avec détection par un équipement Waters 996 PDA (190 - 400 nm). La détection UV est réalisée à 214 et 254 nm.

Les purifications préparatives sont réalisées avec une chaîne Waters prep LC4000 system avec une colonne (phase stationnaire) Guard-PakTM cartridges Delta-PakTM C18 (25 x 10 mm) avec détection par un équipement Waters 2487 Dual 1 Absorbance Detector.

Les masses moléculaires sont déterminées en utilisant un spectromètre de masse à électrospray d'ions (ESI) en mode positif. Les spectres sont obtenus en utilisant un appareil Waters MicromassQuattro Micro (analyseur quadripôle) équipé d'un système HPLC Waters Alliance 2690 permettant le couplage LC-MS.

Les conditions d'analyses LC-MS utilisées sont les suivantes :
- colonne C18 Chromolith Flash (4,6 x 25 mm),
- debit de 3 mL/min,
- gradient linéaire de 0 à 100 % de B en 2,5 min (A : H2O/HCO2H 0,1 % ; B : ACN/HCO2H 0,1 %).

L'acquisition des spectres de masse en mode electrospray positif se fait à un débit de 100-200 µL/min. Les données sont obtenues en mode scan de 200 à 1700 *m*/*z* à intervalles de 0,1 s.

### EXEMPLE VII

### Liaison et endocytose des peptides synthétisés, affins pour le hLDLR dans les lignées CHO-LDLR-GFP.

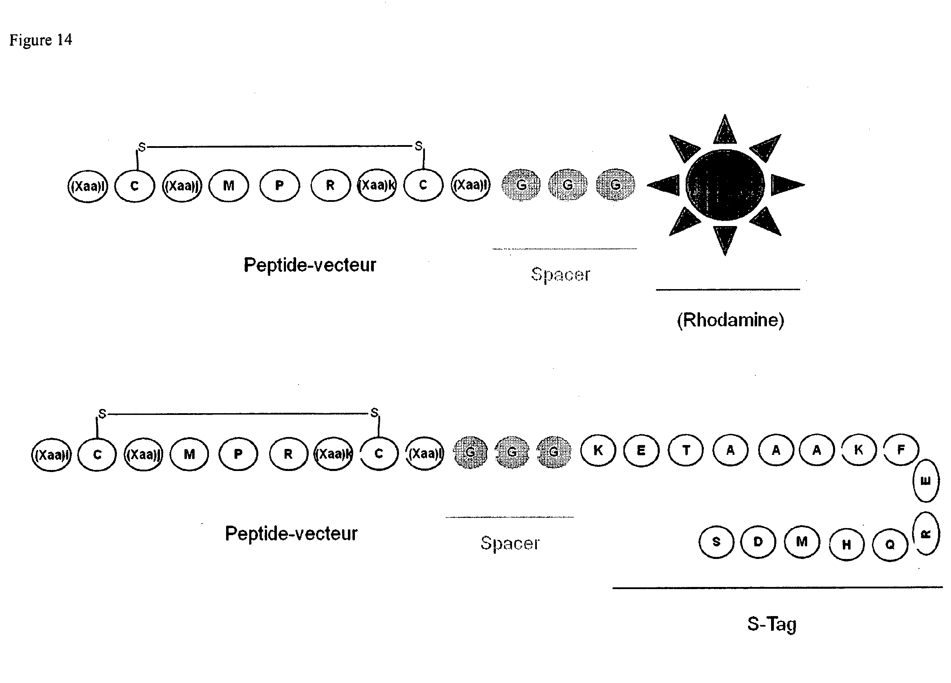

Des peptides affins pour hLDLR-GFP, ainsi que des peptides contrôles (*random peptides,* résidus identiques aux peptides affins mais dans le désordre) ont été synthétisés, et couplés/conjugués en C-term à différentes molécules « traceurs », soit la rhodamine, soit le S-Tag, séparés par un bras espaceur constitué de 3 résidus Gly (Figure 14). Le S-Tag (peptide de 15 acides aminés dérivé de la séquence 1-15 de la ribonucléase A pancréatique bovine) peut d'une part être reconnu par un anticorps anti-S-Tag pour des approches d'immunocytochimie ou de FACS, et d'autre part reconstituer une activité enzymatique par liaison à la ribonuclease S-protein (portion C-term, acides aminés 21-124) dans des tests d'activité *in vitro* utilisant le FRETWorks S-Tag *assay kit* (Novagen 70724-3). La ribonucléase ainsi activée digère un substrat d'ARN libérant un agent fluorescent masqué révélé par FRET *(Fluorescence Resonance Energy Transfert*) et quantifié en plaque de 96 puits dans un spectrofluorimètre Beckmann. Pour ces expériences de FRET, des cellules CHO contrôles et la GFP fusionnée en C-term au hLDLR et au mLDLR, qui génère un bruit de fond important aux longueurs d'ondes utilisées pour le FRET a été remplacée par la *Red Fluorescent Protein* (RFP). Les lignées stables générées pour les expériences de FRET sont donc CHO-RFP et CHO-hLDLR-RFP.

Pour les approches de FRET, les cellules sont lavées 2 fois avec 2 ml de PBS puis incubées 1h à 37°C avec 250 µl de solution de peptide. Elles sont à nouveau lavées 2 fois avec 2 ml de PBS puis 2 fois avec 1 ml de PBS, puis grattées dans 1 ml de PBS et centrifugées 5 min à 1250 rpm. Le surnageant est alors aspiré et le culot cellulaire est lysé dans 80 µl de PBS + 0.1% de Triton X100. Vingt µl de chaque lysat cellulaire sont analysés par mesure de l'émission de fluorescence après réaction de FRET.

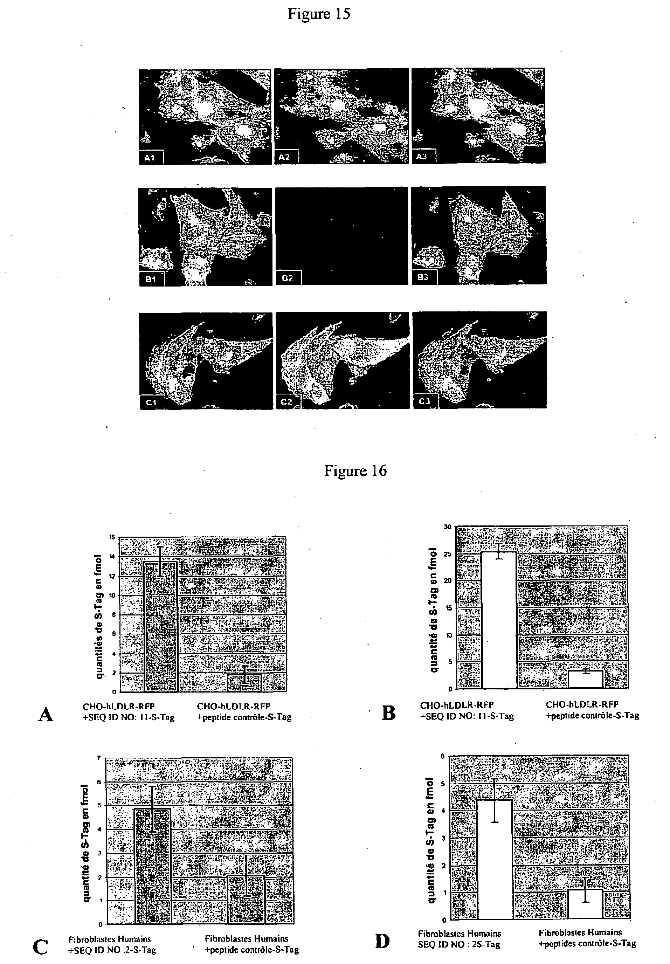

Des expériences d'incubation des peptides sur différentes cellules exprimant le hLDLR ont été réalisées. Les résultats obtenus avec le peptide SEQ ID NO : 1 conjugué à la rhodamine ou le peptide SEQ ID NO : 2 conjugué au S-Tag sont montrés pour exemple et indiquent que les peptides se lient bien aux cellules CHO-LDLR-GFP et qu'ils sont endocytés pour s'accumuler dans les cellules de la lignée qui expriment le hLDLR, ce qui n'est pas le cas des peptides contrôles (Figure 15). Dans ces expériences, des incubations préalables des peptides conjugués au S-Tag, avec un anticorps primaire (Ac primaire) dirigé contre le S-Tag, et d'un anticorps secondaire (Ac secondaire) dirigé contre l'anticorps primaire montrent que le complexe SEQ ID NO: 2/S-Tag/Ac primaire/Ac secondaire se lie aux cellules exprimant le hLDLR et est internalisé par endocytose. Ces résultats indiquent que les peptides de la famille SEQ ID NO : 2 peuvent se lier à des cellules exprimant le hLDL et vectoriser des charges importantes (2 anticorps), i.e. internalisation par endocytose de ces charges.

L'endocytose des peptides affins pour le hLDLR a été quantifiée. Dans ce but, les peptides SEQ ID NO : 11/S-Tag et contrôle *(randonna peptide*) ont été incubés avec les cellules CHO-hLDLR-RFP pendant 1h à 37°C. Les cellules ont été lavées afin d'éliminer toute trace de peptide non fixé. Les lavages ont été effectués d'une part avec du PBS ce qui permet de quantifier par FRET les peptides fixés à la membrane cellulaire et ceux internalisés par endocytose (Figure 16-A), d'autre part avec une solution acide (glycine 0.2M, NaCl 0.15M, pH3) qui permet de dissocier les peptides fixés au hLDLR à la membrane cellulaire. Seuls les peptides endocytés par les cellules sont alors détectés par FRET (Figure 16-B). De même avec les peptides SEQ ID NO : 2/S-Tag sur fibroblastes humains (Figure 16-C-D).

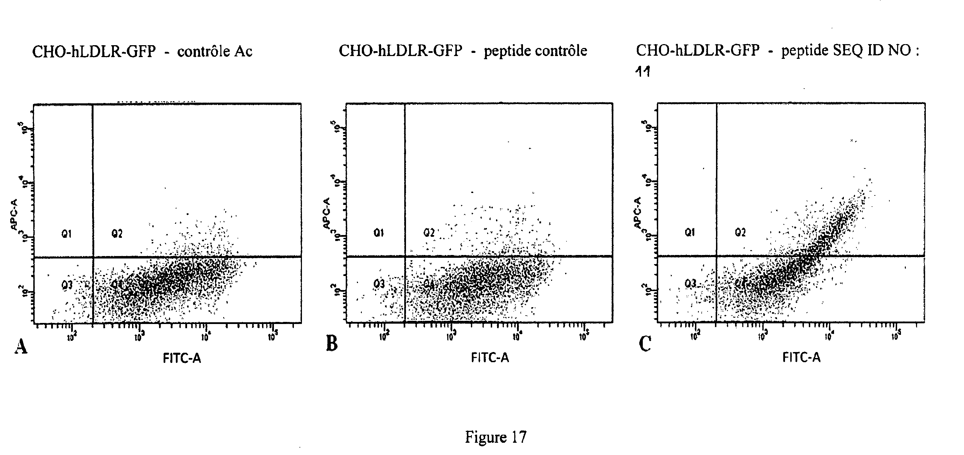

Ces observations ont également été confirmées sur cellules non adhérentes par FACS. Un exemple est donné de la liaison sur les cellules de la lignée CHO-hLDLR-GFP du peptide SEQ ID NO : 11 à l'inverse du peptide contrôle, tous deux étant conjugués au S-Tag, (Figure 17).

### EXEMPLE VIII

Toxicité, endocytose et transcytose des peptides synthétisés, affins pour le hLDLR, sur des cellules endothéliales dans un modèle de BHE *in vitro.*

Les potentiels effets toxiques des peptides sur cellules endothéliales, la liaison/accumulation des peptides dans ces cellules, et le passage par transcytose des peptides ont été évalués sur modèles de BHE *in vitro.* Les cellules nécessaires à la mise en place du modèle (co-culture de cellules endothéliales issues de micro-vaisseaux cérébraux et astrocytes) sont des cellules bovines *(bovine brain microvascular endothelial cells,* BBMEC) distribuées par la société Cellial Technologies (Lens, France). Ce modèle de BHE *in vitro* est utilisé pour évaluer le passage passif ou le transport actif de nombreuses molécules, notamment des agents pharmacologiques, à travers la BHE et donc leur capacité à atteindre le tissu nerveux du SNC. Le modèle présente des propriétés ultra-structurales caractéristiques de l'endothélium cérébral notamment les jonctions serrées, l'absence de pores, l'absence de canaux trans-endothéliaux, la faible perméabilité aux molécules hydrophiles et une haute résistance électrique. En outre, ce modèle a montré une bonne corrélation entre les résultats des mesures effectuées sur différentes molécules évaluées *in vitro* et *in vivo* pour leur propriété de passage au travers de la BHE. A ce jour, toutes les données obtenues révèlent que ce modèle de BHE imite de près la situation *in vivo* en reproduisant certaines des complexités de l'environnement cellulaire qui existent *in vivo,* tout en conservant les avantages associés à l'expérimentation de culture de tissus. De nombreuses études ont validé cette co-culture cellulaire comme l'un des plus reproductible modèle de BHE *in vitro.*

Le modèle de BHE *in vitro* met en jeu une co-culture de BBMECs et d'astrocytes. Préalablement à la culture cellulaire, des inserts de plaque (Millicell-PC 3,0 µM ; 30 mm de diamètre) sont traités sur la partie supérieure avec du collagène de queue de rat afin de permettre une adhésion optimale des BBMECs et créer les conditions d'une lame basale. Des cultures primaires d'astrocytes mixtes sont établies à partir de cortex cérébral de rat nouveau-né *(*Dehouck et al., 1990, J. Neurochem., 54, 1798-1801)*.* Brièvement, après avoir ôté les méninges, le tissu cérébral est passé sur un tamis de nylon à 82 µm. Les astrocytes sont distribués dans les puits de microplaques à une concentration de 1,2x10⁵ cellules/ml et 2 ml de milieu de culture optimal (DMEM) complémenté avec 10% de sérum de veau foetal inactivé par chauffage. Le milieu est changé deux fois par semaine. Les BBMECs obtenus auprès de Cellial Technologies sont cultivées en présence de milieu DMEM complémenté avec 10% (v/v) de sérum de cheval et 10% de sérum de veau inactivé par chauffage, 2 mM de glutamine, 50 µg/ml de gentamycine, et de 1 ng/ml de facteur de croissance basique de fibroblastes, ajouté tous les deux jours. Les BBMECs sont ensuite distribuées sur la face supérieure des filtres dans 2 ml de la co-culture. Ce milieu de BBMECs est changé trois fois par semaine. Dans ces conditions, des BBMECs différenciées forment une monocouche de cellules confluentes 7 jours plus tard. Les expériences rapportées ci-dessous sont réalisées entre 5 et 7 jours après que la confluence ait été atteinte.

Le peptide SEQ ID NO : 1 et un peptide contrôle, tous deux conjugués à la rhodamine, ont été incubés dans la chambre supérieure du système de culture, en contact avec les cellules endothéliales pendant 1h, 5h et 24h. Le milieu de culture de la chambre inférieure est collecté à différents temps et la fluorescence quantifiée par analyse fluorimétrique. Les résultats sont exprimés en perméabilité de surface endothéliale (ou Pe) exprimée en 10⁻³ cm/min. Le *Lucifer Yellow* (LY), une petite molécule fluorescente qui passe peu la BHE, est utilisé d'une part pour contrôler l'intégrité de la BHE *in vitro,* dans tous les puits analysés, mais d'autre part, en co-incubation avec les peptides, afin de contrôler l'absence de toxicité des peptides pour les cellules endothéliales qui forment cette BHE. La barrière *in vitro* est considérée « perméable » ou « ouverte » si la valeur de passage Pe du LY est supérieure à 1.10⁻³ cm/min. La mesure de la *Trans Endothelial Electrical Resistence* (TEER), évaluée avec un Ohm-mètre et exprimée en Ohm.cm², permet également de mesurer l'intégrité de la BHE *in vitro* lors des tests de passage à travers cette BHE. La valeur du seuil de qualité est fixée à >500 Ohm.cm².

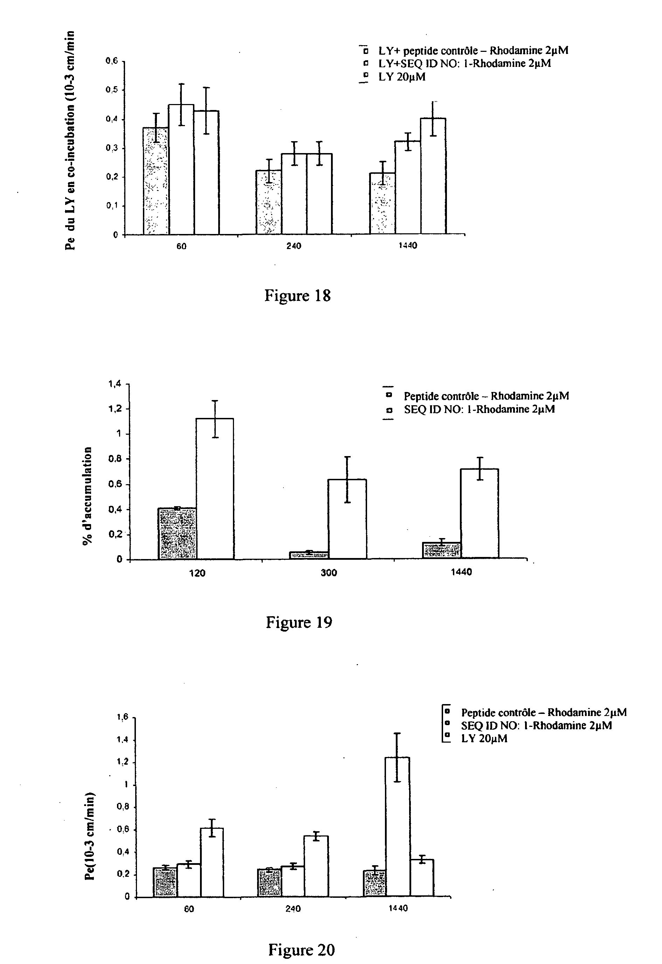

Les analyses réalisées avec les peptides montrent une absence de toxicité du peptide SEQ ID NO : 1, de même pour le peptide contrôle utilisé, et une absence d'effets délétères sur les propriétés de perméabilité de la BHE, puisque les valeurs de Pe mesurées avec le LY n'augmentent pas en présence ou absence des peptides, même après 24h d'incubation (Figure 18).

Le taux de liaison et/ou d'internalisation d'un peptide contrôle et du peptide SEQ ID NO : 1, tous deux conjugués à la rhodamine, a été déterminé sur le modèle de BHE *in vitro* décrit ci-dessus. Cette analyse a été réalisée par lyse des cellules endothéliales à différents temps (2h, 5h, 24h), et mesure par fluorimétrie de la quantité de fluorescence (rhodamine), associée aux cellules (compartiments membranaires et cytoplasmiques obtenus par centrifugation ces cellules après lyse). Les valeurs mesurées indiquent que le peptide SEQ ID NO : 1 conjugué à la rhodamine a plus d'affinité pour les cellules endothéliales dans un modèle de BHE *in vitro* que le peptide contrôle, à tous les temps analysés (Figure 19).

Le passage d'un peptide contrôle et du peptide SEQ ID NO : 1, tous deux conjugués à la rhodamine a été déterminé sur le modèle de BHE *in vitro* décrit ci-dessus. Cette analyse est réalisée en mesurant par fluorimétrie la quantité de fluorescence accumulée dans le puits receveur à différents temps (1h, 4h, 24h). L'intégrité de la BHE dans les différents puits analysés a été évaluée par mesure simultanée du taux de LY qui passe d'un compartiment à l'autre en fonction du temps. Les valeurs de Pe mesurées indiquent qu'aux temps courts (1h et 4h), les taux de peptide SEQ ID NO : 1 qui passent par transcytose ne peuvent être distingués du passage non spécifique ou para-cellulaire tel que mesuré pour le peptide contrôle. Par contre, à 24h, le taux de passage du peptide SEQ ID NO : 1 conjugué à la rhodamine est très significativement supérieur à celui du peptide contrôle. Les mesures de LY montrent également que l'intégrité de la BHE est préservée à 1h, 4h et 24h (Figure 20). D'autres mesures montrent que la concentration du peptide SEQ ID NO : 1 conjugué à la rhodamine est très supérieure dans le compartiment receveur à 24h, comparée à celle du compartiment donneur, suggérant un transport actif du peptide, certainement via les récepteurs, sans quoi un équilibre de concentration aurait été atteint entre les 2 compartiments.

### EXEMPLE IX

### Optimisation chimique du peptide vecteur SEQ ID NO : 11.

Une étude des relations structure/activité (affinité) a été réalisée sur le peptide vecteur SEQ ID NO : 11 par l'intermédiaire de la technique de l'alanine scanning (Ala-scan) et par troncation en N- et C-term afin de déterminer l'importance de chacun des 15 acides aminés constitutifs de ce peptide. L'affinité pour le hLDLR de chacun des peptides néo-synthétisés issus de l'optimisation chimique a été déterminée par fluorimétrie en mesurant le taux de déplacement du peptide SEQ ID NO : 11 conjugué en C-term via un bras espaceur de 3 Gly au peptide S-Tag (SEQ ID NO : 11/S-Tag) (cf. EXEMPLE VII). Brièvement, les cellules utilisées CHO-hLDLR-RFP sont adhérentes et à confluence. Elles sont cultivées dans des plaques 6 puits. Trois puits de cellules sont utilisés par condition.

Une solution contenant 10µM de peptide SEQ ID NO : 11/S-Tag est préparée dans du milieu de culture HamF12-1% BSA. A cette solution est ajouté 10 µM de peptide issu de l'Ala-scan à évaluer (compétition).

Plusieurs solutions contrôles sont aussi préparées :
(i) Milieu HamF12-1% BSA.
(ii) Milieu HamF12-1% BSA + 10 µM de peptide contrôle CTRL-S-Tag (évaluation de la liaison non spécifique de tout peptide comportant un S-Tag).
(iii) Milieu HamF12-1% BSA + 10 µM de peptide SEQ ID NO : 11/S-Tag + 10 µM de peptide contrôle CTRL (évaluation de la compétition « non spécifique » entre le peptide d'intérêt et le peptide contrôle CTRL).

Les approches de FRET utilisées sont celles décrites dans l'EXEMPLE VII.

Une soixantaine de peptides ont été testés par ces approches. 36 de ces peptides (SEQ ID NO : 30 à SEQ ID NO : 65) présentent une affinité *in vitro* pour le LDLR.

Cette étude a par ailleurs confirmé l'importance des cystéines et donc du cycle et du motif tripeptidique MPR.

Cette étude a également permis de réduire la taille du peptide vecteur puisque le peptide cyclique de 8 acides aminés (SEQ ID NO : 48) comprenant au minimum un acide aminé de configuration D, présente une excellente affinité pour le LDLR.

Selon le même protocole, la SEQ ID NO: 48/S-Tag sert de référence pour l'optimisation chimique (étude de la taille du cycle, introduction d'acides aminés non naturels) du peptide-vecteur SEQ ID NO : 48, et mesure de l'affinité de conjugués sur le LDLR.

Ainsi, les conjugués SEQ ID NO : 66 à SEQ ID NO : 72, décrits dans la présente demande, présentent également une affinité *in vitro* pour le LDLR.

### EXEMPLE X

Stratégie pour la synthèse chimique de conjugués vecteur/molécule d'intérêt thérapeutique ou agent d'imagerie (ou de diagnostic) ou toute autre molécule comme une sonde moléculaire.

Une molécule d'intérêt thérapeutique ou agent d'imagerie ou de diagnostic ou toute autre molécule comme une sonde moléculaire peut être relargué(e) du vecteur après transport et passage au travers des membranes cellulaires et plus particulièrement de la BHE, par exemple au travers d'une stratégie prodrogue par hydrolyse ou clivage enzymatique d'une liaison chimique entre le vecteur et la substance active.

Le couplage covalent, entre le vecteur peptidique totalement protégé sur ses fonctions réactives latérales (cas des couplages en C-term et N-term) ou partiellement protégé (cas du couplage sur une fonction réactive d'une chaîne latérale), et la molécule d'intérêt thérapeutique, est réalisé via 2 stratégies générales (Figure 2) :
- synthèse en tandem (i.e. couplage direct sans intermédiaire entre les 2 entités),
- synthèse au travers d'un *linker* (Temsamani et al., 2004, Drug Discov. Today, 23, 1012-1019*).*

A titre d'exemple, la synthèse en tandem du conjugué peptidique SEQ ID NO : 66 entre un peptide thérapeutique analgésique, la dalargine, et le peptide vecteur SEQ ID NO : 48 a été réalisée comme décrit pour la synthèse de peptides dans l'EXEMPLE VI.

A titre d'exemple, les synthèses via *linkers* peptidiques (GGG, GFLG, ALAL, β-Ala, Ahx, GFAL) des conjugués peptidiques SEQ ID NO : 67 à SEQ ID NO : 72 entre un peptide thérapeutique analgésique, la dalargine, et le peptide vecteur SEQ ID NO : 48 ont été réalisées comme décrit pour la synthèse de peptides dans l'EXEMPLE VI.

En fonction du vecteur peptidique sélectionné et de la molécule d'intérêt thérapeutique choisie, l'une ou l'autre des diverses stratégies est appliquée soit sur le C-term, soit sur le N-term, soit sur une fonction réactive d'une chaîne latérale. Idéalement, les bras espaceurs sélectionnés doivent permettre une bonne libération de la substance active et l'amélioration de la solubilité du conjugué (Molema et al., 2001, Drug targeting, organ-specific stratégies. In Methods and principles in medicinal chemistry, vol. 12*).* Diverses liaisons chimiques covalentes labiles peuvent ainsi être générées entre les 2 entités (vecteur et substance active) au travers ou non d'un bras espaceur : amides, carbamates, esters, thioester, disulfure, etc. Par exemple, il a été montré dans la littérature que les liaisons disulfures, relativement stables dans le plasma, peuvent être clivées à l'intérieur du compartiment intracérébral pour redonner une fonction thiol libre *(*Saito et al., 2003, Adv. Drug Deliv. Rev., 55, 199-215)*.*

D'autres composés intéressants sont ceux dans lesquels le bras espaceur représente un polymère tel que le polyéthylène glycol (PEG). En effet, il a été montré dans la littérature que la conjugaison d'une molécule organique d'intérêt biologique avec un PEG permettait d'augmenter la demi-vie plasmatique de cette molécule *(*Green wald et al., 2003, Adv. Drug Deliv. Rev., 55, 217-250*)* et de diminuer sa clairance.

Les conjugués vecteurs/substances actives ou d'intérêt sont utilisables en diagnostic, imagerie ou thérapie d'une pathologie, d'une lésion ou d'un trouble du SNC pour la préparation d'un médicament capable de traverser la BHE, d'une tumeur cancéreuse cérébrale ou d'un autre type de cellules cancéreuses pour la préparation d'un médicament capable de traverser les membranes cellulaires cancéreuses, et/ou de pathologies infectieuses pour la préparation d'un médicament capable de traverser les membranes cellulaires et de cibler les cellules infectées des pathologies infectieuses cérébrales ou autres, de type bactériennes, virales, parasitaires ou fongiques.

### EXEMPLE XI

Perfusion cérébrale *in situ* pour les vecteurs et les conjugués vecteur/molécule d'intérêt thérapeutique ou agent d'imagerie (ou de diagnostic) ou toute autre molécule comme une sonde moléculaire, et étude de leur cinétique de transport au travers de la BHE, et de leur accumulation dans le cerveau de souris.

La technique de perfusion cérébrale *in situ* (chez la souris mâle adulte OF1) est utilisée ici pour sélectionner les meilleurs vecteurs et apporter la preuve de leur mécanisme d'action pour leur passage dans le cerveau au travers de la BHE.

Au préalable, les vecteurs peptidiques sont radiomarqués au tritium (³H), qui offre la plus forte sensibilité pour la détection de composés radioactifs notamment sur des coupes de tissus. La préparation de peptides radioactifs à haute radioactivité spécifique (RAS, pouvant aller jusqu'à 100 Ci/mmole) est réalisée par une stratégie d'acylation de la fonction amine N-term par de l'anhydride propionique (ou propanoïque) tritié ou du propionyl-N-succinimide (NPS) tritié. Cette méthode de tritiation peut être appliquée à tous les peptides, à condition que la modification du N-term n'affecte pas l'affinité des peptides pour le récepteur ciblé (i.e. LDLR) ou leur activité biologique dans le cas de peptides thérapeutiques.

La réaction de tritiation des peptides vecteurs SEQ ID NO : 11 et SEQ ID NO : 48 en N-term par propionylation est réalisée dans le DMF (1mg de peptide dans 100 à 450 µl selon solubilité) par ajout de 0,1 équivalent de NPS tritié pendant 5 mn à T ambiante, puis de 0,9 équivalent de NPS froid (non tritié) pendant 1h, puis d'un nouvel équivalent de NPS froid pendant 5 h. Le milieu réactionnel est ensuite laissé à 4°C pendant la nuit et purifié le lendemain par HPLC. La RAS pour chaque peptide tritié est comprise entre 5 et 10 Ci/mmol (théoriquement de l'ordre de 7,7 Ci/mmol). La quantité totale de radioactivité préparée par synthèse est comprise entre 600 et 950 µCi.

Dans le cas des conjugués vecteurs/substances actives, il est important, pour éviter les synthèses chimiques radiomarquées nécessitant le développement de nouvelles voies de synthèse longues et coûteuses, de choisir une substance active modèle déjà commercialisée avec marquage au carbone 14 (¹⁴C). Le couplage covalent des peptides radiomarqués (au ³H par exemple) à une substance active radiomarquée (au ¹⁴C par exemple) est effectué, tel que décrit dans l'EXEMPLE X. Comme mentionné précédemment, ce couplage covalent est réalisé en fonction de la structure et des propriétés physico-chimiques de la substance active, en particulier la présence de groupes chimiques fonctionnels pouvant être modifiés sans diminuer l'activité biologique de cette substance. Les synthèses des conjugués radiomarqués sont réalisées par extrapolation à partir des voies de synthèse mise au point sur les conjugués non-radiomarqués.

Une autre stratégie consiste en la synthèse d'un conjugué entre un peptide thérapeutique et un peptide vecteur via un *linker* (pouvant être de nature peptidique ou organique) ou non, comme dans le cas des conjugués SEQ ID NO : 66 à SEQ ID NO : 72 décrits dans la présente demande. Ainsi, le conjugué SEQ ID NO : 72 tritié en N-term a été préparé comme décrit ci-dessus.

Les techniques brièvement résumées ci-dessous ont été développées pour étudier la distribution cérébrale de substances actives et en particulier le rôle de la BHE et plus particulièrement du LDLR dans la pénétration de ces molécules dans le cerveau. Les techniques de perfusion cérébrale *in situ* sont parmi les plus exigeantes techniquement et les plus difficiles à réaliser chez la souris. Toutefois, la perfusion cérébrale *in situ* (comme les modèles *in vitro)* permet un contrôle total de la composition du perfusât artificiel, afin de maintenir les cellules et la vascularisation du cerveau dans des conditions physiologiques et anatomiques normales au sein de l'animal, sans le facteur perturbant de distribution systémique.

Cette stratégie de perfusion *cérébrale in situ* normalement effectuée chez le rat a été adaptée chez la souris *(*Dagenais et al., 2000, J Cereb Blood Flow Metab., 20(2), 381-6) afin d'élargir son application à évaluer les paramètres cinétiques de transport au niveau de la BHE et de la barrière sang-rétine, dans des souris transgéniques et KO mutantes pour les récepteurs, enzymes ou transporteurs de substances actives. Il s'agit d'une cathétérisation d'une carotide chez des souris OF1 anesthésiées, et de la ligature de certaines branches de cette carotide (externe, thyroïdienne, occipitale) afin de perfuser spécifiquement la carotide interne et les artères ptérygopalatines, qui sont utilisées pour évaluer la captation cérébrale des vecteurs et conjugués. Le cathéter permet de remplacer la circulation générale par une infusion avec un perfusât bien contrôlé (tampon bicarbonate, plasma ou sang) en passant par la carotide. Du tampon bicarbonate de Krebs oxygéné est utilisé en premier lieu afin d'évaluer les capacités de passage cérébral des vecteurs et conjugués. Après cathétérisation de la carotide, le débit sanguin endogène est stoppé en sectionnant les ventricules du coeur afin d'éviter le mélange du tampon avec le sang et l'élévation de pression sanguine. La durée de la perfusion à débit fixe est contrôlée. La perfusion avec tampon peut être prolongée jusqu'à 20 min, voire jusqu'à 1h en présence de transporteurs d'oxygène (érythrocytes lavés) pour les études de *Receptor-Mediated Transport* (RMT).

L'étude du peptide vecteur SEQ ID NO : 11 a permis de déterminer son transport cérébral ou coefficient de transfert (Kin). Le temps de perfusion cérébrale pour ces expériences est de 5 min avec un débit de perfusât à 2 ml/min. Ainsi, le calcul du Kin (volume de distribution sur le temps de perfusion cérébrale) du peptide vecteur SEQ ID NO : 11 donne une valeur de 3,5 ± 0,2 x 10⁻⁴ ml/s/g.
A noter que la transferrine (Tf) a un Kin de 3,0 x 10⁻⁴ ml/s/g (Demeule et al., 2008, J. Neurochem., 106 (4), 1534-1544*).* Le Kin de la protéine RAP est de 1,0 x 10⁻⁵ ml/s/g *(*Pan et al., 2004, J. Cell Sci., 117, 5071-5078*).*

L'optimisation chimique du peptide vecteur SEQ ID NO : 11 en un peptide vecteur optimisé SEQ ID NO : 48 a permis une amélioration du passage cérébral du peptide vecteur d'un facteur 1,37. Le peptide (SEQ ID NO : 73, CMPRC) a servi de contrôle pour cette étude de perfusion cérébrale.
Kin de SEQ ID NO : 48 = 4,8 ± 0,4 x 10⁻⁴ ml/s/g
Kin de SEQ ID NO : 73 = 1,5 ± 0,2 x 10⁻⁴ ml/s/g

Comme décrit précédemment, la conjugaison chimique de la dalargine (SEQ ID NO : 74, Y-(D)-AGFLR) via un *linker* en N-term du peptide vecteur (SEQ ID NO : 48) a permis d'obtenir le conjugué SEQ ID NO : 72.

La dalargine (SEQ ID NO : 74) libre non vectorisée a servi de contrôle pour cette étude de perfusion cérébrale.

Ainsi, le passage cérébral de la dalargine vectorisée (SEQ ID NO : 72) par rapport à sa forme libre non vectorisée (SEQ ID NO : 74) est multiplié d'un facteur 21,78.
Kin de SEQ ID NO : 72 = 19,6 ± 3,9 x 10⁻⁴ ml/s/g
Kin de SEQ ID NO : 74 = 0,9 ± 0,1 x 10⁻⁴ ml/s/g

Par ailleurs, ce type d'expérience de perfusion cérébrale *in situ* permet également d'établir une distinction entre les composés qui restent dans le compartiment vasculaire cérébral de ceux ayant franchi la membrane endothéliale abluminale pour entrer dans le parenchyme cérébral. En effet, la technique de la déplétion capillaire post-perfusion permet de mesurer si la molécule traverse effectivement l'endothélium afin d'entrer dans le parenchyme cérébral. L'utilisation de cette technique permet de démontrer que les vecteurs peptidiques spécifiques (ou les conjugués) ont tendance à s'accumuler dans le parenchyme cérébral. Cette technique *(*Triguero et al., 1990, J Neurochem., 54(6), 1882-8) est utilisée afin de faire la distinction entre la fraction de vecteurs (ou conjugués), qui a transitée par l'endothélium et est entrée dans le cerveau par l'espace extracellulaire ou les cellules du cerveau, et la fraction restant associée aux cellules endothéliales.

Ainsi, les étapes clés des études de perfusion cérébrale i*n situ* chez la souris peuvent se résumer comme suit pour les vecteurs et conjugués étudiés :
i) Evaluation de la tolérance (non toxicité) des vecteurs et conjugués au niveau de la BHE, conservation de l'intégrité de cette barrière physiologique.
ii) Etude de la cinétique et de la linéarité de la captation cérébrale via RMT par le LDLR.
iii) Etude du taux de captation cérébrale en fonction de la concentration en vecteurs ou en conjugués (Tmax, Km).
iv) Etude des mécanismes de transport à l'aide de substrats inhibiteurs ou modulateurs du LDLR.
v) Distribution dans les compartiments du cerveau : déplétion capillaire *(*Triguero et al., 1990, J Neurochem., 54(6), 1882-8*).*
vi) Evaluation du taux de liaison des vecteurs et conjugués aux protéines plasmatiques (albumines, etc.), étude de leur influence sur la captation cérébrale de ces molécules
vii) Administration intraveineuse et évaluation de la distribution tissulaire (cerveau et autres organes) en fonction du temps.

Par ailleurs, les Kin de ces différents peptides, au travers de la barrière sang-rétine (BSR), ont également été déterminés.
Kin de SEQ ID NO : 11 = 1,2 ± 0,2 x 10⁻⁴ ml/s/g.
Kin de SEQ ID NO : 48 = 2,4 ± 0,3 x 10⁻⁴ ml/s/g
Kin de SEQ ID NO : 73 = 0,0 ± 0,0 x 10⁻⁴ ml/s/g
Kin de SEQ ID NO : 72 = 11,5 ± 3,3 x 10⁻⁴ ml/s/g
Kin de SEQ ID NO : 74 = 1,8 ± 0,2 x 10⁻⁴ ml/s/g

Ainsi, le passage de la BSR par la dalargine vectorisée (SEQ ID NO : 72) est multiplié d'un facteur 6,39 par rapport à sa forme libre non vectorisée (SEQ ID NO : 74).

## Revendications

1. Peptide ou pseudo-peptide comprenant une séquence d'acides aminés naturels et/ou non naturels, **caractérisé en ce qu'**il lie le récepteur humain aux lipoprotéines de faible densité (hLDLR) à la surface de membranes cellulaires, et **en ce qu'**il répond à la formule générale (I) suivante :
(Xaa)ᵢZ(Xaa)ⱼ-M-P-R-(Xaa)ₖW(Xaa)ₗ (I)
dans laquelle Xaa représente un acide aminé naturel ou non, y compris un acide aminé de configuration D, un acide aminé non codé, ou un acide aminé contenant une liaison peptidomimétique ; Z et W représentent deux acides aminés identiques ou différents permettant la cyclisation du peptide ; i, j, k et 1 sont des nombres entiers, identiques ou différents, compris entre 0 et 5 ; M désigne la méthionine ou un de ses isostères ou un de ses analogues, P désigne la proline ou un de ses isostères ou un de ses analogues, R désigne l'arginine ou un de ses isostères ou un de ses analogues.

2. Peptide ou pseudo-peptide selon la revendication 1, **caractérisé en ce que** i est un entier choisi parmi 0, 1, 3 ou 4 ; j est un entier choisi parmi 0, 3 ou 4 ; k est un entier choisi parmi 0, 1 ou 3 ; et/ou 1 est un entier choisi parmi 0, 1 ou 3.

3. Peptide ou pseudo-peptide selon l'une des revendications 1 à 2, **caractérisé en ce que** les acides aminés permettant la cyclisation du peptide sont choisis parmi la cystéine (Cys), l'acide 3-mercaptopropanoïque (Mpa), la pénicillamine (Pen), la déshydroalanine, l'allylglycine (allylGly), l'acide glutamique, l'acide aspartique, ou la lysine, de préférence Z et W représentent une cystéine.

4. Peptide ou pseudo-peptide, **caractérisé en ce qu'**il lie le récepteur humain aux lipoprotéines de faible densité (hLDLR) à la surface de membranes cellulaires et **en ce qu'**il est choisi parmi les peptides de séquence SEQ ID NOs : 1 à 65.

5. Peptide ou pseudo-peptide selon l'une quelconque des revendications précédentes, **caractérisé en ce qu'**il est de configuration cyclique.

6. Peptide ou pseudo-peptide linéaire ou cyclique selon l'une quelconque des revendications précédentes, **caractérisé en ce qu'**il comprend au moins une liaison peptidomimétique, choisie de préférence parmi l'intercalation d'un groupe méthylène (-CH₂-) ou phosphate (-PO₂-), amine secondaire (-NH-) ou oxygène (-O-), des alpha-azapeptides, alpha-alkylpeptides, N-alkylpeptides, phosphonamidates, depsipeptides, hydroxyméthylènes, hydroxyéthylènes, dihydroxyéthylènes, hydroxyéthylamines, retro-inverso, méthylèneoxy, cétométhylène, esters, phosphinates, phosphiniques, phosphonamides, analogues carba.

7. Peptide ou pseudo-peptide selon l'une quelconque des revendications précédentes, **caractérisé en ce que** la fonction N-terminale (N-term) et/ou C-terminale (C-term) est protégée respectivement par une acylation, ou par une amidation ou une estérification.

8. Utilisation d'un peptide ou pseudo-peptide linéaire ou cyclique selon l'une quelconque des revendications précédentes pour la préparation d'une composition pharmaceutique ou diagnostique pour vectoriser une substance active ou d'intérêt en diagnostic, imagerie ou thérapie, ou pour augmenter l'activité biologique ou diminuer la toxicité d'une substance active ou d'intérêt à laquelle il est couplé.

9. Composé conjugué de formule (II) suivante :
VxDy (II)
dans laquelle V représente un peptide ou pseudo-peptide linéaire ou cyclique selon l'une quelconque des revendications 1 à 7, D représente une substance active ou d'intérêt, et x et y sont des nombres entiers compris entre 1 et 5.

10. Composé conjugué de formule (III) suivante :
VxLzDy (III)
dans laquelle V représente un peptide ou pseudo-peptide linéaire ou cyclique selon l'une quelconque des revendications 1 à 7, L représente un bras espaceur, D représente une substance active ou d'intérêt, x et y sont des nombres entiers compris entre 1 et 5 et z est un entier compris entre 1 et 10.

11. Composé conjugué selon la revendication 10, **caractérisé en ce que** x=z=y=1 ou x=z>y ou z>x>y.

12. Composé conjugué selon l'une des revendications 9 à 11, **caractérisé en ce qu'**il est représenté par les peptides de séquence SEQ ID NO : 66 à SEQ ID NO : 72.

13. Composé conjugué selon l'une des revendications 9 à 12, **caractérisé en ce que** la substance active ou d'intérêt est une molécule d'intérêt thérapeutique, un agent de diagnostic ou d'imagerie médicale, ou une sonde moléculaire, choisie de préférence parmi une petite molécule chimique, un peptide ou polypeptide, une protéine, un antigène, un anticorps ou une partie d'anticorps, un acide nucléique ou un oligonucléotide, un ribozyme, un marqueur ou un traceur.

14. Composé conjugué selon l'une des revendications 9 à 13, **caractérisé en ce que** le couplage entre V et D, ou entre V et L d'une part et entre L et D d'autre part, est réalisé par une ou plusieurs liaisons covalentes, hydrophobes ou ioniques, clivables ou non en milieu physiologique ou à l'intérieur de cellules.

15. Composé conjugué selon l'une quelconque des revendications 9 à 14, **caractérisé en ce que** D est couplé à V, le cas échéant via L, au niveau d'une ou des extrémités N-term et/ou C-term de V et/ou au niveau d'un ou plusieurs groupements réactifs portés par les chaînes latérales des acides aminés naturels ou non, constitutifs de V.

16. Procédé de préparation d'un composé conjugué selon l'une des revendications 9 à 15, **caractérisé en ce qu'**il comprend une étape de couplage entre un peptide ou pseudo-peptide V et une substance D, le cas échéant via L, de préférence par voie chimique, biochimique, enzymatique, ou par génie génétique.

17. Composition pharmaceutique **caractérisée en ce qu'**elle comprend au moins un composé conjugué selon l'une des revendications 9 à 15 et un ou des excipients pharmaceutiquement acceptables.

18. Composition diagnostique **caractérisée en ce qu'**elle comprend un agent de diagnostic ou d'imagerie médicale constitué d'un composé conjugué selon l'une des revendications 9 à 15.

## Patentansprüche

1. Peptid oder Pseudopeptid, umfassend eine Sequenz natürlicher und/oder nicht-natürlicher Aminosäuren, **dadurch gekennzeichnet, dass** es den humanen Rezeptor für Lipoproteine geringer Dichte (hLDLR) auf der Zellmembranoberfläche bindet und dass es der folgenden allgemeinen Formel (I) entspricht:
(Xaa)ᵢZ(Xaa)ⱼ-M-P-R-(Xaa)ₖW(Xaa)ₗ (I)
worin Xaa für eine natürliche oder nicht-natürliche Aminosäure, einschließlich einer Aminosäure mit D-Konfiguration, einer nicht-codierten Aminosäure oder einer Aminosäure, die eine peptidomimetische Verbindung enthält, steht; Z und W für zwei identische oder verschiedene Aminosäuren stehen, die den Ringschluss des Peptids erlauben; i, j, k und l identische oder verschiedene ganze Zahlen zwischen 0 und 5 sind; M Methionin oder eines seiner Isostere oder eines seiner Analoga bezeichnet, P Prolin oder eines seiner Isostere oder eines seiner Analoga bezeichnet, R Arginin oder eines seiner Isostere oder eines seiner Analoga bezeichnet.

2. Peptid oder Pseudopeptid gemäß Anspruch 1, **dadurch gekennzeichnet, dass** i eine ganze Zahl ausgewählt aus 0, 1, 3 oder 4 ist; j eine ganze Zahl ausgewählt aus 0, 3 oder 4 ist; k eine ganze Zahl ausgewählt aus 0, 1 oder 3 ist; und/oder l eine ganze Zahl ausgewählt aus 0, 1 oder 3 ist.

3. Peptid oder Pseudopeptid gemäß einem der Ansprüche 1 bis 2, **dadurch gekennzeichnet, dass** die Aminosäuren, die den Ringschluss des Peptids erlauben, aus Cystein (Cys), 3-Mercaptopropansäure (Mpa), Penicillamin (Pen), Dehydroalanin, Allylglycin (allylGly), Glutaminsäure, Asparaginsäure oder Lysin ausgewählt sind, Z und W bevorzugt für Cystein stehen.

4. Peptid oder Pseudopeptid, **dadurch gekennzeichnet, dass** es den humanen Rezeptor für Lipoproteine geringer Dichte (hLDLR) auf der Zellmembranoberfläche bindet und dass es aus den Peptiden der Sequenzen SEQ ID NOs: 1 bis 65 ausgewählt ist.

5. Peptid oder Pseudopeptid gemäß einem der vorhergehenden Ansprüche, **dadurch gekennzeichnet, dass** es eine cyclische Konfiguration aufweist.

6. Lineares oder cyclisches Peptid oder Pseudopeptid gemäß einem der vorhergehenden Ansprüche, **dadurch gekennzeichnet, dass** es wenigstens eine peptidomimetische Bindung umfasst, die bevorzugt aus der Interkalation von einer Methylen- (-CH₂-) oder Phosphatgruppe (-PO₂-), sekundärem Amin (-NH-) oder Sauerstoff (-O-), alpha-Azapeptiden, alpha-Alkylpeptiden, N-Alkylpeptiden, Phosphonamidaten, Depsipeptiden, Hydroxymethylenen, Hydroxyethylenen, Dihydroxyethylenen, Hydroxyethylaminen, retro-inverso, Methylenoxy, Ketomethylen, Estern, Phosphinaten, Phosphinen, Phosphonamiden, Carba-Analogen ausgewählt ist.

7. Peptid oder Pseudopeptid gemäß einem der vorhergehenden Ansprüche, **dadurch gekennzeichnet, dass** die N-terminale (N-term) und/oder C-terminale (C-term) Funktion jeweils durch eine Acylierung oder durch eine Amidierung oder eine Veresterung geschützt ist.

8. Verwendung eines linearen oder cyclischen Peptids oder Pseudopeptids gemäß einem der vorhergehenden Ansprüche zur Herstellung einer pharmazeutischen oder diagnostischen Zusammensetzung, um eine aktive Substanz oder eine Substanz von Interesse für Diagnose, Bildgebung oder Therapie zu vektorisieren oder um die biologische Aktivität zu erhöhen oder die Toxizität einer aktiven Substanz oder Substanz von Interesse, an der es gekoppelt ist, zu verringern.

9. Konjugierte Verbindung der folgenden Formel (II):
VxDy (II)
worin V für ein lineares oder cyclisches Peptid oder Pseudopeptid gemäß einem der Ansprüche 1 bis 7 steht, D für eine aktive Substanz oder eine Substanz von Interesse steht und x und y ganze Zahlen zwischen 1 und 5 sind.

10. Konjugierte Verbindung der folgenden Formel (III):
VxLzDy (III)
worin V für ein lineares oder cyclisches Peptid oder Pseudopeptid gemäß einem der Ansprüche 1 bis 7 steht, L für einen Spacer steht, D für eine aktive Substanz oder eine Substanz von Interesse steht und x und y ganze Zahlen zwischen 1 und 5 sind und z eine ganze Zahl zwischen 1 und 10 ist.

11. Konjugierte Verbindung gemäß Anspruch 10, **dadurch gekennzeichnet, dass** x = y =z = 1 oder x = z > y oder z > x > y.

12. Konjugierte Verbindung gemäß einem der Ansprüche 9 bis 11, **dadurch gekennzeichnet, dass** sie von den Peptiden der Sequenzen SEQ ID NO: 66 bis SEQ ID NR: 72 dargestellt ist.

13. Konjugierte Verbindung gemäß einem der Ansprüche 9 bis 12, **dadurch gekennzeichnet, dass** die aktive Substanz oder die Substanz von Interesse ein Molekül von therapeutischem Interesse, ein Agens für Diagnose oder medizinische Bildgebung oder eine molekulare Sonde ist, die bevorzugt aus einem kleinen chemischen Molekül, einem Peptid oder Polypeptid, einem Protein, einem Antigen, einem Antikörper oder einem Teil eines Antikörpers, einer Nukleinsäure oder einem Oligonukleotid, einem Ribozym, einem Marker oder einem Tracer ausgewählt ist.

14. Konjugierte Verbindung gemäß einem der Ansprüche 9 bis 13, **dadurch gekennzeichnet, dass** die Kopplung zwischen V und D oder zwischen V und L einerseits und zwischen L und D andererseits durch eine oder mehrere kovalente, hydrophobe oder ionische Bindungen erfolgt, die in physiologischem Milieu oder im Zellinnern gespalten werden können.

15. Konjugierte Verbindung gemäß einem der Ansprüche 9 bis 14, **dadurch gekennzeichnet, dass** D an V, gegebenenfalls über L an einem oder mehreren N-term- und/oder C-term-Enden von V und/oder an einer oder mehreren reaktiven Gruppen in den Seitenketten der natürlichen oder nicht-natürlichen Aminosäuren, die für V konstitutiv sind, gekoppelt ist.

16. Verfahren zur Herstellung einer konjugierten Verbindung gemäß einem der Ansprüche 9 bis 15, **dadurch gekennzeichnet, dass** es einen Kopplungsschritt zwischen einem Peptid oder Pseudopeptid V und einer Substanz D, gegebenenfalls über L, bevorzugt auf chemische, biochemische, enzymatische Weise oder mittels Gentechnik umfasst.

17. Pharmazeutische Zusammensetzung, **dadurch gekennzeichnet, dass** sie wenigstens eine konjugierte Verbindung gemäß einem der Ansprüche 9 bis 15 und einen oder mehrere pharmazeutisch verträgliche Träger umfasst.

18. Diagnostische Zusammensetzung, **dadurch gekennzeichnet, dass** sie ein Agens für Diagnose oder medizinische Bildgebung umfasst, das aus einer konjugierten Verbindung gemäß einem der Ansprüche 9 bis 15 besteht.

## Claims

1. A peptide or pseudo-peptide comprising a sequence of natural and/or non-natural amino acids, **characterised in that** it binds human low density lipoprotein receptor (hLDLR) at the surface of cell membranes and **in that** it is of general formula (I) as follows:
(Xaa)ᵢZ(Xaa)ⱼ-M-P-R-(Xaa)ₖW(Xaa)ₗ (I)
wherein Xaa represents a natural or non-natural amino acid, such as an amino acid of configuration D, a non-coded amino acid, or an amino acid containing a peptidomimetic bond, Z and W represent two identical or different amino acids enabling cyclisation of the peptide, and i, j, k and l are integers, identical or different, between 0 and 5, M designates methionine or an isostere thereof or an analogue thereof, P designates proline or an isostere thereof or an analogue thereof, R designates arginine or an isostere thereof or an analogue thereof.

2. The peptide or pseudo-peptide according to claim 1, **characterised in that** i is an integer selected from 0, 1, 3 or 4; j is an integer selected from 0, 3 or 4; k is an integer selected from 0, 1 or 3; and/or l is an integer selected from 0, 1 or 3.

3. The peptide or pseudo-peptide according to any one of claims 1 to 2, **characterised in that** the amino acids enabling cyclisation of the peptide are selected from cysteine (Cys), 3-mercaptopropanoic acid (Mpa), penicillamine (Pen), dehydroalanine, allylglycine (allylGly), glutamic acid, aspartic acid, or lysine, preferably Z and W represent a cysteine.

4. A peptide or pseudo-peptide, **characterised in that** it binds human low density lipoprotein receptor (hLDLR) at the surface of cell membranes and **in that** it is selected from the peptides of sequence SEQ ID NOs: 1 to SEQ ID NO: 65.

5. The peptide or pseudo-peptide according to any of the preceding claims, **characterised in that** it is of cyclic configuration.

6. The linear or cyclic peptide or pseudo-peptide according to anyone of the preceding claims, **characterised in that** it comprises at least one peptidomimetic bond chosen preferably among intercalation of a methylene (-CH₂-) or phosphate (-PO₂-) group, secondary amine (-NH-) or oxygen (-O-), alpha-azapeptides, alpha-alkylpeptides, N-alkylpeptides, phosphonamidates, depsipeptides, hydroxymethylenes, hydroxyethylenes, dihydroxyethylenes, hydroxyethylamines, retro-inverso peptides, methyleneoxy, cetomethylene, esters, phosphinates, phosphinics, phosphonamides, carba analogues.

7. The peptide or pseudo-peptide according to anyone of the preceding claims, **characterised in that** the N-terminus (N-term) and/or C-terminus (C-term) function is respectively protected by acylation, or by amidation or esterification.

8. Use of a linear or cyclic peptide or pseudo-peptide according to anyone of the preceding claims for preparing a pharmaceutical or diagnostic composition to vectorise an active substance or substance of interest in diagnostics, imaging or therapy, or to increase the biological activity or decrease the toxicity of an active substance or substance of interest to which it is coupled.

9. A conjugate compound of formula (II) as follows:
VxDy (II)
wherein V represents a linear or cyclic peptide or pseudo-peptide according to anyone of claims 1 to 7, D represents an active substance or substance of interest, and x and y are integers between 1 and 5.

10. A conjugate compound of formula (III) as follows:
VxLzDy (III)
wherein V represents a linear or cyclic peptide or pseudo-peptide according to any of claims 1 to 7, L represents a spacer, D represents an active substance or substance of interest, x and y are integers between 1 and 5 and z is an integer between 1 and 10.

11. The conjugate compound according to claim 10, **characterised in that** x=z=y=1 or x=z>y or z>x>y.

12. The conjugate compound according to anyone of claims 9 to 11, **characterised in that** it is represented by the peptides of sequence SEQ ID NO: 66 to SEQ ID NO: 72.

13. The conjugate compound according to anyone of claims 9 to 12, **characterised in that** the active substance or substance of interest is a molecule of therapeutic interest, a diagnostic or medical imaging agent, or a molecular probe, preferably selected from a small chemical molecule, a peptide or polypeptide, a protein, an antigen, an antibody or part of an antibody, a nucleic acid or an oligonucleotide, a ribozyme, a marker or a tracer.

14. The conjugate compound according to anyone of claims 9 to 13, **characterised in that** coupling between V and D, or between V and L on the one hand and L and D on the other hand, is carried out by one or more covalent, hydrophobic or ionic bonds, cleavable or non-cleavable in physiological medium or within cells.

15. The conjugate compound according to anyone of claims 9 to 14, **characterised in that** D is coupled with V, if need be via L, at one or more N-term and/or C-term ends of V and/or at one or more reactive groups carried by the natural or non-natural amino acid side chains constitutive of V.

16. A method for preparing a conjugate compound according to anyone of claims 9 to 15, **characterised in that** it comprises a step of coupling between a peptide or pseudo-peptide V and a substance D, if need be via L, preferably by a chemical, biochemical or enzymatic pathway, or by genetic engineering.

17. A pharmaceutical composition **characterised in that** it comprises at least one conjugate compound according to anyone of claims 9 to 15 and one or several pharmaceutically acceptable excipients.

18. A diagnostic composition **characterised in that** it comprises a diagnostic or medical imaging agent composed of a conjugate compound according to anyone of claims 9 to 15.


### SEQUENCE LISTING

<110> VECT-HORUS CNRS UNIVERSITE DE LA MEDITERRANNEE
<120> DERIVES PEPTIDIQUES ET LEUR UTILISATION COMME VECTEURS DE MOLECULES SOUS FORME DE CONJUGUES
<130> B783
<160> 74
<170> PatentIn version 3.3
<210> 1
   <211> 12
   <212> PRT
   <213> artificial sequence
<220>
   <223> peptide
<400> 1
   His Leu Asp Cys Met Pro Arg Gly Cys Phe Arg Asn
   1               5                   10
<210> 2
   <211> 10
   <212> PRT
   <213> artificial sequence
<220>
   <223> peptide
<400> 2
   Ala Cys Gln Val Lys Ser Met Pro Arg Cys
   1               5                   10
<210> 3
   <211> 10
   <212> PRT
   <213> artificial sequence
<220>
   <223> peptide
<400> 3
   Ala Cys Thr Thr Pro Met Pro Arg Leu Cys
   1               5                   10
<210> 4
   <211> 10
   <212> PRT
   <213> artificial sequence
<220>
   <223> peptide
<400> 4
   Ala Cys Lys Ala Pro Gln Met Pro Arg Cys
   1               5                   10
<210> 5
   <211> 10
   <212> PRT
   <213> artificial sequence
<220>
   <223> peptide
<400> 5
   Ala Cys Leu Asn Pro Ser Met Pro Arg Cys
   1               5                   10
<210> 6
   <211> 10
   <212> PRT
   <213> artificial sequence
<220>
   <223> peptide
<400> 6
   Ala Cys Leu Val Ser Ser Met Pro Arg Cys
   1               5                   10
<210> 7
   <211> 10
   <212> PRT
   <213> artificial sequence
<220>
   <223> peptide
<400> 7
   Ala Cys Leu Gln Pro Met Pro Arg Leu Cys
   1 5 10
<210> 8
   <211> 10
   <212> PRT
   <213> artificial sequence
<220>
   <223> peptide
<400> 8
   Ala Cys Pro Val Ser Ser Met Pro Arg Cys
   1 5 10
<210> 9
   <211> 10
   <212> PRT
   <213> artificial sequence
<220>
   <223> peptide
<400> 9
   Ala Cys Gln Ser Pro Met Pro Arg Leu Cys
   1 5 10
<210> 10
   <211> 10
   <212> PRT
   <213> artificial sequence
<220>
   <223> peptide
<400> 10
   Ala Cys Leu Thr Pro Met Pro Arg Leu Cys
   1 5 10
<210> 11
   <211> 15
   <212> PRT
   <213> artificial sequence
<220>
   <223> peptide
<400> 11
   Asp Ser Gly Leu Cys Met Pro Arg Leu Arg Gly Cys Asp Pro Arg
   1               5                   10                  15
<210> 12
   <211> 15
   <212> PRT
   <213> artificial sequence
<220>
   <223> peptide
<400> 12
   Met Thr Val Met Pro Thr Gly Leu Trp Asn Pro Leu Ile Pro Ser
   1               5                   10                  15
<210> 13
   <211> 15
   <212> PRT
   <213> artificial sequence
<220>
   <223> peptide
<400> 13
   Ser Ala Ser Trp Phe Ala Val Pro Ile Pro Pro Leu Arg Leu Glu
   1               5                   10                  15
<210> 14
   <211> 15
   <212> PRT
   <213> artificial sequence
<220>
   <223> peptide
<400> 14
   Met Thr Pro Met Ser Thr Pro Arg Met Leu Pro Val Tyr Val Ala
   1               5                   10                  15
<210> 15
   <211> 15
   <212> PRT
   <213> artificial sequence
<220>
   <223> peptide
<400> 15
   Met Thr Ala Thr His Leu Ser Thr Leu Phe Gln Pro Leu Thr Tyr
   1 5 10 15
<210> 16
   <211> 15
   <212> PRT
   <213> artificial sequence
<220>
   <223> peptide
<400> 16
   Met Ser Pro Ile Pro Pro Ala Ala Ser Thr Trp Ala Asn Thr Leu
   1 5 10 15
<210> 17
   <211> 15
   <212> PRT
   <213> artificial sequence
<220>
   <223> peptide
<400> 17
   Met Thr Ala Asn Pro Leu Gln Asn Ala Pro Gly Pro Leu Ser Leu
   1 5 10 15
<210> 18
   <211> 15
   <212> PRT
   <213> artificial sequence
<220>
   <223> peptide
<400> 18
   Met Gln Thr Ala Pro Pro Pro Pro Leu Thr Arg Val Gln Trp Ser
   1 5 10 15
<210> 19
   <211> 15
   <212> PRT
   <213> artificial sequence
<220>
   <223> peptide
<400> 19
   Gly Thr Pro Arg Met His Ile Pro Leu Asn Val Asp His Leu Pro
   1 5 10 15
<210> 20
   <211> 15
   <212> PRT
   <213> artificial sequence
<220>
   <223> peptide
<400> 20
   Leu Thr Leu Pro Pro Ile Ser Gly Leu Ser Ser Tyr Pro Leu Pro
   1 5 10 15
<210> 21
   <211> 15
   <212> PRT
   <213> artificial sequence
<220>
   <223> peptide
<400> 21
   Thr Pro Ser Ala His Ala Met Ala Leu Gln Ser Leu Ser Val Gly
   1 5 10 15
<210> 22
   <211> 15
   <212> PRT
   <213> artificial sequence
<220>
   <223> peptide
<400> 22
   Leu Thr Leu Pro Pro Ile Ser Gly Leu Ser Ser Tyr Pro Leu Pro
   1 5 10 15
<210> 23
   <211> 15
   <212> PRT
   <213> artificial sequence
<220>
   <223> peptide
<400> 23
   Met Gly Thr Leu Asn Ala Pro Thr Ala Tyr Pro Gln Asp Ser Leu
   1 5 10 15
<210> 24
   <211> 15
   <212> PRT
   <213> artificial sequence
<220>
   <223> peptide
<400> 24
   Leu Thr Asn Pro Pro Ala Tyr Leu Pro Gln Asn Thr Asp Pro His
   1 5 10 15
<210> 25
   <211> 15
   <212> PRT
   <213> artificial sequence
<220>
   <223> peptide
<400> 25
   Met Gly Leu Pro Leu Pro Tyr Ile Gln Thr Ile Leu His Thr Pro
   1 5 10 15
<210> 26
   <211> 15
   <212> PRT
   <213> artificial sequence
<220>
   <223> peptide
<400> 26
   Ser Ala Ala Leu Ile Ala Met Ser Ser Phe Lys Ser Ile Thr Ala
   1 5 10 15
<210> 27
   <211> 15
   <212> PRT
   <213> artificial sequence
<220>
   <223> peptide
<400> 27
   Ser Gly Phe Ala Phe Ala Arg Ser Val Pro Thr Glu Ser Arg Arg
   1               5                   10                  15
<210> 28
   <211> 15
   <212> PRT
   <213> artificial sequence
<220>
   <223> peptide
<400> 28
   Met Thr Ser Pro Tyr Met Ser Leu Pro Pro Ser Thr Asp Asp Met
   1               5                   10                  15
<210> 29
   <211> 15
   <212> PRT
   <213> artificial sequence
<220>
   <223> peptide
<400> 29
   Leu Thr Asn Pro Pro Ala Tyr Leu Pro Gln Asn Thr Asp Pro His
   1               5                   10                  15
<210> 30
   <211> 10
   <212> PRT
   <213> artificial sequence
<220>
   <223> peptide
<400> 30
   Ala Cys Met Pro Arg Leu Arg Gly Cys Ala
   1               5                   10
<210> 31
   <211> 15
   <212> PRT
   <213> artificial sequence
<220>
   <223> peptide
<400> 31
   Ala Ser Gly Leu Cys Met Pro Arg Leu Arg Gly Cys Asp Pro Arg
   1               5                   10                  15
<210> 32
   <211> 15
   <212> PRT
   <213> artificial sequence
<220>
   <223> peptide
<400> 32
   Asp Ala Gly Leu Cys Met Pro Arg Leu Arg Gly Cys Asp Pro Arg
   1               5                   10                  15
<210> 33
   <211> 15
   <212> PRT
   <213> artificial sequence
<220>
   <223> peptide
<400> 33
   Asp Ser Ala Leu Cys Met Pro Arg Leu Arg Gly Cys Asp Pro Arg
   1               5                   10                  15
<210> 34
   <211> 15
   <212> PRT
   <213> artificial sequence
<220>
   <223> peptide
<400> 34
   Asp Ser Gly Ala Cys Met Pro Arg Leu Arg Gly Cys Asp Pro Arg
   1               5                   10                  15
<210> 35
   <211> 15
   <212> PRT
   <213> artificial sequence
<220>
   <223> peptide
<400> 35
   Asp Ser Gly Leu Cys Met Pro Arg Ala Arg Gly Cys Asp Pro Arg
   1               5                   10                  15
<210> 36
   <211> 15
   <212> PRT
   <213> artificial sequence
<220>
   <223> peptide
<400> 36
   Asp Ser Gly Leu Cys Met Pro Arg Leu Ala Gly Cys Asp Pro Arg
   1 5 10 15
<210> 37
   <211> 15
   <212> PRT
   <213> artificial sequence
<220>
   <223> peptide
<400> 37
   Asp Ser Gly Leu Cys Met Pro Arg Leu Arg Ala Cys Asp Pro Arg
   1 5 10 15
<210> 38
   <211> 15
   <212> PRT
   <213> artificial sequence
<220>
   <223> peptide
<400> 38
   Asp Ser Gly Leu Cys Met Pro Arg Leu Arg Gly Cys Ala Pro Arg
   1 5 10 15
<210> 39
   <211> 15
   <212> PRT
   <213> artificial sequence
<220>
   <223> peptide
<400> 39
   Asp Ser Gly Leu Cys Met Pro Arg Leu Arg Gly Cys Asp Ala Arg
   1 5 10 15
<210> 40
   <211> 15
   <212> PRT
   <213> artificial sequence
<220>
   <223> peptide
<400> 40
   Asp Ser Gly Leu Cys Met Pro Arg Leu Arg Gly Cys Asp Pro Ala
   1 5 10 15
<210> 41
   <211> 14
   <212> PRT
   <213> artificial sequence
<220>
   <223> peptide
<400> 41
   Ser Gly Leu Cys Met Pro Arg Leu Arg Gly Cys Asp Pro Arg
   1 5 10
<210> 42
   <211> 13
   <212> PRT
   <213> artificial sequence
<220>
   <223> peptide
<400> 42
   Gly Leu Cys Met Pro Arg Leu Arg Gly Cys Asp Pro Arg
   1 5 10
<210> 43
   <211> 8
   <212> PRT
   <213> artificial sequence
<220>
   <223> peptide
<400> 43
   Cys Met Pro Arg Leu Arg Gly Cys
   1 5
<210> 44
   <211> 8
   <212> PRT
   <213> artificial sequence
<220>
   <223> peptide
<400> 44
   Cys Met Pro Arg Ala Arg Gly Cys
   1               5
<210> 45
   <211> 8
   <212> PRT
   <213> artificial sequence
<220>
   <223> peptide
<400> 45
   Cys Met Pro Arg Leu Ala Gly Cys
   1               5
<210> 46
   <211> 8
   <212> PRT
   <213> artificial sequence
<220>
   <223> peptide
<400> 46
   Cys Met Pro Arg Leu Arg Ala Cys
   1               5
<210> 47
   <211> 8
   <212> PRT
   <213> artificial sequence
<220>
   <223> peptide
<400> 47
   Cys Met Pro Arg Leu Lys Gly Cys
   1               5
<210> 48
   <211> 8
   <212> PRT
   <213> artificial sequence
<220>
   <223> peptide
<220>
   <221> MISC_FEATURE
   <222> (1)..(1)
   <223> Xaa = (D)-Cystein
<400> 48
   Xaa Met Pro Arg Leu Arg Gly Cys
   1               5
<210> 49
   <211> 8
   <212> PRT
   <213> artificial sequence
<220>
   <223> peptide
<220>
   <221> MISC_FEATURE
   <222> (5)..(5)
   <223> Xaa = (D)-Leucin
<400> 49
   Cys Met Pro Arg Xaa Arg Gly Cys
   1               5
<210> 50
   <211> 8
   <212> PRT
   <213> artificial sequence
<220>
   <223> peptide
<220>
   <221> MISC_FEATURE
   <222> (6)..(6)
   <223> Xaa = (D)-Arginin
<400> 50
   Cys Met Pro Arg Leu Xaa Gly Cys
   1               5
<210> 51
   <211> 8
   <212> PRT
   <213> artificial sequence
<220>
   <223> peptide
<220>
   <221> MISC_FEATURE
   <222> (8)..(8)
   <223> Xaa = (D)-Cystein
<400> 51
   Cys Met Pro Arg Leu Arg Gly Xaa
   1               5
<210> 52
   <211> 8
   <212> PRT
   <213> artificial sequence
<220>
   <223> peptide
<220>
   <221> MISC_FEATURE
   <222> (1)..(1)
   <223> Xaa = (D)-Cystein
<220>
   <221> MISC_FEATURE
   <222> (8)..(8)
   <223> Xaa = (D)-Cystein
<400> 52
   Xaa Met Pro Arg Leu Arg Gly Xaa
   1               5
<210> 53
   <211> 8
   <212> PRT
   <213> artificial sequence
<220>
   <223> peptide
<220>
   <221> MISC_FEATURE
   <222> (1)..(1)
   <223> Xaa = (D)-Cystein
<220>
   <221> MISC_FEATURE
   <222> (7)..(7)
   <223> Xaa = Sar
<400> 53
   Xaa Met Pro Arg Leu Arg Xaa Cys
   1               5
<210> 54
   <211> 8
   <212> PRT
   <213> artificial sequence
<220>
   <223> peptide
<220>
   <221> MISC_FEATURE
   <222> (1)..(1)
   <223> Xaa = (D)-Cystein
<400> 54
   Xaa Met Pro Arg Lys Arg Gly Cys
   1 5
<210> 55
   <211> 8
   <212> PRT
   <213> artificial sequence
<220>
   <223> peptide
<220>
   <221> MISC_FEATURE
   <222> (1)..(1)
   <223> Xaa = (D)-Cystein
<400> 55
   Xaa Met Pro Arg Leu Arg Cys Gly
   1 5
<210> 56
   <211> 8
   <212> PRT
   <213> artificial sequence
<220>
   <223> peptide
<220>
   <221> MISC_FEATURE
   <222> (1)..(1)
   <223> Xaa = (D)-Cystein
<400> 56
   Xaa Met Pro Arg Leu Cys Arg Gly
   1 5
<210> 57
   <211> 8
   <212> PRT
   <213> artificial sequence
<220>
   <223> peptide
<220>
   <221> MISC_FEATURE
   <222> (1)..(1)
   <223> Xaa = (D)-Cystein
<400> 57
   Xaa Met Pro Arg Cys Leu Arg Gly
   1               5
<210> 58
   <211> 6
   <212> PRT
   <213> artificial sequence
<220>
   <223> peptide
<400> 58
   Cys Met Pro Arg Gly Cys
   1               5
<210> 59
   <211> 8
   <212> PRT
   <213> artificial sequence
<220>
   <223> peptide
<220>
   <221> MISC_FEATURE
   <222> (1)..(1)
   <223> Xaa = Pen
<400> 59
   Xaa Met Pro Arg Leu Arg Gly Cys
   1               5
<210> 60
   <211> 8
   <212> PRT
   <213> artificial sequence
<220>
   <223> peptide
<220>
   <221> MISC_FEATURE
   <222> (1)..(1)
   <223> Xaa = (D)-Pen
<400> 60
   Xaa Met Pro Arg Leu Arg Gly Cys
   1 5
<210> 61
   <211> 8
   <212> PRT
   <213> artificial sequence
<220>
   <223> peptide
<220>
   <221> MISC_FEATURE
   <222> (1)..(1)
   <223> Xaa = (D)-Cystein
<220>
   <221> MISC_FEATURE
   <222> (8)..(8)
   <223> Xaa = Pen
<400> 61
   Xaa Met Pro Arg Leu Arg Gly Xaa
   1 5
<210> 62
   <211> 8
   <212> PRT
   <213> artificial sequence
<220>
   <223> peptide
<220>
   <221> MISC_FEATURE
   <222> (1)..(1)
   <223> Xaa = Pen
<220>
   <221> MISC_FEATURE
   <222> (8)..(8)
   <223> Xaa = Pen
<400> 62
   Xaa Met Pro Arg Leu Arg Gly Xaa
   1 5
<210> 63
   <211> 8
   <212> PRT
   <213> artificial sequence
<220>
   <223> peptide
<220>
   <221> MISC_FEATURE
   <222> (1)..(1)
   <223> Xaa = (D)-Pen
<220>
   <221> MISC_FEATURE
   <222> (8)..(8)
   <223> Xaa = Pen
<400> 63
   Xaa Met Pro Arg Leu Arg Gly Xaa
   1               5
<210> 64
   <211> 8
   <212> PRT
   <213> artificial sequence
<220>
   <223> peptide
<220>
   <221> MISC_FEATURE
   <222> (1)..(1)
   <223> Xaa = (D)-Pen
<220>
   <221> MISC_FEATURE
   <222> (8)..(8)
   <223> Xaa = (D)-Pen
<400> 64
   Xaa Met Pro Arg Leu Arg Gly Xaa
   1               5
<210> 65
   <211> 8
   <212> PRT
   <213> artificial sequence
<220>
   <223> peptide
<220>
   <221> MISC_FEATURE
   <222> (1)..(1)
   <223> Xaa = Mpa
<400> 65
   Xaa Met Pro Arg Leu Arg Gly Cys
   1               5
<210> 66
   <211> 14
   <212> PRT
   <213> artificial sequence
<220>
   <223> peptide
<220>
   <221> MISC_FEATURE
   <222> (2)..(2)
   <223> Xaa = (D)-Alanin
<220>
   <221> MISC_FEATURE
   <222> (7)..(7)
   <223> Xaa = (D)-Cystein
<400> 66
   Tyr Xaa Gly Phe Leu Arg Xaa Met Pro Arg Leu Arg Gly Cys
   1               5                   10
<210> 67
   <211> 17
   <212> PRT
   <213> artificial sequence
<220>
   <223> peptide
<220>
   <221> MISC_FEATURE
   <222> (2)..(2)
   <223> Xaa = D-Alanin
<220>
   <221> MISC_FEATURE
   <222> (10)..(10)
   <223> Xaa = D-Cystein
<400> 67 Cys
<210> 68
   <211> 18
   <212> PRT
   <213> artificial sequence
<220>
   <223> peptide
<220>
   <221> MISC_FEATURE
   <222> (2)..(2)
   <223> Xaa = D-alanin
<220>
   <221> MISC_FEATURE
   <222> (11)..(11)
   <223> Xaa = D-cystein
<400> 68
   Tyr Xaa Gly Phe Leu Arg Gly Phe Leu Gly Xaa Met Pro Arg Leu Arg
   1               5                   10                  15
   Gly Cys
<210> 69
   <211> 18
   <212> PRT
   <213> artificial sequence
<220>
   <223> peptide
<220>
   <221> MISC_FEATURE
   <222> (2)..(2)
   <223> Xaa = D-alanin
<220>
   <221> MISC_FEATURE
   <222> (11)..(11)
   <223> Xaa = D-cystein
<400> 69
   Tyr Xaa Gly Phe Leu Arg Ala Leu Ala Leu Xaa Met Pro Arg Leu Arg
   1               5                   10                  15
   Gly Cys
<210> 70
   <211> 15
   <212> PRT
   <213> artificial sequence
<220>
   <223> peptide
<220>
   <221> MISC_FEATURE
   <222> (2)..(2)
   <223> Xaa = D-alanin
<220>
   <221> MISC_FEATURE
   <222> (7)..(7)
   <223> Ala= beta-alanine
<220>
   <221> MISC_FEATURE
   <222> (8)..(8)
   <223> Xaa = D-cystein
<400> 70
   Tyr Xaa Gly Phe Leu Arg Ala Xaa Met Pro Arg Leu Arg Gly Cys
   1               5                   10                  15
<210> 71
   <211> 15
   <212> PRT
   <213> artificial sequence
<220>
   <223> peptide
<220>
   <221> MISC_FEATURE
   <222> (2)..(2)
   <223> Xaa = D-alanin
<220>
   <221> MISC_FEATURE
   <222> (7)..(7)
   <223> Xaa = Ahx
<220>
   <221> MISC_FEATURE
   <222> (8)..(8)
   <223> Xaa = D-cystein
<400> 71
   Tyr Xaa Gly Phe Leu Arg Xaa Xaa Met Pro Arg Leu Arg Gly Cys
   1               5                   10                  15
<210> 72
   <211> 18
   <212> PRT
   <213> artificial sequence
<220>
   <223> peptide
<220>
   <221> MISC_FEATURE
   <222> (2)..(2)
   <223> Xaa = D-alanin
<220>
   <221> MISC_FEATURE
   <222> (11)..(11)
   <223> Xaa = D-cystein
<400> 72
   Tyr Xaa Gly Phe Leu Arg Gly Phe Ala Leu Xaa Met Pro Arg Leu Arg
   1               5                   10                  15
   Gly Cys
<210> 73
   <211> 5
   <212> PRT
   <213> artificial sequence
<220>
   <223> peptide
<400> 73
   Cys Met Pro Arg Cys
   1               5
<210> 74
   <211> 6
   <212> PRT
   <213> artificial sequence
<220>
   <223> peptide
<220>
   <221> MISC_FEATURE
   <222> (2)..(2)
   <223> Xaa = D-alanin
<400> 74
   Tyr Xaa Gly Phe Leu Arg
   1               5